(19) **Europäisches Patentamt / European Patent Office / Office européen des brevets**

(11) **EP 4 324 929 B1**

(12) **EUROPEAN PATENT SPECIFICATION**

(45) Date of publication and mention of the grant of the patent:
**17.09.2025 Bulletin 2025/38**

(21) Application number: **23183318.7**

(22) Date of filing: **16.05.2017**

(51) International Patent Classification (IPC):
**C12Q 1/68** (2018.01)

(52) Cooperative Patent Classification (CPC):
(C-Sets available)
**C12Q 1/6816** (Cont.)

(54) **METHODS FOR DETECTING TARGET NUCLEIC ACIDS IN A SAMPLE**

VERFAHREN ZUR ERKENNUNG VON ZIELNUKLEINSÄUREN IN EINER PROBE

PROCÉDÉS POUR DÉTECTER DES ACIDES NUCLÉIQUES DANS UN ÉCHANTILLON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2016 US 201662337074 P**
**01.05.2017 US 201762492889 P**

(43) Date of publication of application:
**21.02.2024 Bulletin 2024/08**

(62) Document number(s) of the earlier application(s) in accordance with Art. 76 EPC:
**17726083.3 / 3 458 601**

(73) Proprietor: **Bruker Spatial Biology, Inc.**
**Billerica, MA 01821 (US)**

(72) Inventors:
- **KHAFIZOV, Rustem**
  **Seattle, WA 98109 (US)**
- **DUNAWAY, Dwayne L.**
  **Seattle, WA 98115 (US)**
- **GREGORY, Mark**
  **Seattle, WA 98125 (US)**

(74) Representative: **D Young & Co LLP**
**3 Noble Street**
**London EC2V 7BQ (GB)**

(56) References cited:
**WO-A1-2011/106583     WO-A1-2016/081740**
**WO-A2-2013/055995**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6816, C12Q 2523/319, C12Q 2563/113,
C12Q 2563/185**

**Description**

SEQUENCE LISTING

The instant application contains a Sequence Listing which has been submitted in ASCII format *via* EFS-Web and is hereby incorporated by reference in its entirety. Said ASCII copy, created on May 16, 2017, is named NATE-032001WO_ST25.txt and is 22,780 bytes in size.

BACKGROUND OF THE INVENTION

Although there are currently a variety of methods for detecting nucleic acids in a biological sample, a need remains for improved, accurate, rapid, and sensitive multiplexed detection, identification, and quantification of target nucleic acids. The present invention addresses this need.

SUMMARY OF THE INVENTION

The present invention is defined in the appended claims. The present disclosure provides probes, methods, kits, and apparatuses that provide accurate, rapid, and sensitive multiplexed detection, identification, and quantification of target nucleic acids in a sample.

One aspect of the present disclosure, that is not part of the present invention, is a method for detecting at least one target nucleic acid in a sample. The method comprises a first step of contacting the sample with at least one probe capable of recognizing and binding a first specific region of the at least one target molecule in which the at least one probe comprises a target binding domain and a barcode domain in which the target binding domain comprises at least four nucleotides, preferably six or more nucleotides, and is capable of recognizing and binding the first specific region of the target nucleic acid and in which the target binding domain comprises a known nucleotide sequence; in which the barcode domain comprises a barcode domain comprising a first attachment region comprising a nucleic acid sequence capable of being bound by a first complementary nucleic acid molecule , a first complementary nucleic acid molecule of a first reporter complex or a first hybridizing nucleic acid molecule and an at least second attachment region comprising a nucleic acid sequence capable of being bound by an at least second complementary nucleic acid molecule, an at least second complementary nucleic acid molecule of an at least second reporter complex or an at least second hybridizing nucleic acid molecule, in which the sequence of the first attachment region is different from the sequence of the at least second attachment region. The method comprises further steps of (2) binding to the first attachment region a first complementary nucleic acid molecule comprising a detectable label or a first complementary nucleic acid molecule of a first reporter complex comprising a detectable label, thereby associating a detectable label with the first attachment region; (3) detecting the detectable label associated with the first attachment region; (4) removing the first detectable label or first complementary nucleic acid molecule; (5) binding to the at least second attachment region an at least second complementary nucleic acid molecule comprising a detectable label or an at least second complementary nucleic acid molecule of an at least second reporter complex comprising a detectable label, thereby associating a detectable label with the at least second attachment region; and (6) detecting the detectable label associated with the at least second attachment region; in which the linear or sequential order of the detectable labels associated with the first attachment region and the detectable label associated with the at least second attachment region identifies the specific region of the at least one target molecule, thereby detecting the at least one target nucleic acid in the sample. Steps (4) and (5) may occur sequentially or concurrently.

In embodiments, removal of the first complementary nucleic acid in step (4) comprises contacting the first attachment region with a first hybridizing nucleic acid molecule lacking a detectable label thereby unbinding the first complementary nucleic acid molecule and binding to the first attachment region the first hybridizing nucleic acid molecule lacking a detectable label, or a change in pH, salt concentration, and/or temperature sufficient to remove the first complementary nucleic acid molecule.

In embodiments, the barcode domain may comprise an at least third attachment region comprising a nucleic acid sequence capable of being bound by an at least third complementary nucleic acid molecule, an at least third complementary nucleic acid molecule of an at least third reporter complex or an at least third hybridizing nucleic acid molecule, in which the sequence of the at least third attachment region is different from the sequence of another attachment region.

In embodiments, the method may further comprise steps of (7) removing the second detectable label or second complementary nucleic acid molecule; (8) binding to the at least third attachment region an at least third complementary nucleic acid molecule comprising a detectable label or an at least third complementary nucleic acid molecule of an at least third reporter complex comprising a detectable label, thereby associating a detectable label with the at least third attachment region; and (9) detecting the detectable label associated with the at least third attachment region, in which the linear or sequential order of the detectable label associated with the first attachment region, the detectable label associated with the at least second attachment region, and the detectable label associated with the at least third attachment region identifies the specific region of the at least one target molecule, thereby detecting the at least one target nucleic acid in the sample. Steps (7) and (8) may occur sequentially or concurrently.

In embodiments, removal of the second complementary nucleic acid in step (7) comprises contacting the second attachment region with a second hybridizing nucleic acid molecule lacking a detectable label thereby unbinding the second complementary nucleic acid molecule and binding to the second attachment region the second hybridizing nucleic acid molecule lacking a detectable label, or a change in pH, salt concentration, and/or temperature sufficient to remove the second complementary nucleic acid molecule.

In embodiments, the barcode domain may comprise an at least fourth attachment region comprising a nucleic acid sequence capable of being bound by an at least fourth complementary nucleic acid molecule, an at least fourth reporter complex or an at least fourth hybridizing nucleic acid molecule, in which the sequence of the at least fourth attachment region is different from the sequence of another attachment region. In embodiments, the barcode domain may comprise an at least fifth attachment region comprising a nucleic acid sequence capable of being bound by an at least fifth complementary nucleic acid molecule, an at least fifth reporter complex or an at least fifth hybridizing nucleic acid molecule, in which the sequence of the at least fifth attachment region is different from the sequence of another attachment region. In embodiments, the barcode domain may comprise an at least sixth attachment region comprising a nucleic acid sequence capable of being bound by an at least sixth complementary nucleic acid molecule, an at least sixth reporter complex or an at least sixth hybridizing nucleic acid molecule, in which the sequence of the at least sixth attachment region is different from the sequence of another attachment region. In embodiments, the barcode domain may comprise an at least seventh attachment region comprising a nucleic acid sequence capable of being bound by an at least seventh complementary nucleic acid molecule, an at least seventh reporter complex or an at least seventh hybridizing nucleic acid molecule, in which the sequence of the at least seventh attachment region is different from the sequence of another attachment region.

In embodiments, the steps of removing the respective detectable label or complementary nucleic acid molecule; binding to the respective attachment region a complementary nucleic acid molecule comprising a detectable label or a complementary nucleic acid molecule of a reporter complex comprising a detectable label, thereby associating a detectable label with the respective attachment region; and detecting the respective detectable label associated with the attachment region are repeated until each attachment region in the barcode domain has been sequentially bound by a complementary nucleic acid molecule comprising a detectable label and the detectable label of the sequentially bound complementary nucleic acid molecule has been detected, in which the linear or sequential order of the detectable labels associated with each attachment region identifies the specific region of the at least one target molecule, thereby detecting the at least one target nucleic acid in the sample.

In embodiments, the first hybridizing nucleic acid molecule lacking a detectable label comprises at least the nucleic acid sequence of the first complementary nucleic acid molecule.

In embodiments, the first attachment region may be adjacent to at least one flanking single-stranded polynucleotide or polynucleotide analogue. The first hybridizing nucleic acid molecule lacking a detectable label may further comprise a nucleic acid sequence partially complementary to the at least one flanking single-stranded polynucleotide adjacent to said first attachment region.

In embodiments, the at least second hybridizing nucleic acid molecule lacking a detectable label comprises at least the nucleic acid sequence of the at least second complementary nucleic acid molecule.

In embodiments, the at least second attachment region may be adjacent to at least one flanking single-stranded polynucleotide or polynucleotide analogue. The at least second hybridizing nucleic acid molecule lacking a detectable label may comprise a nucleic acid sequence partially complementary to the at least one flanking single-stranded polynucleotide adjacent to the at least second attachment region.

In embodiments, the barcode domain may comprise a synthetic backbone comprising a polysaccharide, a peptide, a peptide nucleic acid, a polypeptide, or a polynucleotide selected from single stranded-stranded DNA, single-stranded RNA, or single-stranded PNA.

In embodiments, the at least one probe may comprise a single-stranded or double-stranded RNA, DNA, PNA, or other polynucleotide analogue or PEG spacer between the target binding domain and the barcode domain. The spacer may be a double-stranded DNA.

In embodiments, the first complementary nucleic acid molecule of a first reporter complex, at least second complementary nucleic acid molecule and at least second complementary nucleic acid molecule of an at least second reporter complex are independently RNA, DNA, PNA, or other polynucleotide analogue.

In embodiments, the at least third complementary nucleic acid or at least third complementary nucleic acid of a third reporter complex may be RNA, DNA, PNA, or other polynucleotide analogue.

In embodiments, the at least one nucleotide in said target binding domain may be a modified nucleotide or a nucleic acid analogue. At least two, at least three, at least four, at least five or at least six nucleotides in said target binding domain may be a modified nucleotide or a nucleic acid analogue. Each nucleotide in said target binding domain may a modified nucleotide or a nucleic acid analogue. The at least one modified nucleotide or the at least one nucleic acid analogue may be a locked nucleic acid (LNA). The least one modified nucleotide or the at least one nucleic acid analogue may comprise a universal base.

In embodiments, the target nucleic acid may be first immobilized to a substrate by at least binding a first position of the target nucleic acid with a first capture probe that comprises a first affinity binding reagent that selectively binds to the substrate. In embodiments, the target nucleic acid is immobilized to a substrate after binding to the probe by at least binding a first position of the target nucleic acid with a first capture probe that comprises a first binding affinity reagent that selectively binds to the substrate. In embodiments, the first capture probe binds the target nucleic acid at a different position on the target nucleic acid than the at least one probe binds to the target nucleic acid. The target nucleic acid may be elongated by applying a force (e.g., gravity, hydrodynamic force, electromagnetic force, flow-stretching, a receding meniscus technique, or a combination thereof) sufficient to extend the target nucleic acid that is immobilized to the substrate at a first position. The target nucleic acid may be further immobilized to the substrate by binding an at least second position of the target nucleic acid with an at least second capture probe that comprises an affinity binding reagent that selectively binds to the substrate. Typically, the second capture probe binds the target nucleic acid at a different position on the target nucleic acid than the at least one probe and first capture probe binds to the target nucleic acid. The target nucleic acid may be further immobilized to the substrate by binding an at least a portion of the probe or a portion of a complementary nucleic acid molecule or a reporter complex with an at least third capture probe that comprises a third affinity binding reagent that selectively binds to the substrate. The target nucleic acid may be further immobilized to the substrate by binding a portion of the probe, a portion of the at least one complementary nucleic acid molecule or at least one reporter complex to the substrate via a fourth affinity binding reagent. Typical affinity binding reagents include ligands, antigens, carbohydrates, receptors, lectins, antibodies, biotin, avidin, haptens, and nucleic acids having a known sequence. The target nucleic acid may be immobilized to the substrate at about three to at least ten positions. The force can be removed once a second position of the target nucleic acid is immobilized to the substrate. In embodiments, the immobilized target nucleic acid is elongated.

In embodiments, the first capture probe may comprise a second affinity reagent.

In embodiments, the second affinity reagent of the first capture probe is different from the first affinity reagent of the at least one probe.

In embodiments, the first capture probe may further comprise a third affinity reagent that is different from the second affinity reagent.

In embodiments, the first affinity reagent, the second affinity reagent, and the third affinity reagent are different.

In embodiments, the number of nucleotides in a target binding domain equals the number of different attachment regions in the barcode domain.

In embodiments, the number of nucleotides in a target binding domain may be at least one more than the number of different attachment regions in the barcode domain.

In embodiments, the number of nucleotides in a target binding domain is at least twice the number of attachment regions in the barcode domain.

In embodiments, the number of nucleotides in a target binding domain is eight and the number of attachment regions in the barcode domain is three.

In embodiments, the number of nucleotides in a target binding domain may be at least one less than the number of different attachment regions in the barcode domain.

In embodiments, the target binding domain of the probe comprises at least 6 nucleotides, or at least 8 nucleotides.

In embodiments, the target binding domain of the probe comprises 10-100, 20-60 or 3550 nucleotides.

In embodiments, at least the first attachment region branches from a first position on the barcode domain. In embodiments, the at least second attachment region branches from an at least second position on the barcode domain. In embodiments, each attachment region branches from a position on the barcode domain. The barcode domain may comprise a first position comprising at least two first attachment regions, in which the at least two first attachment regions comprise an identical nucleic acid sequence that is capable of being bound by a first complementary nucleic acid molecule or a first complementary nucleic acid molecule of a first reporter complex. The barcode domain may comprise an at least second position comprising two at least second attachment regions, in which the at least two second attachment regions comprise an identical nucleic acid sequence that is capable of being bound by an at least second complementary nucleic acid molecule or a second complementary nucleic acid molecule of a second reporter complex. The barcode domain may comprise an at least third position comprising two at least third attachment regions, in which the at least two third attachment regions comprise an identical nucleic acid sequence that is capable of being bound by an at least third complementary nucleic acid molecule or a third complementary nucleic acid molecule of a third reporter complex.

In embodiments, each position in a barcode domain may comprise the same number of attachment regions. In embodiments, at least one position in a barcode domain may comprise more than one attachment region. Each position in a barcode domain may comprise more than one attachment region.

In embodiments, at least one position in a barcode domain may comprise a greater number of attachment regions than another position.

In embodiments, at least one position on a barcode domain may comprise one to fifty copies of its attachment region, e.g., each position on a barcode domain may comprise one to fifty copies of its attachment region.

In embodiments, the at least one probe may include multiple copies of the target binding domain operably linked to a barcode domain.

In embodiments, each reporter complex comprising a detectable label may comprise a complementary nucleic acid molecule directly linked to a primary nucleic acid molecule.

In embodiments, each reporter complex comprising a detectable label may comprise a complementary nucleic acid molecule indirectly linked to a primary nucleic acid molecule via a nucleic acid spacer.

In embodiments, each reporter complex comprising a detectable label may comprise a complementary nucleic acid molecule indirectly linked to a primary nucleic acid molecule via a polymeric spacer with a similar mechanical properties as a nucleic acid spacer.

In embodiments, each reporter complex comprising a detectable label includes a complementary nucleic acid molecule indirectly linked to a primary nucleic acid molecule via a cleavable linker.

In embodiments, the cleavable linker is photo-cleavable, chemically cleavable or enzymatically cleavable. Typically, each cleavable linker is independently cleavable from all other linkers.

In embodiments, the photo-cleavable linker is cleaved by a light source such as an arc-lamp, a laser, a focused UV light source or light emitting diode.

In embodiments, each complementary nucleic acid molecule may comprise between about 8 nucleotides and about 20 nucleotides, e.g., about 10 nucleotides, about 12 nucleotides, and about 14 nucleotides.

In embodiments, each primary nucleic acid molecule may be hybridized to at least one secondary nucleic acid molecule, e.g., at least two secondary nucleic acid molecules, at least three secondary nucleic acid molecules, at least four secondary nucleic acid molecules, at least five secondary nucleic acid molecules, and at least six secondary nucleic acid molecules. The secondary nucleic acid molecule or molecules may include at least one detectable label.

In embodiments, the secondary nucleic acid molecules may include a cleavable linker. For example, the cleavable linker is photo-cleavable, chemically cleavable or enzymatically cleavable. In embodiments, the various secondary nucleic acid molecules hybridized to a primary nucleic acid molecule may all include the same cleavable linker, no cleavable linker, combinations of various cleavable linkers or combinations of various cleavable linkers and no cleavable linker.

In embodiments, each secondary nucleic acid molecule may be hybridized to at least one tertiary nucleic acid molecule comprising at least one detectable label, e.g., at least two, at least three, at least four, at least five, at least six, or at least seven tertiary nucleic acid molecules comprising at least one detectable label.

In embodiments, at least one secondary nucleic acid molecule may comprise a region that does not hybridize to a primary nucleic acid molecule and does not hybridize to a tertiary nucleic acid molecule. In embodiments, the each secondary nucleic acid molecule may comprise a region that does not hybridize to a primary nucleic acid molecule and does not hybridize to a tertiary nucleic acid molecule. The region that does not hybridize to a primary nucleic acid molecule and does not hybridize to a tertiary nucleic acid molecule may comprise the nucleotide sequence of the complementary nucleic acid molecule that is directly linked to the primary nucleic acid molecule. The region that does not hybridize to a primary nucleic acid molecule and does not hybridize to a tertiary nucleic acid molecule may be located at a terminus of the secondary nucleic acid molecule. The region that does not hybridize to a primary nucleic acid molecule and does not hybridize to a tertiary nucleic acid molecule may comprise between about 8 nucleotides and about 20 nucleotides, e.g., about 12 nucleotides.

In embodiments, the at least one target nucleic acids may comprise 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150, 200, 300, 400, 500, 600, 700, 800, 900, 1000, 2000, 3000, 4000, 5000, or more and any number of different target nucleic acids in between.

In embodiments, the method may further comprise detecting at least one target protein in the sample.

In embodiments, the at least one target protein may comprise 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150, 200, 300, 400, 500, 600, 700, 800, 900, 1000, 2000, 3000, 4000, 5000, or more, and any number of different target proteins in between.

The terms "one or more", "at least one", and the like are understood to include but not be limited to at least 1, 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150, 200, 300, 400, 500, 600, 700, 800, 900, 1000, 2000, 3000, 4000, 5000 or more and any number in between.

The terms "plurality", "at least two", "two or more", "at least second", and the like, are understood to include but not limited to at least 2, 3, 4, 5, 6, 7, 8, 9, 10, 11, 12, 13, 14, 15, 16, 17, 18, 19 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149 or 150, 200, 300, 400, 500, 600, 700, 800, 900, 1000, 2000, 3000, 4000, 5000 or more and any number in between. Thus, "at least two label attachment positions" includes, but is not limited, two label attachment positions, four label attachment positions, six label attachment positions, eight label attachment positions, ten label attachment positions, or more.

The present disclosure, that is not part of the present invention, also provides a method for detecting at least one target nucleic acid in a sample comprising: (1) contacting the sample with at least one probe capable of recognizing and binding a first specific region of the at least one target molecule, wherein the at least one probe comprises: a target binding domain and a barcode domain, wherein the target binding domain comprises at least four nucleotides and is capable of recognizing and binding the first specific region of the target nucleic acid and wherein the target binding domain comprises a known nucleotide sequence; wherein the barcode domain comprises a barcode domain comprising a first attachment region comprising a nucleic acid sequence bound by a first complementary nucleic acid molecule or a first complementary nucleic acid molecule of a first reporter complex and an at least second attachment region bound by an at least second complementary nucleic acid molecule or an at least second complementary nucleic acid molecule of an at least second reporter complex; wherein the first complementary nucleic acid molecule or first complementary nucleic acid molecule of a first reporter complex comprises a first detectable label thereby associating a detectable label with the first attachment region; wherein the at least second complementary nucleic acid molecule or at least second complementary nucleic acid molecule of an at least second reporter complex comprises a second detectable label thereby associating a detectable label with the at least second attachment region; wherein the sequence of the first attachment region is different from the sequence of the at least second attachment region; (2) detecting the first detectable label associated with the first attachment region and the second detectable label associated with the at least second attachment region; (3) removing the first detectable label; (4) detecting the second detectable label associated with the at least second attachment region; wherein the linear or sequential order of the first detectable label associated with the first attachment region and the second detectable label associated with the at least second attachment region identifies the specific region of the at least one target molecule, thereby detecting the at least one target nucleic acid in the sample.

The detecting in step (4) can comprise subtracting a signal from second detectable label associated with the at least second attachment region in step (4) form a signal from detecting the first detectable label associated with the first attachment region and the second detectable label associated with the at least second attachment region in step (2).

The barcode domain can comprise a first attachment region comprising a nucleic acid sequence bound by a first complementary nucleic acid molecule or a first complementary nucleic acid molecule of a first reporter complex, an at least second attachment region bound by an at least second complementary nucleic acid molecule or an at least second complementary nucleic acid molecule of an at least second reporter complex and an at least third attachment region bound by an at least third complementary nucleic acid molecule or an at least third complementary nucleic acid molecule of an at least third reporter complex; wherein the first complementary nucleic acid molecule or the first complementary nucleic acid molecule of a first reporter complex comprises a first detectable label thereby associating a detectable label with the first attachment region; wherein the second complementary nucleic acid molecule or the second complementary nucleic acid molecule of an at least second reporter complex comprises a second detectable label thereby associating a detectable label with the at least second attachment region; wherein the third complementary nucleic acid molecule or the third complementary nucleic acid molecule of an at least third reporter complex comprises a third detectable label thereby associating a detectable label with the at least third attachment region; wherein the sequences of the at least third attachment region, at least second attachment region and at least third attachment region are different; (2) detecting the first detectable label associated with the first attachment region, the second detectable label associated with the at least second attachment region and the at least third detectable label associated with the third attachment region; (3) removing the first detectable label; (4) detecting the second detectable label associated with the at least second attachment region and the at least third detectable label associated with the third attachment region; (5) removing the second detectable label; (6) detecting the third detectable label associated with the at least third attachment region; wherein the linear or sequential order of the first detectable label associated with the first attachment region, the second detectable label associated with the at least second attachment region and the at least third detectable label associated with the third attachment region identifies the specific region of the at least one target molecule, thereby detecting the at least one target nucleic acid in the sample.

The detecting in step (4) can comprise subtracting a signal from the second detectable label associated with the at least second attachment region and the at least third detectable label associated with the third attachment region in step (4) form the signal from detecting the first detectable label associated with the first attachment region, the second detectable label associated with the at least second attachment region and the at least third detectable label associated with the third attachment region in step (2).

The detecting in step (6) can comprise subtracting a signal from the at least third detectable label associated with the third attachment region in step (6) form the signal from detecting the second detectable label associated with the at least second attachment region and the at least third detectable label associated with the third attachment region in step (4).

The present disclosure, that is not part of the present invention, also provides a method for detecting at least one target nucleic acid in a sample comprising: (1) contacting the sample with at least one probe capable of recognizing and binding a first specific region of the at least one target molecule, wherein the at least one probe comprises: a target binding domain and a barcode domain, wherein the target binding domain comprises at least four nucleotides and is capable of recognizing and binding the first specific region of the target nucleic acid and wherein the target binding domain comprises a known nucleotide sequence; wherein the barcode domain comprises a first attachment region comprising a nucleic acid sequence capable of being bound by a first complementary nucleic acid molecule, a first complementary nucleic acid molecule of a first reporter complex or a first hybridizing nucleic acid molecule and an at least second attachment region comprising a nucleic acid sequence capable of being bound by an at least second complementary nucleic acid molecule, an at least second complementary nucleic acid molecule of an at least second reporter complex or an at least second hybridizing nucleic acid molecule; wherein the sequence of the first attachment region is different from the sequence of the at least second attachment region; (2) binding to the first attachment region a first complementary nucleic acid molecule comprising a first detectable label or a first complementary nucleic acid molecule of a first reporter complex comprising a first detectable label, thereby associating a detectable label with the first attachment region; (3) detecting the first detectable label associated with the first attachment region; (4) removing the first detectable label or first complementary nucleic acid molecule; (5) binding to the at least second attachment region an at least second complementary nucleic acid molecule comprising a second detectable label or an at least second complementary nucleic acid molecule of an at least second reporter complex comprising a second detectable label, thereby associating a detectable label with the at least second attachment region; and (6) detecting the second detectable label associated with the at least second attachment region; wherein the linear or sequential order of the first detectable label associated with the first attachment region and the second detectable label associated with the at least second attachment region identifies the specific region of the at least one target molecule, thereby detecting the at least one target nucleic acid in the sample. Steps (4) and (5) can occur sequentially or concurrently.

The barcode domain can comprise an at least third attachment region comprising a nucleic acid sequence capable of being bound by an at least third complementary nucleic acid molecule, an at least third reporter complex or an at least third hybridizing nucleic acid molecule; wherein the sequence of the at least third attachment region is different from the sequence of another attachment region.

The method can further comprise: (7) removing the second detectable label or second complementary nucleic acid molecule; (8) binding to the at least third attachment region an at least third complementary nucleic acid molecule comprising a third detectable label or an at least third complementary nucleic acid molecule of an at least third reporter complex comprising a third detectable label, thereby associating a detectable label with the at least third attachment region; and (9) detecting the third detectable label associated with the at least third attachment region; wherein the linear or sequential order of the first detectable label associated with the first attachment region, the second detectable label associated with the at least second attachment region, and the third detectable label associated with the at least third attachment region identifies the specific region of the at least one target molecule, thereby detecting the at least one target nucleic acid in the sample. Steps (7) and (8) occur sequentially or concurrently.

The removal of the first complementary nucleic acid in step (4) can comprise: (a) contacting the first attachment region with a first hybridizing nucleic acid molecule lacking a detectable label thereby unbinding the first complementary nucleic acid molecule and binding to the first attachment region the first hybridizing nucleic acid molecule lacking a detectable label, (b) a change in pH, salt concentration, and/or temperature sufficient to remove the first complementary nucleic acid molecule.

The removal of the second complementary nucleic acid in step (7) can comprise: (a) contacting the second attachment region with a second hybridizing nucleic acid molecule lacking a detectable label thereby unbinding the second complementary nucleic acid molecule and binding to the second attachment region the second hybridizing nucleic acid molecule lacking a detectable label, (b) a change in pH, salt concentration, and/or temperature sufficient to remove the second complementary nucleic acid molecule.

The barcode domain can comprise an at least fourth attachment region comprising a nucleic acid sequence capable of being bound by an at least fourth complementary nucleic acid molecule, an at least fourth reporter complex or an at least fourth hybridizing nucleic acid molecule; wherein the sequence of the at least fourth attachment region is different from the sequence of another attachment region.

The barcode domain can comprise an at least fifth attachment region comprising a nucleic acid sequence capable of being bound by an at least fifth complementary nucleic acid molecule, an at least fifth reporter complex or an at least fifth hybridizing nucleic acid molecule; wherein the sequence of the at least fifth attachment region is different from the sequence of another attachment region.

The barcode domain can comprise an at least sixth attachment region comprising a nucleic acid sequence capable of being bound by an at least sixth complementary nucleic acid molecule, an at least sixth reporter complex or an at least sixth hybridizing nucleic acid molecule; wherein the sequence of the at least sixth attachment region is different from the sequence of another attachment region.

The barcode domain can comprise an at least seventh attachment region comprising a nucleic acid sequence capable of being bound by an at least seventh complementary nucleic acid molecule, an at least seventh reporter complex or an at least seventh hybridizing nucleic acid molecule; wherein the sequence of the at least seventh attachment region is different from the sequence of another attachment region.

The steps of: (a) removing the respective detectable label or complementary nucleic acid molecule; (b) binding to the respective attachment region a complementary nucleic acid molecule comprising a detectable label or a complementary nucleic acid molecule of a reporter complex comprising a detectable label, thereby associating a detectable label with the respective attachment region; and (c) detecting the respective detectable label associated with the attachment region; are repeated until each attachment region in the barcode domain has been sequentially bound by a complementary nucleic acid molecule comprising a detectable label and the detectable label of the sequentially bound complementary nucleic acid molecule has been detected, wherein the linear or sequential order of the detectable labels associated with each attachment region identifies the specific region of the at least one target molecule, thereby detecting the at least one target nucleic acid in the sample.

The first hybridizing nucleic acid molecule lacking a detectable label can comprise at least the nucleic acid sequence of the first complementary nucleic acid molecule.

The first attachment region can be adjacent to at least one flanking single-stranded polynucleotide or polynucleotide analogue.

The first hybridizing nucleic acid molecule lacking a detectable label further can comprise a nucleic acid sequence partially complementary to the at least one flanking single-stranded polynucleotide adjacent to said first attachment region.

The at least second hybridizing nucleic acid molecule lacking a detectable label can comprise at least the nucleic acid sequence of the at least second complementary nucleic acid molecule.

The at least second attachment region can be adjacent to at least one flanking single-stranded polynucleotide or polynucleotide analogue.

The at least second hybridizing nucleic acid molecule lacking a detectable label can comprise a nucleic acid sequence partially complementary to the at least one flanking single-stranded polynucleotide adjacent to the at least second attachment region.

Removal of the first detectable label in step (3) can comprise contacting the first complementary nucleic acid molecule or the first complementary nucleic acid molecule of a first reporter complex with a force to a location of the first complementary nucleic acid molecule sufficient to release the first detectable label.

Removal of the second detectable label in step (5) can comprise contacting the second complementary nucleic acid molecule or the second complementary nucleic acid molecule of an at least second reporter complex with a force to a location of the second complementary nucleic acid molecule sufficient to release the second detectable label.

Removal of the first detectable label in step (4) can comprise contacting the first complementary nucleic acid molecule or the first complementary nucleic acid molecule of an at least first reporter complex with a force to a location of the first complementary nucleic acid molecule sufficient to release the first detectable label.

Removal of the second detectable label in step (7) can comprise contacting the second complementary nucleic acid molecule or the second complementary nucleic acid molecule of an at least second reporter complex with a force to a location of the second complementary nucleic acid molecule sufficient to release the second detectable label.

At least one of the first complementary nucleic acid molecule, first complementary nucleic acid molecule of a first reporter complex, at least second complementary nucleic acid molecule, at least second complementary nucleic acid molecule of an at least second reporter complex, at least third complementary nucleic acid molecule or at least third complementary nucleic acid molecule of an at least third reporter complex can comprise at least one cleavable linker.

The at least one cleavable linker can be independently selected from the group photo-cleavable, chemically cleavable and enzymatically cleavable. Each cleavable linker can be independently cleavable from all other linkers. The photo-cleavable linker can be cleaved by a light source selected from the group consisting of an arc-lamp, a laser, a focused UV light source, and light emitting diode. The force can be light.

The method of the present disclosure can further comprise washing the probe from the at least one target nucleic acid. The washing can comprise a change in pH, salt concentration, and/or temperature sufficient to remove the probe from the target molecule.

The methods of the present disclosure can further comprise: (i) contacting the sample with at least a second probe capable of recognizing and binding a second specific region of the at least one target molecule, wherein the second specific region is different from the first specific region of the at least one target molecule; (ii) contacting the sample with an at least second copy of the first probe capable of recognizing and binding the first specific region of the at least one target molecule; or (iii) contacting the sample with an at least third probe capable of recognizing and binding a first specific region of an at least second target molecule, wherein the at least second target molecule is different from the at least one target molecule; wherein the probe comprises: a target binding domain and a barcode domain, wherein the target binding domain comprises at least four nucleotides; and, wherein the barcode domain comprises a barcode domain comprising a first attachment region comprising a nucleic acid sequence bound by a first complementary nucleic acid molecule or a first complementary nucleic acid molecule of a first reporter complex and an at least second attachment region bound by an at least second complementary nucleic acid molecule or an at least second complementary nucleic acid molecule of an at least second reporter complex.

The methods of the present disclosure can further comprise: (i) contacting the sample with at least a second probe capable of recognizing and binding a second specific region of the at least one target molecule, wherein the second specific region is different from the first specific region of the at least one target molecule; (ii) contacting the sample with an at least second copy of the first probe capable of recognizing and binding the first specific region of the at least one target molecule; or (iii) contacting the sample with an at least third probe capable of recognizing and binding a first specific region of an at least second target molecule, wherein the at least second target molecule is different from the at least one target molecule; wherein the probe comprises: a target binding domain and a barcode domain, wherein the target binding domain comprises at least four nucleotides; and, wherein the barcode domain comprises a first attachment region comprising a nucleic acid sequence capable of being bound by a first complementary nucleic acid molecule, a first complementary nucleic acid molecule of a first reporter complex or a first hybridizing nucleic acid molecule and an at least second attachment region comprising a nucleic acid sequence capable of being bound by an at least second complementary nucleic acid molecule, an at least second complementary nucleic acid molecule of an at least second reporter complex or an at least second hybridizing nucleic acid molecule.

The method can further comprise repeating steps (1) to (6) of claim 3 with the at least second probe, the at least second copy of the first probe, or the at least third probe. The method can further comprise repeating steps (1) to (9) with the at least second probe, the at least second copy of the first probe, or the at least third probe. After washing the probe from the at least one target nucleic acid, steps (1) to (6) or steps (1) to (9) can be repeated up to about fifty times.

The detectable label can comprise multiple moieties each capable of being identified by their emission spectrum. The detectable label can comprise quantum dots, fluorescent moieties, colorimetric moieties or combinations thereof. Preferably, the detectable label can comprise fluorescent moieties. The emission spectrum of each moiety can be the same or different. The emission spectrum of at least one moiety can be different than the other moieties. In a preferable aspect, the signal is an emission spectrum. In embodiments, the emission spectrum or spectra of the label is a detectable signal.

The barcode domain can comprise a synthetic backbone comprising a polysaccharide, a peptide, a peptide nucleic acid, a polypeptide, or a polynucleotide selected from single stranded-stranded DNA, single-stranded RNA, or single-stranded PNA. At least one probe can comprise a single-stranded or double-stranded RNA, DNA, PNA, or other polynucleotide analogue or PEG spacer between the target binding domain and the barcode domain. In one preferred aspect, the spacer is double-stranded DNA.

The first complementary nucleic acid, first complementary nucleic acid molecule of a first reporter complex, at least second complementary nucleic acid molecule and at least second complementary nucleic acid molecule of an at least second reporter complex can be independently RNA, DNA, PNA, or other polynucleotide analogue. The at least third complementary nucleic acid or at least third complementary nucleic acid of a third reporter complex can be RNA, DNA, PNA, or other polynucleotide analogue.

At least one nucleotide in said target binding domain can be a modified nucleotide or a nucleic acid analogue. At least two, at least three, at least four, at least five or at least six nucleotides in said target binding domain can be a modified nucleotide or a nucleic acid analogue. Each nucleotide in said target binding domain can be a modified nucleotide or a nucleic acid analogue. Each nucleotide in said target binding domain can be a modified nucleotide or a nucleic acid analogue except for the first and last nucleotides.

The at least one modified nucleotide or the at least one nucleic acid analogue can be a locked nucleic acid (LNA). The at least one modified nucleotide or the at least one nucleic acid analogue can comprise a universal base.

The target nucleic acid can be first immobilized to a substrate prior to contact by a probe, by at least binding a first position of the target nucleic acid with a first capture probe that comprises a first affinity binding reagent that selectively binds to the substrate, wherein the first capture probe binds the target nucleic acid at a different position on the target nucleic acid than the at least one probe binds to the target nucleic acid.

The target nucleic acid can immobilized to a substrate after binding to the probe by at least binding a first position of the target nucleic acid with a first capture probe that comprises a first binding affinity reagent that selectively binds to the substrate, wherein the first capture probe binds the target nucleic acid at a different position on the target nucleic acid than the at least one probe binds to the target nucleic acid.

The target nucleic acid can elongated by applying a force sufficient to extend the target nucleic acid that is immobilized to the substrate at a first position. The force can be gravity, hydrodynamic force, electromagnetic force, flow-stretching, a receding meniscus technique, or a combination thereof.

The target nucleic acid can be further immobilized to the substrate by binding an at least second position of the target nucleic acid with an at least second capture probe that comprises a second affinity binding reagent that selectively binds to the substrate, wherein the second capture probe binds the target nucleic acid at a different position on the target nucleic acid than the at least one probe and first capture probe binds to the target nucleic acid.

The target nucleic acid can be further immobilized to the substrate by binding an at least a portion of the probe or a portion of a complementary nucleic acid molecule or a reporter complex with an at least third capture probe that comprises a third affinity binding reagent that selectively binds to the substrate.

The probe, at least one complementary nucleic acid or at least one reporter complex can comprise a fourth affinity binding reagent.

The target nucleic acid can be further immobilized to the substrate by binding a portion of the probe, a portion of the at least one complementary nucleic acid molecule or at least one reporter complex to the substrate via the fourth affinity binding reagent.

The force can be removed once the second position of the target nucleic acid is immobilized to the substrate.

The affinity binding reagent can be independently selected from the group consisting of a ligand, an antigen, a carbohydrate, a receptor, a lectin, an antibody, biotin, avidin, a hapten, and a nucleic acid having a known sequence.

The first capture probe can comprise a target binding domain comprising 20-60 nucleotides and wherein the first capture probe binds the target nucleic acid at a different position on the target nucleic acid than the at least one probe binds to the target nucleic acid. The first capture probe can comprise a target binding domain comprising 35-50 nucleotides.

The first affinity binding reagent can be different from the second affinity binding reagent.

At least one of the first affinity binding reagent, second affinity binding reagent, third affinity binding reagent and fourth affinity binding reagent can be different from the other affinity binding reagents.

The number of nucleotides in a target binding domain can be at least twice the number of attachment regions in the barcode domain. The number of nucleotides in a target binding domain can be 8 and the number of attachment regions in the barcode domain can be three. The target binding domain can comprise at least 6 nucleotides. The target binding domain can comprise at least 8 nucleotides. The target binding domain can comprise 10-100 nucleotides. The target binding domain can comprise 20-60 nucleotides. The target binding domain can comprise 35-50 nucleotides.

Each complementary nucleic acid molecule can comprise between about 8 nucleotides and about 20 nucleotides. Each complementary nucleic acid molecule can comprise about 12 nucleotides. Each complementary nucleic acid molecule can comprise about 14 nucleotides.

The at least the first attachment region ca branch from a first position on the barcode domain. The at least second attachment region can branch from an at least second position on the barcode domain. Each attachment region can branch from a position on the barcode domain.

The barcode domain can comprise a first position comprising at least two first attachment regions, wherein the at least two first attachment regions comprise an identical nucleic acid sequence that is capable of being bound by a first complementary nucleic acid molecule or a first complementary nucleic acid molecule of a first reporter complex.

The barcode domain can comprise an at least second position comprising at least two second attachment regions, wherein the at least two second attachment regions comprise an identical nucleic acid sequence that is capable of being bound by an at least second complementary nucleic acid molecule or an at least second complementary nucleic acid molecule of an at least second reporter complex.

The barcode domain can comprise an at least third position comprising at least two third attachment regions, wherein the at least two third attachment regions comprise an identical nucleic acid sequence that is capable of being bound by an at least third complementary nucleic acid molecule or an at least third complementary nucleic acid molecule of an at least third reporter complex.

Each position in a barcode domain can comprise the same number of attachment regions. At least one position in a barcode domain can comprise more than one attachment region. At least one position in a barcode domain can comprise a greater number of attachment regions than another position.

At least one probe can comprise multiple copies of the target binding domain operably linked to a barcode domain.

Each reporter complex can comprise a detectable label comprises a complementary nucleic acid molecule directly linked to a primary nucleic acid molecule. Each reporter complex can comprise a detectable label comprises a complementary nucleic acid molecule indirectly linked to a primary nucleic acid molecule via a nucleic acid spacer. Each reporter complex can comprise a detectable label comprises a complementary nucleic acid molecule indirectly linked to a primary nucleic acid molecule via a polymeric spacer with a similar mechanical properties as a nucleic acid spacer. Each reporter complex can comprise a detectable label comprises a complementary nucleic acid molecule indirectly linked to a primary nucleic acid molecule via a cleavable linker.

The cleavable linker can be independently selected from the group photo-cleavable, chemically cleavable and enzymatically cleavable. Each cleavable linker can be independently cleavable from all other linkers. The photo-cleavable linker can be cleaved by a light source selected from the group consisting of an arc-lamp, a laser, a focused UV light source, and light emitting diode.

Each primary nucleic acid molecule can be hybridized to at least one, at least two, at least three, at least four, at least five or at least six secondary nucleic acid molecules.

The secondary nucleic acid molecule or molecules can comprise at least one detectable label. Each secondary nucleic acid molecule can be hybridized to at least one, at least two, at least three, at least four, at least five, at least six or at least seven tertiary nucleic acid molecules comprising at least one detectable label. At least one secondary nucleic acid molecule can comprise a region that does not hybridize to a primary nucleic acid molecule and does not hybridize to a tertiary nucleic acid molecule. The region that does not hybridize to a primary nucleic acid molecule and does not hybridize to a tertiary nucleic acid molecule can comprise the nucleotide sequence of the complementary nucleic acid molecule that is directly linked to the primary nucleic acid molecule. The region that does not hybridize to a primary nucleic acid molecule and does not hybridize to a tertiary nucleic acid molecule can be located at a terminus of the secondary nucleic acid molecule. The region that does not hybridize to a primary nucleic acid molecule and does not hybridize to a tertiary nucleic acid molecule can comprise between about 8 nucleotides and about 20 nucleotides. The region that does not hybridize to a primary nucleic acid molecule and does not hybridize to a tertiary nucleic acid molecule can comprise about 12 nucleotides.

The present disclosure also provides a kit comprising the reagents for performing the any of the methods disclosed herein.

Any of the above aspects and embodiments can be combined with any other aspect or embodiment.

Unless otherwise defined, all technical and scientific terms used herein have the same meaning as commonly understood by one of ordinary skill in the art to which this invention belongs.

As used herein, the singular forms of a word also include the plural form of the word, unless the context clearly dictates otherwise; as examples, the terms "a," "an," and "the" are understood to be singular or plural and the term "or" is understood to be inclusive. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

BRIEF DESCRIPTION OF THE DRAWINGS

The patent or application file contains at least one drawing executed in color. Copies of this patent or patent application publication with color drawings will be provided by the Office upon request and payment of the necessary fee.

The above and further features will be more clearly appreciated from the following detailed description when taken in conjunction with the accompanying drawings.

Figure 1 shows a schematic of an exemplary probe of the present invention.
Figure 2 shows a schematic of an exemplary probe of the present invention.
Figure 3 shows a schematic of an exemplary probe of the present invention.
Figure 4 shows a schematic of an exemplary probe of the present invention.
Figure 5A illustrates a step of a method of the present invention.
Figure 5B illustrates a step of the method of the present invention begun in Figure 5A.
Figure 5C illustrates a step of the method of the present invention begun in Figure 5A.
Figure 5D illustrates a step of the method of the present invention begun in Figure 5A.
Figure 6 illustrates an example of One-Step Purification in which a probe and a capture probe are together added to a target nucleic acid, thereby forming a tripartite complex. The tripartite complex is purified by being immobilized to a substrate via the capture probe's affinity reagent.
Figure 7 illustrates another example of One-Step Purification in which a capture probe comprising a binding moiety is bound to a target nucleic acid, then the capture probe-target nucleic acid complex is immobilized to a substrate via the binding moiety and then a probe is bound to the immobilized complex.

Figure 8A illustrates an example of Multi-Step Purification. Here, a probe comprising an affinity reagent is bound to a target nucleic acid, and then a probe-target nucleic acid complex is purified via the probe's affinity reagent (not shown). Later, a capture probe comprising a binding moiety is bound to the complex to form a tripartite complex. Lastly, the tripartite complex is purified by being immobilized to a substrate via the capture probes binding moiety.

Figure 8B illustrates another example of Multi-Step Purification. Here, a probe comprising an affinity reagent is bound to a target nucleic acid, and then a probe-target nucleic acid complex is purified via the probe's affinity reagent (not shown). A capture probe, which previously has been immobilized to the substrate via its binding moiety, captures the purified probe-target nucleic acid complex, thus forming a purified and immobilized tripartite complex.

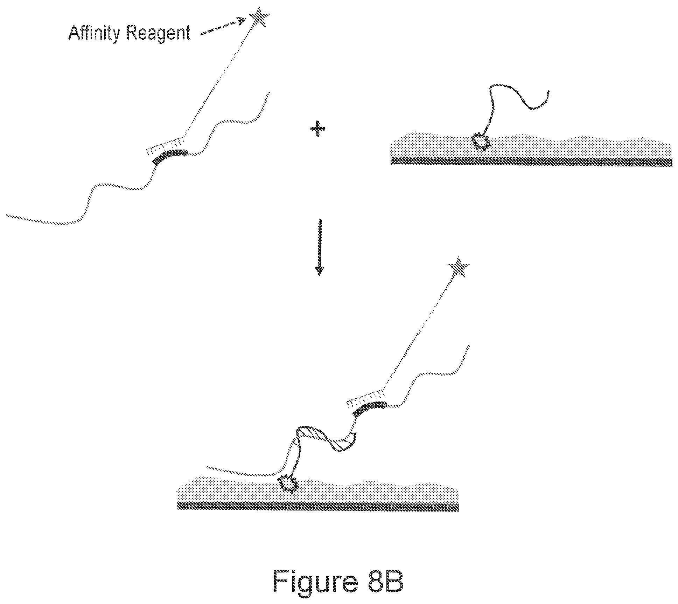

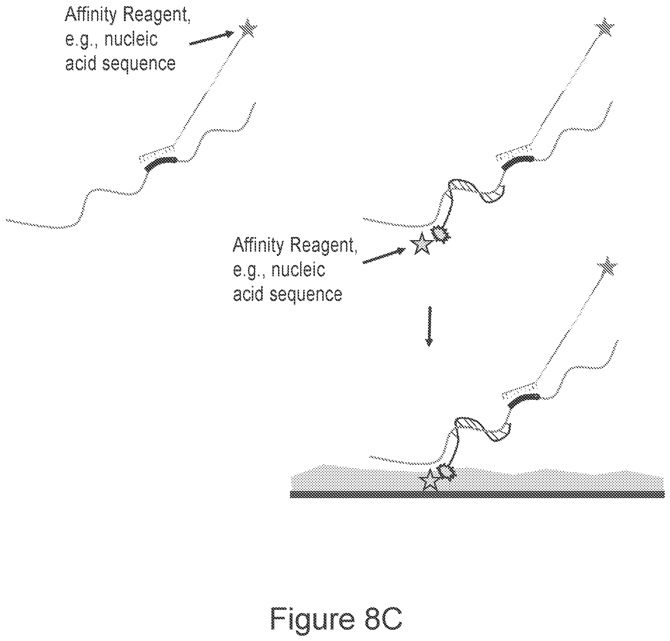

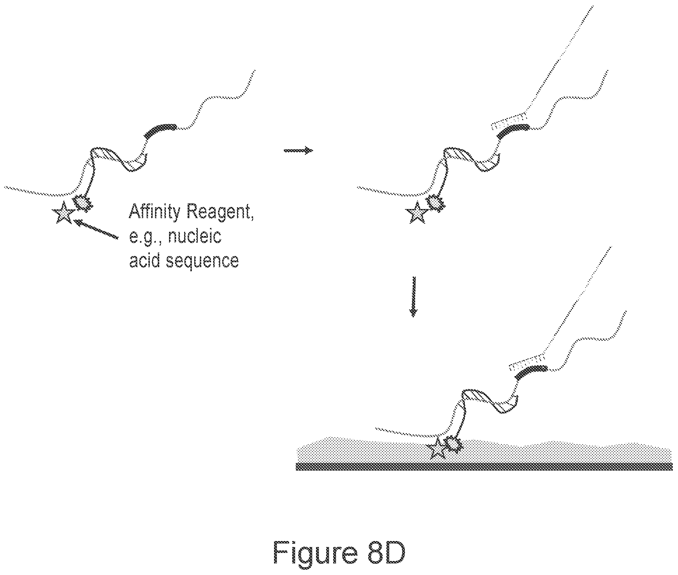

Figure 8C illustrates another example of Multi-Step Purification. Here, a probe comprising an affinity reagent is bound to a target nucleic acid, and then a probe-target nucleic acid complex is purified via the probe's affinity reagent (not shown). Later, a capture probe comprising a binding moiety and an affinity reagent (which is different from the affinity reagent on the probe) is bound to the complex to form a tripartite complex. The tripartite complex is purified via the affinity reagent on the capture probe (not shown). Lastly, the purified tripartite complex is immobilized to a substrate via the binding moiety on the capture probe.

Figure 8D illustrates another example of Multi-Step Purification. Here, a capture probe comprising a binding moiety and an affinity reagent is bound to a target nucleic acid, and then a capture probe-target nucleic acid complex is purified via the capture probe's affinity reagent (not shown). Later, a probe is bound to the complex to form a tripartite complex. Lastly, the tripartite complex is purified by being immobilized to a substrate via the binding moiety of the capture probe.

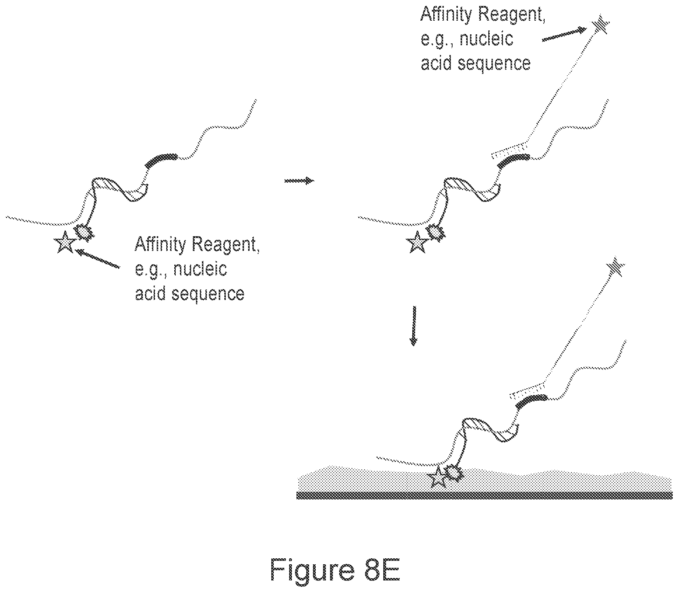

Figure 8E illustrates another example of Multi-Step Purification. Here, a capture probe comprising a binding moiety and an affinity reagent and a binding moiety is bound to a target nucleic acid, and then a capture probe-target nucleic acid complex is purified via the capture probe's affinity reagent (not shown). Later, a probe comprising an affinity reagent (which is different from the affinity reagent on the capture probe) is bound to the complex to form a tripartite complex. The tripartite complex is purified via the affinity reagent on the probe. Lastly, the purified tripartite complex is immobilized to a substrate via the binding moiety on the capture probe.

Figure 9A shows an initial step of a method of the present invention.

Figure 9B shows a schematic of a reporter complex comprising detectable labels.

Figure 9C shows a plurality of reporter complexes each comprising detectable labels.

Figure 9D shows a further step of the method begun in Figure 9A.

Figure 9E shows a further step of the method begun in Figure 9A.

Figure 9F shows a further step of the method begun in Figure 9A.

Figure 10 shows an alternate illustration of the steps shown in Figure 9D and Figure 9E and exemplary data obtained therefrom. The fragment of the probe shown has the sequence of SEQ ID NO: 70.

Figure 11 illustrates a variation of the method shown in Figure 10. The fragment of the probe shown likewise has the sequence of SEQ ID NO: 70.

Figure 12A shows various designs of reporter complexes of the present invention.

Figure 12B shows fluorescent counts obtained from the reporter complexes shown in Figure 12A.

Figure 12C shows exemplary recipes for constructing reporter complexes of the present invention.

Figure 13A shows designs of reporter complexes comprising "extra-handles."

Figure 13B shows fluorescent counts obtained from the reporter complexes having "extra-handles".

Figure 14A shows hybridization kinetics of two exemplary designs of reporter complexes of the present invention.

Figure 14B shows hybridization kinetics of two exemplary designs of reporter complexes of the present invention.

Figure 15A describes a small barcode probe design.

Figure 15B shows data obtained with a method of the present invention when probes are provided at a lower concentration.

Figure 15C shows data obtained with a method of the present invention when probes are provided at a higher concentration.

Figure 16A shows data obtained with a method of the present invention in which a plurality of target nucleic acids are simultaneously detected.

Figure 16B compares data obtained with the present methods and data obtained with probes comprising detectable labels.

Figure 17A demonstrates Hyb & Count capture and detection of specific DNA targets.

Figure 17B shows detection of targets in a 100plex capture panel.

Figure 18 displays the intensity distributions of the multi-color reporters.

Figure 19 shows the error rates for a 14 class model (left) and a 10 class model.

Figure 20 shows a schematic of two color reporter probes.

Figure 21 shows probe hybridization workflow for targeted capture of nucleic acids.

Figure 22 shows targeted capture of nucleic acids used for long range phasing of haplotypes.

Figure 23 is a diagram illustrating sequencing cycling using pre-complexed BC with cleavable RPTRs, also known as complementary nucleic acid molecules including a detectable label and cleavable linker.

Figure 24 is a diagram illustrating the method for identification of each RPTR using RPTR cleavage and image subtraction.

Figure 25 is a diagram of the construction of the cleavable RPTR probes and shows examples of cleavage modifications.

Figure 26 shows that incubation time of hybridization was varied and total counts per field of view were used to determine the relative efficiency of the BC/RPTR complex compared to the BC alone followed by RPTRs binding in a second step. The BC and RPTRs used are shown in Figure 27. The BC/RPTR complexes have slower binding kinetics than BC alone but can achieve similar binding efficiency with longer incubation times.

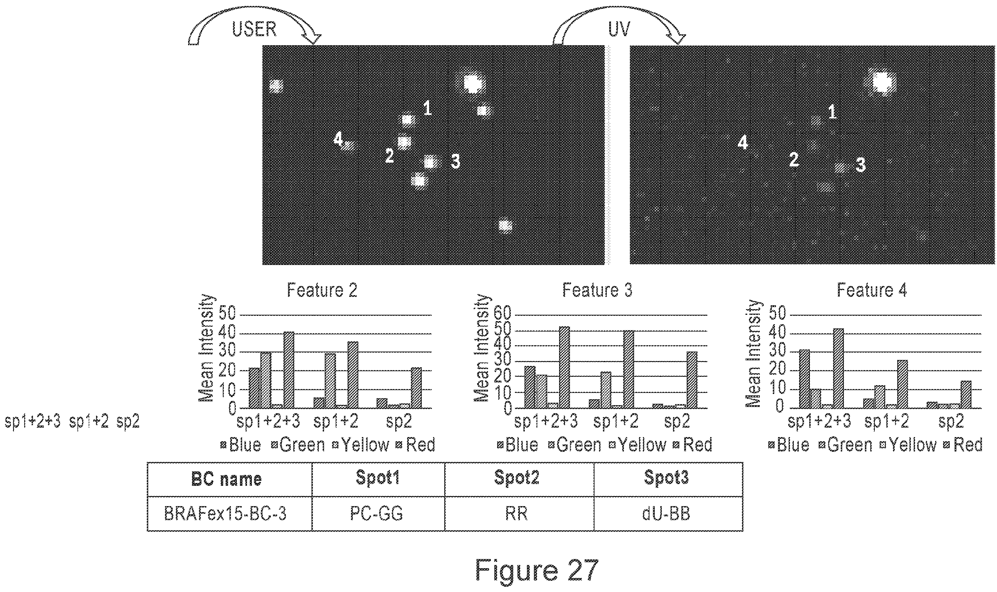

Figure 27 shows that RPTR identities can be determined using image substraction approach. The BRAFex15-BC3 barcode was precomplexed with cleavable RPTRs and processed for one full cycle. Four features are highlighted from a small portion of an image and the changes in each fluorescent channel are shown in the barplots. Cleavage was performed first for the RPTR bound to spot 3 (sp3) using USER enzyme mix, a mixture of Uracil DNA glycosylase (UDG) and DNA glycosylase-lyase Endonuclease VIII, then cleavage was performed for spot 1 (sp1) using exposure to UV light. The RPTR bound to spot 2 (sp2) was not cleavable.

Figure 28 shows the detection and correct identification of half-color GY RPTRs upon cleavage. BCs were complexed with one UV-cleavable RPTR and two non-cleavable RPTRS and hybridized to an immobilized DNA target on the surface of the flow-cell. The fluorescent intensities of the RPTRs were determined before and after UV-exposure to cleave the single RPTR to determine the accuracy/extent to which a half-color (i.e. GY instead of GG, a full color RPTR) could be detected in the presence of other reports.

Figure 29 shows the detection and correct identification of full-color GY RPTRs upon cleavage for comparison to Figure 6. BCs were complexed with one UV-cleavable RPTR and two non-cleavable RPTRS and hybridized to an immobilized DNA target on the surface of the flow-cell. The fluorescent intensities of the RPTRs were determined before and after UV-exposure to cleave the single RPTR to determine the accuracy/extent to which a full-color (i.e. GG) could be detected in the presence of other reports.

## DETAILED DESCRIPTION OF THE INVENTION

The present invention is defined in the appended claims. The disclosure provides probes, methods, kits, and apparatuses that provide accurate, rapid, and sensitive multiplexed detection, identification, and quantification of target molecules in a sample.

### Probes for detecting one or more nucleic acids in a sample

The present invention relates to a probe comprising a target binding domain and a barcode domain. The target binding domain and the barcode domain may be operably linked, e.g., covalently linked. A probe optionally comprises a spacer between the target binding domain and the barcode domain. The spacer can be any polymer with appropriate mechanical properties, for example, a single- or double-stranded DNA spacer (of 1 to 100 nucleotides, e.g., 2 to 50 nucleotides). Non-limiting examples of double-stranded DNA spacers include the sequences covered by SEQ ID NO: 25 to SEQ ID NO: 29. Additional exemplary sequences that may be included in a barcode domain are listed in SEQ ID NO: 30 to SEQ ID NO: 69.

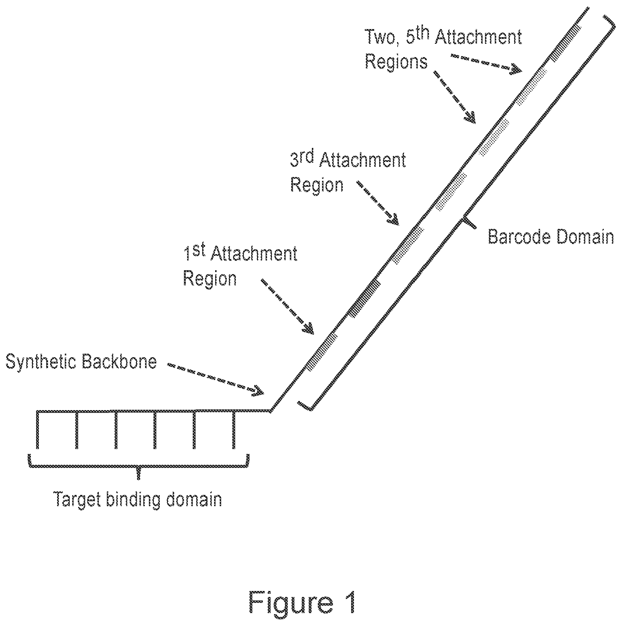

Non-limiting examples of probes of the present invention are shown in Figures 1 to 5.

Figure 1 shows a schematic of a probe of the present invention. This exemplary probe has a target binding domain of six nucleotides. The target binding domain of each probe has a known nucleotide sequence. The barcode domain comprises one or more an attachment regions; in Figure 1, there are six attachment regions. A first attachment region, a third attachment region, and fifth attachment region are noted. The fifth position comprises two attachment regions. Each position on a barcode domain can have multiple attachment regions. For example, a position may have 1 to 50 attachment regions. Certain positions in a barcode domain may have more attachment regions than other positions (as shown here in position 5 relative to positions 1 to 4 and 6); alternately, each position in a barcode domain has the same number of attachment regions. Although not shown, each attachment region comprises at least one (*i.e.,* one to fifty, *e.g.,* ten to thirty) copies of a nucleic acid sequence(s) capable of reversibly binding to a complementary nucleic acid molecule (RNA or DNA). In Figure 1, the attachment regions are integral to the linear polynucleotide molecule that makes up the barcode domain. The linear order of attachment positions and/or linear order of positions identify a specific region of a target nucleic acid to which the target binding domain binds.

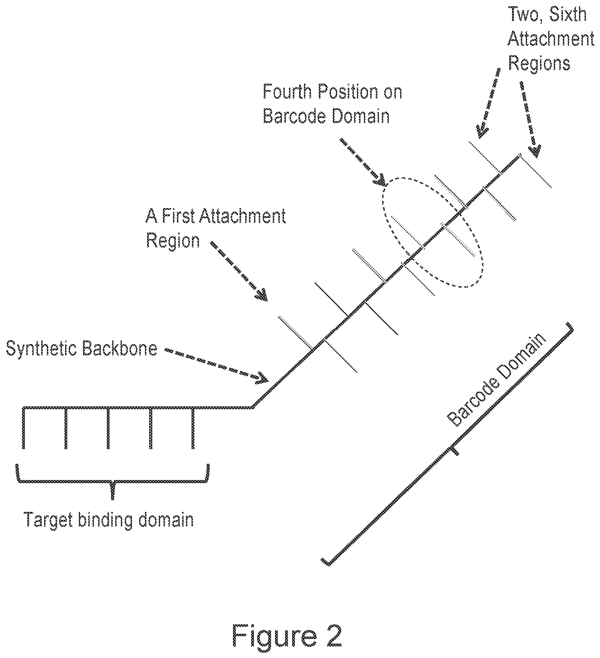

Figure 2 shows a schematic of a probe of the present invention. This exemplary probe has a target binding domain of five nucleotides. The target binding domain of each probe has a known nucleotide sequence. A first attachment region is noted; the first position on the barcode domain comprises two first attachment regions that are bound to (not integral) to the barcode domain. The fourth position on the barcode domain, which comprises a portion of the barcode domain and two fourth attachment regions are encircled. Two sixth attachments regions are noted. Here, each position has two attachment regions; however, each position on a barcode domain can have one attachment region or multiple attachment regions, e.g., 2 to 50 attachment regions. Although not shown, each attachment region comprises at least one (*i.e.*, one to fifty, *e.g.*, ten to thirty) copies of a nucleic acid sequence(s) capable of reversibly binding to a complementary nucleic acid molecule (RNA or DNA). In Figure 2, the barcode domain is a linear polynucleotide molecule to which the attachment regions are linked/branched; the attachment regions are not integral to the polynucleotide molecule. The linear order of attachment positions and/or linear order of positions identify a specific region of a target nucleic acid to which the target binding domain binds.

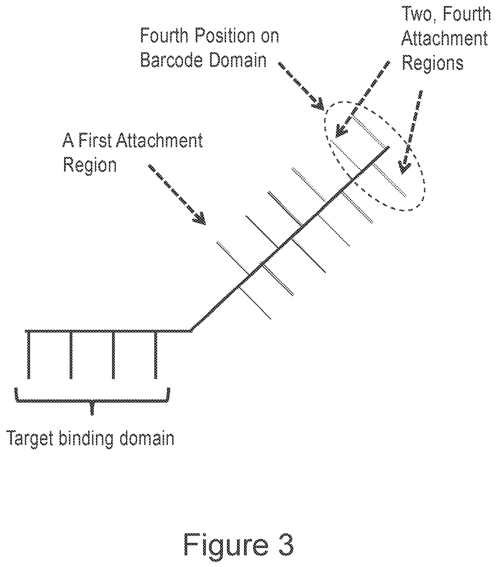

Figure 3 shows another a schematic of a probe of the present invention. This exemplary probe has a target binding domain of four nucleotides. Each position is shown with three attachment regions that are linked to/branched from the position.

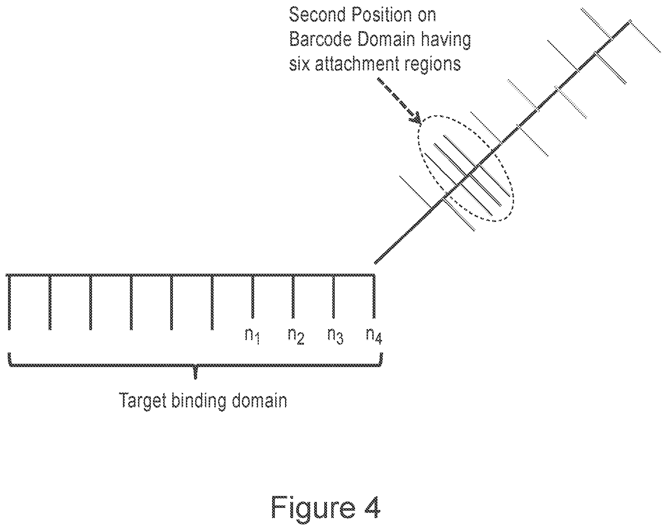

Figure 4 shows yet another schematic of a probe of the present invention. This exemplary probe has a target binding domain of ten nucleotides. However, only the first six nucleotides are specific to the target nucleic acid. The seventh to tenth nucleotides (indicated by "$n_1$ to $n_4$") are added to increase the length of the target binding domain thereby affecting the likelihood that a probe will hybridize and remain hybridized to a target nucleic acid. The "n" nucleotides may have universal bases (e.g., inosine, 2'-deoxyinosine (hypoxanthine deoxynucleotide) derivatives, nitroindole, nitroazole analogues, and hydrophobic aromatic non-hydrogen-bonding bases) which can base pair with any of the four canonical bases. In embodiments, "n" nucleotides may precede the specific nucleotides of the target binding domain. In embodiments, "n" nucleotides may follow the specific nucleotides of the target binding domain. In Figure 4, four "n" nucleotides are shown; however, a target binding domain may include more or less than four "n" nucleotides. A target binding domain may lack "n" nucleotides. The second position includes six attachment regions that are linked to/branched from the second position of the barcode domain.

The target binding domain has at least four nucleotides, e.g., at least, 4, 5, 6, 7, 8, 9, 10, 11, 12, or more nucleotides. The target binding domain can include 10-100, 20-160 or 35-50 nucleotides. The target binding domain preferably is a polynucleotide. The target binding domain is capable of binding a target nucleic acid.

A probe may include multiple copies of the target binding domain operably linked to a synthetic backbone.

Probes can be designed to control the likelihood of hybridization and/or de-hybridization and the rates at which these occur. Generally, the lower a probe's Tm, the faster and more likely that the probe will de-hybridize to/from a target nucleic acid. Thus, use of lower Tm probes will decrease the number of probes bound to a target nucleic acid.

The length of a target binding domain, in part, affects the likelihood of a probe hybridizing and remaining hybridized to a target nucleic acid. Generally, the longer (greater number of nucleotides) a target binding domain is, the less likely that a complementary sequence will be present in the target nucleotide. Conversely, the shorter a target binding domain is, the more likely that a complementary sequence will be present in the target nucleotide. For example, there is a 1/256 chance that a four-mer sequence will be located in a target nucleic acid versus a 1/4096 chance that a six-mer sequence will be located in the target nucleic acid. Consequently, a collection of shorter probes will likely bind in more locations for a given stretch of a nucleic acid when compared to a collection of longer probes.

The term "target nucleic acid" shall mean a nucleic acid molecule (DNA, RNA, or PNA) whose presence in a sample is to be determined by the probes, methods, and apparatuses of the invention. In general, the terms "target nucleic acid", "nucleic acid molecule,", "nucleic acid sequence," "nucleic acid", "nucleic acid fragment," "oligonucleotide" and "polynucleotide" are used interchangeably and are intended to include, but not limited to, a polymeric form of nucleotides that may have various lengths, either deoxyribonucleotides or ribonucleotides, or analogs thereof. Non-limiting examples of nucleic acids include a gene, a gene fragment, an exon, an intron, intergenic DNA (including, without limitation, heterochromatic DNA), messenger RNA (mRNA), transfer RNA, ribosomal RNA, ribozymes, small interfering RNA (siRNA), non-coding RNA (ncRNA), cDNA, recombinant polynucleotides, branched polynucleotides, plasmids, vectors, isolated DNA of a sequence, isolated RNA of a sequence, nucleic acid probes, and primers.

In certain specific embodiments, that target molecule is not a chromosome. In other specific embodiments, the target molecule is no greater than 1,000 kb (or 1 mb) in size, no greater than 500 kb in size, no greater than 250 kb in size, no greater than 175 kb in size, no greater than 100 kb in size, no greater than 50 kb in size, no greater than 20 kb in size, or no greater than 10 kb in size. In yet other specific embodiments, the target molecule is isolated from its cellular milieu.

The present methods identify and quantify a nucleic acid molecule obtained from a sample, *e.g.,* a sample from an organism, and, preferably, without a conversion (or amplification) step. As an example, for RNA-identifying methods, the present methods do not require conversion of an RNA molecule to a DNA molecule (*i.e.,* via synthesis of cDNA) before the RNA can be identified. Since no amplification or conversion is required, under most circumstances, a nucleic acid in the present invention will retain any unique base and/or epigenetic marker present in the nucleic acid when the nucleic acid is in the sample or when it was obtained from the sample. Such unique bases and/or epigenetic markers are lost in many methods known in the art.

The target nucleic acid can be obtained from any sample or source of nucleic acid, e.g., any cell, tissue, or organism, in vitro, chemical synthesizer, and so forth. The target nucleic acid can be obtained by any art-recognized method. In embodiments, the nucleic acid is obtained from a blood sample of a clinical subject. The nucleic acid can be extracted, isolated, or purified from the source or samples using methods and kits well known in the art.

As will be appreciated by those in the art, the sample may comprise any number of things, including, but not limited to: cells (including both primary cells and cultured cell lines), cell lysates or extracts (including but not limited to RNA extracts; purified mRNA), tissues and tissue extracts (including but not limited to RNA extracts; purified mRNA); bodily fluids (including, but not limited to, blood, urine, serum, lymph, bile, cerebrospinal fluid, interstitial fluid, aqueous or vitreous humor, colostrum, sputum, amniotic fluid, saliva, anal and vaginal secretions, perspiration and semen, a transudate, an exudate (e.g., fluid obtained from an abscess or any other site of infection or inflammation) or fluid obtained from a joint (e.g., a normal joint or a joint affected by disease such as rheumatoid arthritis, osteoarthritis, gout or septic arthritis) of virtually any organism, with mammalian samples being preferred and human samples being particularly preferred; environmental samples (including, but not limited to, air, agricultural, water and soil samples); biological warfare agent samples; research samples including extracellular fluids, extracellular supernatants from cell cultures, inclusion bodies in bacteria, cellular compartments, cellular periplasm, mitochondria compartment.

The biomolecular samples can be indirectly derived from biological specimens. For example, where the target molecule of interest is a cellular transcript, e.g., a messenger RNA, the biomolecular sample of the invention can be a sample containing cDNA produced by a reverse transcription of messenger RNA. In another example, the biomolecular sample of the invention is generated by subjecting a biological specimen to fractionation, e.g., size fractionation or membrane fractionation.

The biomolecular samples of the invention may be either "native," i.e., not subject to manipulation or treatment, or "treated," which can include any number of treatments, including exposure to candidate agents including drugs, genetic engineering (e.g. the addition or deletion of a gene).

A nucleic acid molecule comprising the target nucleic acid may be fragmented by any means known in the art. Preferably, the fragmenting is performed by an enzymatic or a mechanical means. The mechanical means may be sonication or physical shearing. The enzymatic means may be performed by digestion with nucleases (e.g., Deoxyribonuclease I (DNase I)) or one or more restriction endonucleases.

When a nucleic acid molecule comprising the target nucleic acid is an intact chromosome, steps should be taken to avoid fragmenting the chromosome.

The target nucleic acid can include natural or non-natural nucleotides, comprising modified nucleotides, as well-known in the art.

Probes of the present invention may have overall lengths (including target binding domain, barcode domain, and any optional domains) of about 20 nanometers to about 50 nanometers. A probe's backbone may a polynucleotide molecule comprising about 120 nucleotides.

The barcode domain comprises a synthetic backbone. The synthetic backbone and the target binding domain are operably linked, *e.g.,* are covalently attached or attached via a linker. The synthetic backbone can comprise any material, e.g., polysaccharide, polynucleotide, polymer, plastic, fiber, peptide, peptide nucleic acid, or polypeptide. Preferably, the synthetic backbone is rigid. In embodiments, the backbone comprises "DNA origami" of six DNA double helices (*See, e.g.,* Lin et al, "Submicrometre geometrically encoded fluorescent barcodes self-assembled from DNA." Nature Chemistry; 2012 Oct; 4(10): 832-9). A barcode can be made of DNA origami tiles (Jungmann et al, "Multiplexed 3D cellular super-resolution imaging with DNA-PAINT and Exchange-PAINT", Nature Methods, Vol. 11, No. 3, 2014).

The barcode domain comprises a plurality of positions, e.g., one, two, three, four, five, six, seven, eight, nine, ten, or more positions. The number of positions may be less than, equal to, or more than the number of nucleotides in the target binding domain. In embodiments, it is preferable to include additional nucleotides in a target binding domain than the number of positions in the backbone domain, e.g., one, two, three, four, five, six, seven, eight, nine, ten, or more nucleotides. In embodiments, the number of nucleotides in a target binding domain is at least twice the number of attachment regions in the barcode domain. In additional embodiments, the number of nucleotides in a target binding domain is 8 and the number of attachment regions in the barcode domain is three. The length of the barcode domain is not limited as long as there is sufficient space for at least four positions, as described above.

Each position in the barcode domain comprises at least one attachment region, *e.g.,* one to 50, or more, attachment regions. Certain positions in a barcode domain may have more attachment regions than other positions (e.g., a first position may have three attachment regions whereas a second position may have two attachment positions); alternately, each position in a barcode domain has the same number of attachment regions. Each attachment region comprises at least one (*i.e.,* one to fifty, *e.g.,* ten to thirty) copies of a nucleic acid sequence(s) capable of being reversibly bound by a complementary nucleic acid molecule (e.g., DNA or RNA).

Each attachment region may be linked to a modified monomer (e.g., modified nucleotide) in the synthetic backbone such that the attachment region branches from the synthetic backbone. In embodiments, the attachment regions are integral to a polynucleotide backbone; that is to say, the backbone is a single polynucleotide and the attachment regions are parts of the single polynucleotide's sequence. In embodiments, the terms "barcode domain" and "synthetic backbone" are synonymous.

For each probe, the nucleotide sequence for each attachment region in a position is identical. Thus, in the probe, each first attachment region in a first position has the same nucleotide sequence. Likewise, each ninth attachment region in a ninth position has the same nucleotide sequence.

In a probe, each attachment region or the attachment regions within a position will have a unique sequence. Also, the attachment region of a first position will include a nucleic acid sequence different from the attachment region of a second position. Thus, to a nucleic acid sequence in an attachment region in a first position there will be no binding of a complementary nucleic acid molecule that is specific to an attachment region of a second position. Also, to an attachment region in a second position, there will be no binding of a complementary nucleic acid molecule that is specific to an attachment region of a third position.

Each position on a barcode domain may include one or more (up to fifty, preferably ten to thirty) attachment regions; thus, each attachment region may bind one or more (up to fifty, preferably ten to thirty) complementary nucleic acid molecules. In an embodiment, at least one position in a barcode domain comprises more than one attachment region. In another embodiment, at least one position in a barcode domain comprises a greater number of attachment regions than another position. As examples, the probe in Figure 1 has a fifth position comprising two attachment regions and the probe in Figure 4 has a second position having six attachment regions. In embodiments, the nucleic acid sequences of attachment regions at a position are identical; thus, the complementary nucleic acid molecules that bind those attachment regions are identical.

In alternate embodiments, the nucleic acid sequences of attachment regions at a position are not identical; thus, the complementary nucleic acid molecules that bind those attachment regions are not identical, e.g., each comprises a different nucleic acid sequence and/or detectable label. Therefore, in the alternate embodiment, the combination of non-identical nucleic acid molecules (e.g., their detectable labels) attached to an attachment region together provides a code for identifying a nucleotide in the target nucleic acid.

Table 1 provides exemplary sequences, for illustration purposes only, for attachments regions for probes having up to six positions in its barcode domain and detectable labels on complementary nucleic acid that bind thereto.

Table 1:

| Position in barcode domain | Nucleic Acid Sequence (5' to 3') in Attachment Region | Detectable label of complementary nucleic acid or reporter complex comprising detectable labels | SEQ ID NO |
|---|---|---|---|
| 1 | ATACATCTAG | GFP | 1 |
| 1 | GATCTACATA | RFP | 2 |
| 1 | TTAGGTAAAG | CFP | 3 |
| 1 | TCTTCATTAC | YFP | 4 |
| 2 | ATGAATCTAC | GFP | 5 |
| 2 | TCAATGTATG | RFP | 6 |
| 2 | AATTGAGTAC | CFP | 7 |
| 2 | ATGTTAATGG | YFP | 8 |
| 3 | AATTAGGATG | GFP | 9 |
| 3 | ATAATGGATC | RFP | 10 |
| 3 | TAATAAGGTG | CFP | 11 |
| 3 | TAGTTAGAGC | YFP | 12 |
| 4 | ATAGAGAAGG | GFP | 13 |
| 4 | TTGATGATAC | RFP | 14 |
| 4 | ATAGTGATTC | CFP | 15 |
| 4 | TATAACGATG | YFP | 16 |
| 5 | TTAAGTTTAG | GFP | 17 |
| 5 | ATACGTTATG | RFP | 18 |

(continued)

| Position in barcode domain | Nucleic Acid Sequence (5' to 3') in Attachment Region | Detectable label of complementary nucleic acid or reporter complex comprising detectable labels | SEQ ID NO |
|---|---|---|---|
| 5 | TGTACTATAG | CFP | 19 |
| 5 | TTAACAAGTG | YFP | 20 |
| 6 | AACTATGTAC | GFP | 21 |
| 6 | TAACTATGAC | RFP | 22 |
| 6 | ACTAATGTTC | CFP | 23 |
| 6 | TCATTGAATG | YFP | 24 |

As seen in Table 1, the nucleic acid sequence of a first attachment region may be one of SEQ ID NO: 1 to SEQ ID NO: 4, the nucleic acid sequence of a second attachment may be one of SEQ ID NO: 5 to SEQ ID NO: 8, and the nucleic acid sequence of a third attachment may be one of SEQ ID NO: 9 to SEQ ID NO: 12.

Table 1 shows that a given attachment region may be bound with one of four possible complementary nucleic acids comprising a detectable label or reporter complexes comprising detectable labels. Thus, a first position may be labeled with GFP, if the first position's attachment region comprises SEQ ID NO: 1; alternately, the first position may be labeled with RFP, if the first position's attachment region comprises SEQ ID NO: 2. Detectable labels other than GFP, RFP, CFP and YFP may be used. Additionally, the nucleotide sequence for an attachment region may be different than those listed in Table 1.

When the first position's attachment region comprises SEQ ID NO: 1, the second position's second attachment region comprises SEQ ID NO: 5, and the third position's second attachment region comprises SEQ ID NO: 9, the probe will have a first, second, and third position that is labeled with GFP. This three position GFP code (i.e., a linear order of detectable labels) identifies the target nucleic acid bound by the probe's target biding site (e.g., *GATA3*).

However, for example, when the first position's attachment region comprises SEQ ID NO: 1, the second position's second attachment region comprises SEQ ID NO: 6, and the third position's second attachment region comprises SEQ ID NO: 12, the probe will have a first, second, and third position that is labeled with GFP, RFP, and YFP, respectively. This three position GFP-RFP-YFP code (i.e., a linear order of detectable labels) identifies the target nucleic acid bound by the probe's target biding site (e.g., *MafB*). Together, the selection of attachment regions for each position defines a linear color code that a probe backbone can produce; this linear code is associated with a specific target nucleic acid that is complementary to a known nucleotide sequence of the target binding domain.

Similarly, for example, when the first position's attachment region comprises SEQ ID NO: 1, the second position's second attachment region comprises SEQ ID NO: 6, and the third position's second attachment region comprises SEQ ID NO: 11 the probe will have a first, second, and third position that is labeled with GFP, RFP, and CFP, respectively. This three position GFP-RFP-CFP code (i.e., a linear order of detectable labels) identifies the target nucleic acid bound by the probe's target biding site (e.g., *Fat3*).

Together, the three probes that respectively bind to *GATA3, MafB,* and *Fat3* can be simultaneously applied to a sample and the presence and quantity of each of *GATA3, MafB,* and *Fat3* can be detected due to a difference in their linear order of detectable labels.

In embodiments, a complementary nucleic acid molecule may be bound by a detectable label. In alternate embodiments, a complementary nucleic acid is associated with a reporter complex comprising detectable labels.

The nucleotide sequence of a complementary nucleic acid is not limited; preferably it lacks substantial homology (e.g., 50% to 99.9%) with a known nucleotide sequence; this helps avoid undesirable hybridization of a complementary nucleic acid and a target nucleic acid.

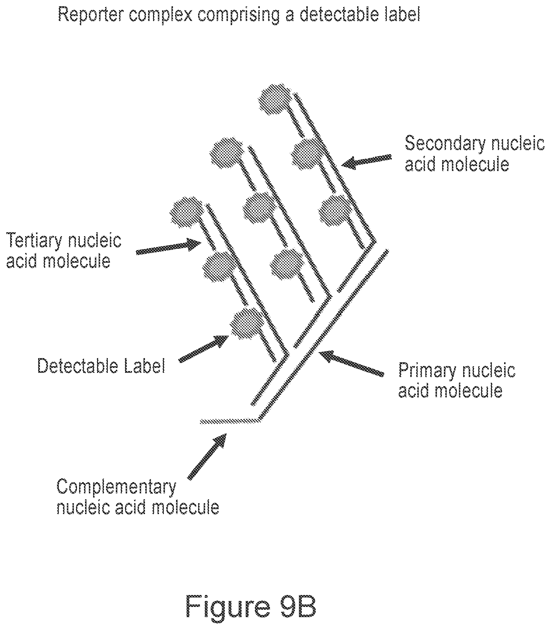

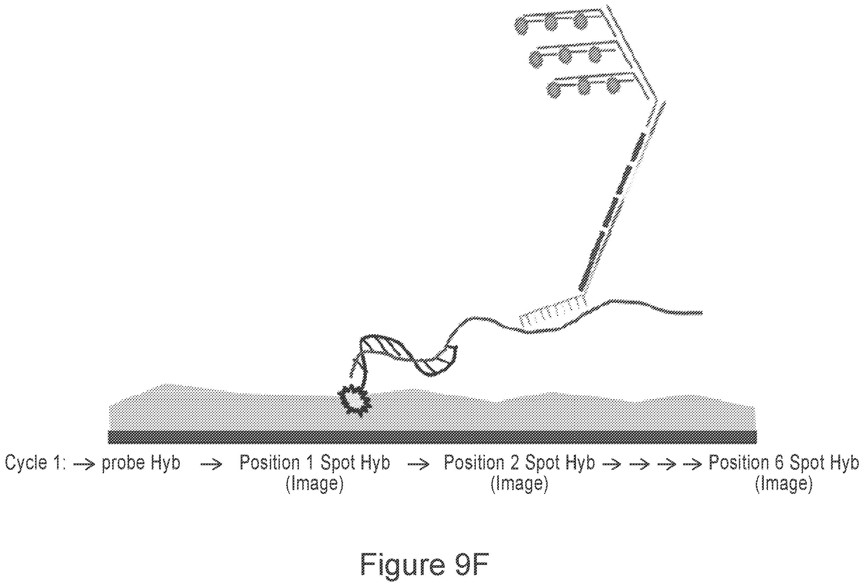

An example of the reporter complex useful in the present invention is shown in Figure 9B. In this example, a complementary nucleic acid is linked to (branches from) a primary nucleic acid molecule, which in turn is hybridized to a plurality of secondary nucleic acid molecules, each of which is in turn hybridized to a plurality of tertiary nucleic acid molecules having attached thereto one or more detectable labels.

In embodiments, a primary nucleic acid molecule may comprise about 90 nucleotides. A secondary nucleic acid molecule may comprise about 87 nucleotides. A tertiary nucleic acid molecule may comprise about 15 nucleotides.

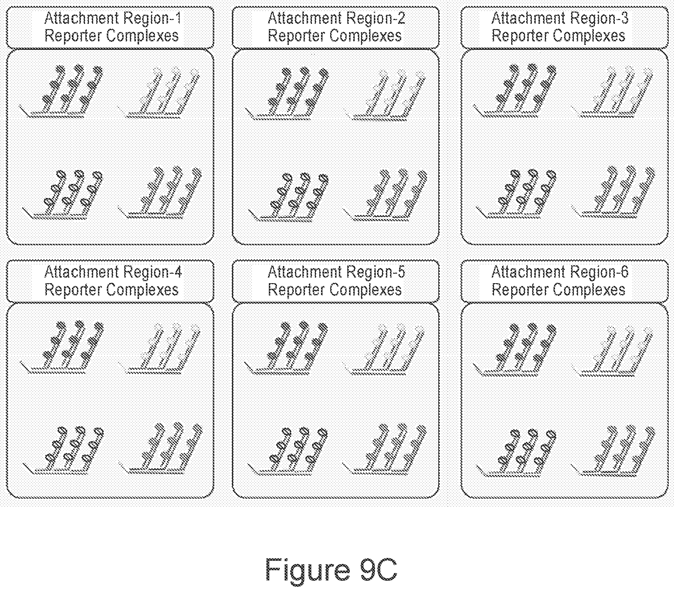

Figure 9C shows a population of exemplary reporter complexes. Included in the top left panel of Figure 9C are the four complexes that hybridize to attachment region 1 of a probe. There is one type of reporter complex for each possible nucleotide that can be present in nucleotide position 1 of a probe's target binding domain.

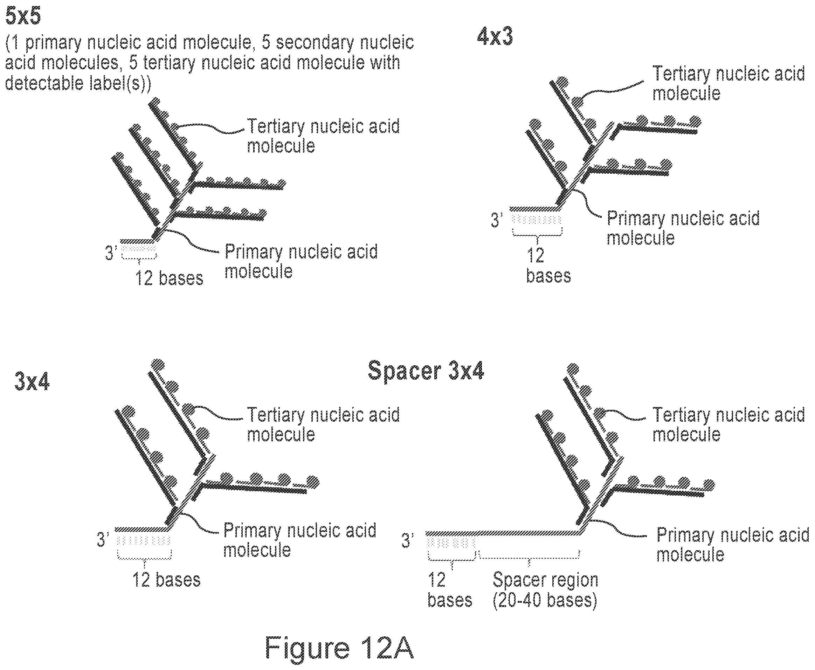

Reporter complexes can be of various designs. For example, a primary nucleic acid molecule can be hybridized to at least one (e.g., 1, 2, 3, 4, 5, 6, 7, 8, 9, 10, or more) secondary nucleic acid molecules. Each secondary nucleic acid molecule may be hybridized to at least one (e.g., 1, 2, 3, 4, 5, 6, 7, 8, 9, 10, or more) tertiary nucleic acid molecules. Exemplary reporter complexes are shown in Figure 12A. Here, the "4x3" reporter complex has one primary nucleic acid molecule (that is linked to/ branches from a complementary nucleic acid molecule) hybridized to four secondary nucleic acid molecules, each of which is hybridized to three tertiary nucleic acid molecules (each comprising a detectable label). In this figure, each complementary nucleic acid of a complex is 12 nucleotides long ("12 bases"); however, the length of the complementary nucleic is non-limited and can be less than 12 or more than 12 nucleotides. The bottom-right complex includes a spacer region between its complementary nucleic acid and its primary nucleic acid molecule. The spacer is identified as 20 to 40 nucleotides long; however, the length of a spacer is non-limiting and it can be shorter than 20 nucleotides or longer than 40 nucleotides.

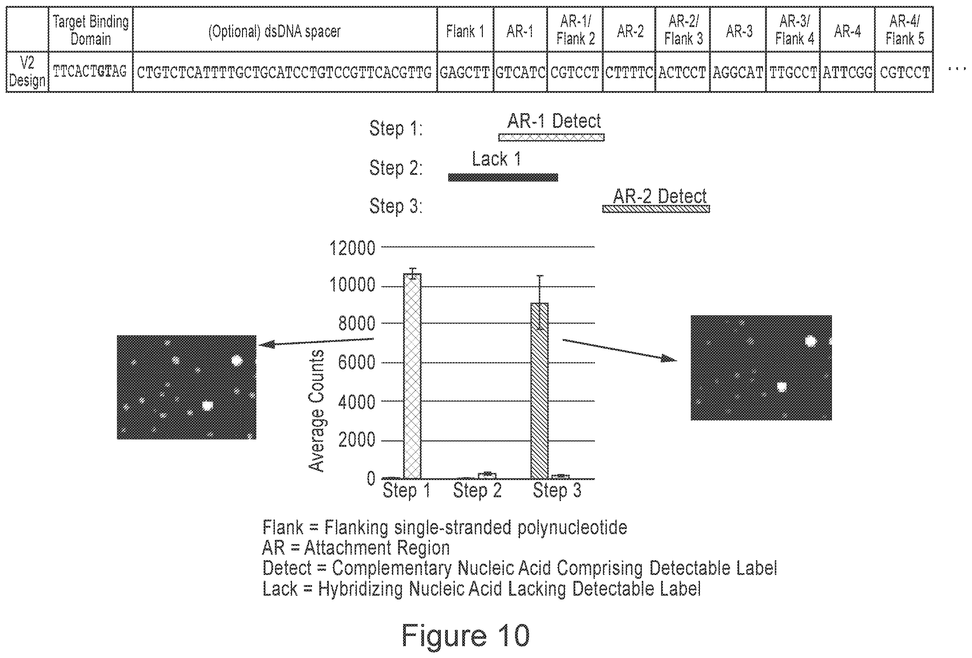

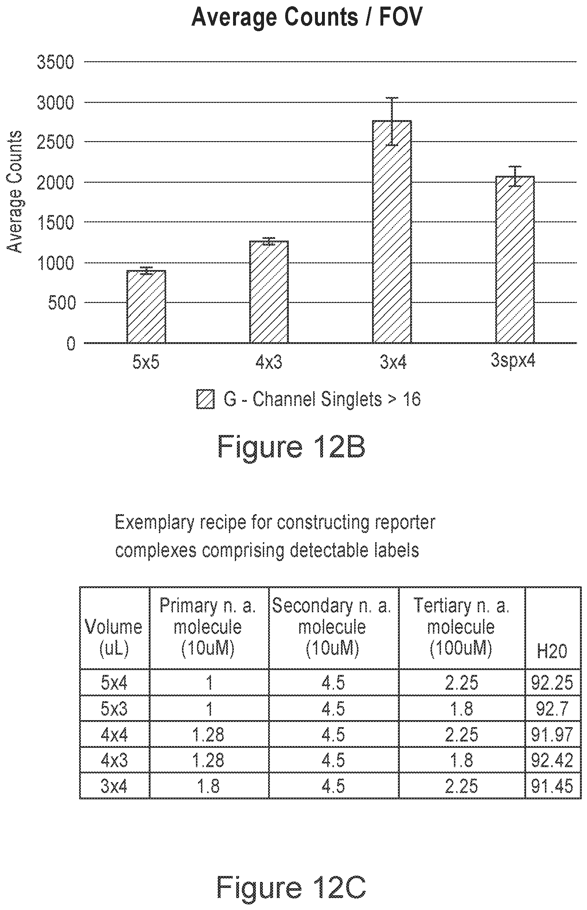

Figure 12B shows variable average (fluorescent) counts obtained from the four exemplary reporter complexes shown in Figure 12A. In Figure 12B, 10pM of biotinylated target template was attached onto a streptavidin-coated flow-cell surface, 10nM of a reporter complex was flowed onto the flow-cell; after a one minute incubation, the flow-cell was washed, the flow-cell was imaged, and fluorescent features were counted.

In embodiments, the reporter complexes are "pre-constructed". That is, each polynucleotide in the complex is hybridized prior to contacting the complex with a probe. An exemplary recipe for pre-constructing five exemplary reporter complexes is shown in Figure 12C.

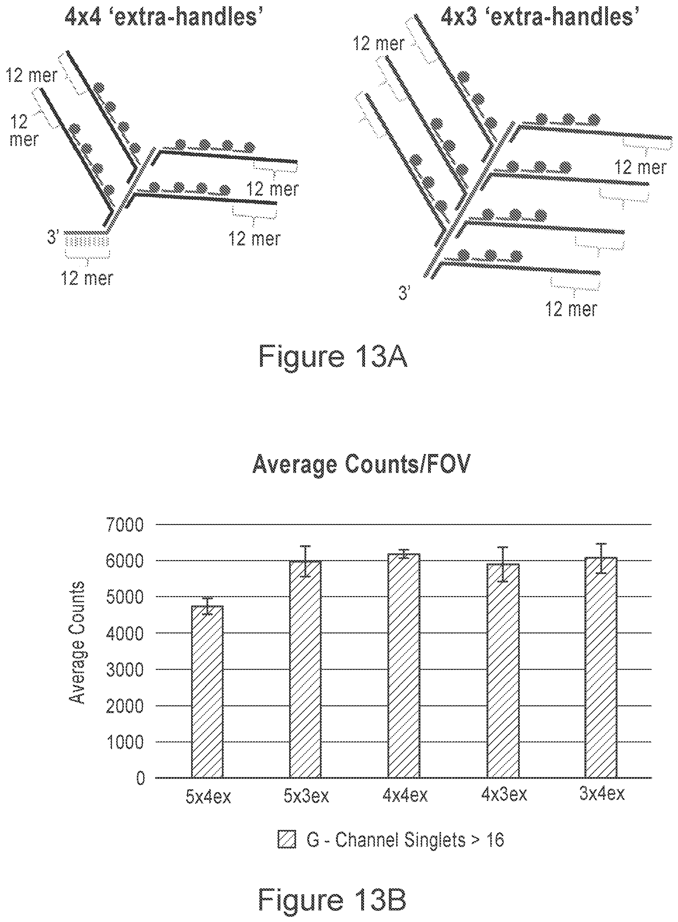

Figure 13A shows alternate reporter complexes in which the secondary nucleic acid molecules have "extra-handles" that are not hybridized to a tertiary nucleic acid molecule and are distal to the primary nucleic acid molecule. In this figure, each "extra-handle" is 12 nucleotides long ("12 mer"); however, their lengths are non-limited and can be less than 12 or more than 12 nucleotides. In embodiments, the "extra-handles" each comprise the nucleotide sequence of the complementary nucleic acid; thus, when a reporter complex comprises "extra-handles", the reporter complex can hybridize to a probe either via the reporter complex's complementary nucleic acid or via an "extra-handle." Accordingly, the likelihood that a reporter complex binds to a probe is increased. The "extra-handle" design may also improve hybridization kinetics. Without being bound to theory, the "extra-handles" essentially increase the effective concentration of the reporter complex's complementary nucleic acid.

Figure 13B shows variable average (fluorescent) counts obtained from the five exemplary reporter complexes having "extra-handles" using the procedure described for Figure 12B.

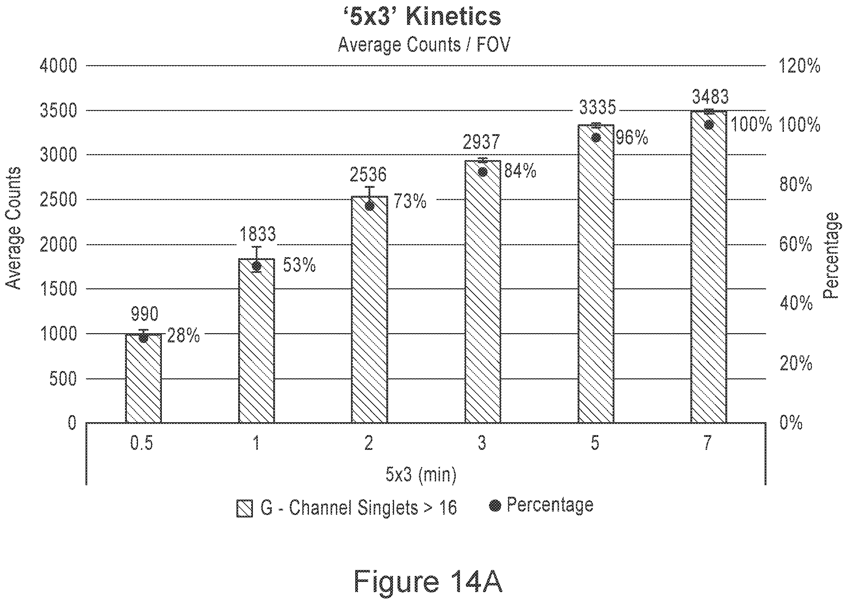

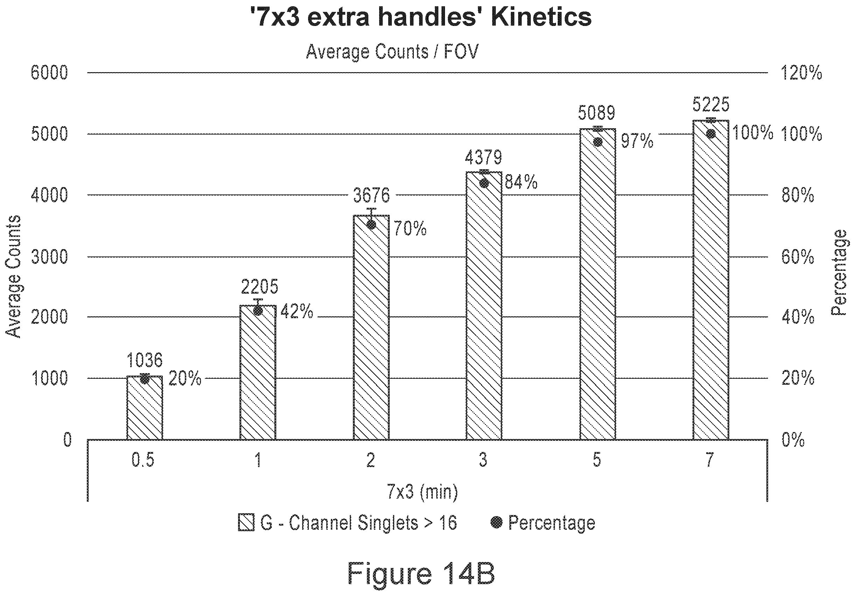

Figure 14A and 14B show hybridization kinetics and fluorescent intensities for two exemplary reporter complexes. By about five minutes, total counts start to plateau indicating that most reporter complex added have found an available target.

A detectable moiety, label or reporter can be bound to a complementary nucleic acid or to a tertiary nucleic acid molecule in a variety of ways, including the direct or indirect attachment of a detectable moiety such as a fluorescent moiety, colorimetric moiety and the like. A detectable label can include multiple detectable moieties that each have an individual emission spectra which can be the same or different. For example, a detectable label can include multiple fluorophores each having an emission spectra which can be the same or different. One of skill in the art can consult references directed to labeling nucleic acids. Examples of fluorescent moieties include, but are not limited to, yellow fluorescent protein (YFP), green fluorescent protein (GFP), cyan fluorescent protein (CFP), red fluorescent protein (RFP), umbelliferone, fluorescein, fluorescein isothiocyanate, rhodamine, dichlorotriazinylamine fluorescein, cyanines, dansyl chloride, phycocyanin, phycoerythrin and the like. Fluorescent labels and their attachment to nucleotides and/or oligonucleotides are described in many reviews, including Haugland, Handbook of Fluorescent Probes and Research Chemicals, Ninth Edition (Molecular Probes, Inc., Eugene, 2002); Keller and Manak, DNA Probes, 2nd Edition (Stockton Press, New York, 1993); Eckstein, editor, Oligonucleotides and Analogues: A Practical Approach (IRL Press, Oxford, 1991); and Wetmur, Critical Reviews in Biochemistry and Molecular Biology, 26:227-259 (1991). Particular methodologies applicable to the invention are disclosed in the following sample of references: U.S. Patent Nos. 4,757,141; 5,151,507; and 5,091,519. In one aspect, one or more fluorescent dyes are used as labels for labeled target sequences, e.g., as disclosed by U.S. Patent Nos. 5,188,934 (4,7-dichlorofluorescein dyes); 5,366,860 (spectrally resolvable rhodamine dyes); 5,847,162 (4,7-dichlororhodamine dyes); 4,318,846 (ether-substituted fluorescein dyes); 5,800,996 (energy transfer dyes); Lee *et al.* 5,066,580 (xanthine dyes); 5,688,648 (energy transfer dyes); and the like. Labelling can also be carried out with quantum dots, as disclosed in the following patents and patent publications: U.S. Patent Nos. 6,322,901; 6,576,291; 6,423,551; 6,251,303; 6,319,426; 6,426,513; 6,444,143; 5,990,479; 6,207,392; 2002/0045045; and 2003/0017264. As used herein, the term "fluorescent label" comprises a signaling moiety that conveys information through the fluorescent absorption and/or emission properties of one or more molecules. Such fluorescent properties include fluorescence intensity, fluorescence lifetime, emission spectrum characteristics, energy transfer, and the like. A fluorescent label, as used herein, can include multiple detectable moieties that each have an individual fluorescent absorption and/or emission property which can be the same or different. For example, a fluorescent label can include multiple fluorophores each having an emission spectra which can be the same or different. In a further non-limiting example, a fluorescent label can include any combination of the fluorophores ALEXA FLUOR™ 350, ALEXA FLUOR™

405, ALEXA FLUOR™ 430, ALEXA FLUOR™ 532, ALEXA FLUOR™ 546, ALEXA FLUOR™ 568, ALEXA FLUOR™ 594 and ALEXA FLUOR™ 647.

Commercially available fluorescent nucleotide analogues readily incorporated into nucleotide and/or oligonucleotide sequences include, but are not limited to, Cy3-dCTP, Cy3-dUTP, Cy5-dCTP, Cy5-dUTP (Amersham Biosciences, Piscataway, NJ), fluorescein- 12-dUTP, tetramethylrhodamine-6-dUTP, TEXAS RED™-5-dUTP, CASCADE BLUE™-7-dUTP, BODIPY TMFL-14-dUTP, BODIPY TMR-14-dUTP, BODIPY TMTR-14-dUTP, RHODAMINE GREEN™-5-dUTP, OREGON GREENR™ 488-5-dUTP, TEXAS RED™- 12-dUTP, BODIPY TM 630/650- 14-dUTP, BODIPY TM 650/665- 14-dUTP, ALEXA FLUOR™ 488-5-dUTP, ALEXA FLUOR™ 532-5-dUTP, ALEXA FLUOR™ 568-5-dUTP, ALEXA FLUOR™ 594-5-dUTP, ALEXA FLUOR™ 546- 14-dUTP, fluorescein- 12-UTP, tetramethylrhodamine-6-UTP, TEXAS RED™-5-UTP, mCherry, CASCADE BLUE™-7-UTP, BODIPY TM FL-14-UTP, BODIPY TMR-14-UTP, BODIPY TM TR-14-UTP, RHODAMINE GREEN™-5-UTP, ALEXA FLUOR™ 488-5-UTP, LEXA FLUOR™ 546- 14-UTP (Molecular Probes, Inc. Eugene, OR) and the like. Alternatively, the above fluorophores and those mentioned herein may be added during oligonucleotide synthesis using for example phosphoroamidite or NHS chemistry. Protocols are known in the art for custom synthesis of nucleotides having other fluorophores (*See,* Henegariu *et al.* (2000) Nature Biotechnol. 18:345). 2-Aminopurine is a fluorescent base that can be incorporated directly in the oligonucleotide sequence during its synthesis. Nucleic acid could also be stained, a priori, with an intercalating dye such as DAPI, YOYO- 1 , ethidium bromide, cyanine dyes (e.g., SYBR Green) and the like.

Other fluorophores available for post-synthetic attachment include, but are not limited to, ALEXA FLUOR™ 350, ALEXA FLUOR™ 405, ALEXA FLUOR™ 430, ALEXA FLUOR™ 532, ALEXA FLUOR™ 546, ALEXA FLUOR™ 568, ALEXA FLUOR™ 594, ALEXA FLUOR™ 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, Pacific Orange, rhodamine 6G, rhodamine green, rhodamine red, tetramethyl rhodamine, Texas Red (available from Molecular Probes, Inc., Eugene, OR), Cy2, Cy3, Cy3.5, Cy5, Cy5.5, Cy7 (Amersham Biosciences, Piscataway, NJ) and the like. FRET tandem fluorophores may also be used, including, but not limited to, PerCP-Cy5.5, PE-Cy5, PE-Cy5.5, PE-Cy7, PE-Texas Red, APC-Cy7, PE-Alexa dyes (610, 647, 680), APC-Alexa dyes and the like.

Metallic silver or gold particles may be used to enhance signal from fluorescently labeled nucleotide and/or oligonucleotide sequences (Lakowicz *et al.* (2003) BioTechniques 34:62).

Other suitable labels for an oligonucleotide sequence may include fluorescein (FAM, FITC), digoxigenin, dinitrophenol (DNP), dansyl, biotin, bromodeoxyuridine (BrdU), hexahistidine (6xHis), phosphor-amino acids (*e.g.*, P-tyr, P-ser, P-thr) and the like. In one embodiment the following hapten/antibody pairs are used for detection, in which each of the antibodies is derivatized with a detectable label: biotin/a-biotin, digoxigenin/a-digoxigenin, dinitrophenol (DNP)/a-DNP, 5-Carboxyfluorescein (FAM)/a-FAM.

Detectable labels described herein are spectrally resolvable. "Spectrally resolvable" in reference to a plurality of fluorescent labels means that the fluorescent emission bands of the labels are sufficiently distinct, *i.e.*, sufficiently non-overlapping, that molecular tags to which the respective labels are attached can be distinguished on the basis of the fluorescent signal generated by the respective labels by standard photodetection systems, e.g., employing a system of band pass filters and photomultiplier tubes, or the like, as exemplified by the systems described in U.S. Patent Nos. 4,230,558; 4,811,218; or the like, or in Wheeless et al., pgs. 21-76, in Flow Cytometry: Instrumentation and Data Analysis (Academic Press, New York, 1985). In one aspect, spectrally resolvable organic dyes, such as fluorescein, rhodamine, and the like, means that wavelength emission maxima are spaced at least 20 nm apart, and in another aspect, at least 40 nm apart. In another aspect, chelated lanthanide compounds, quantum dots, and the like, spectrally resolvable means that wavelength emission maxima are spaced at least 10 nm apart, and in a further aspect, at least 15 nm apart.

## Method for detecting a nucleic acid

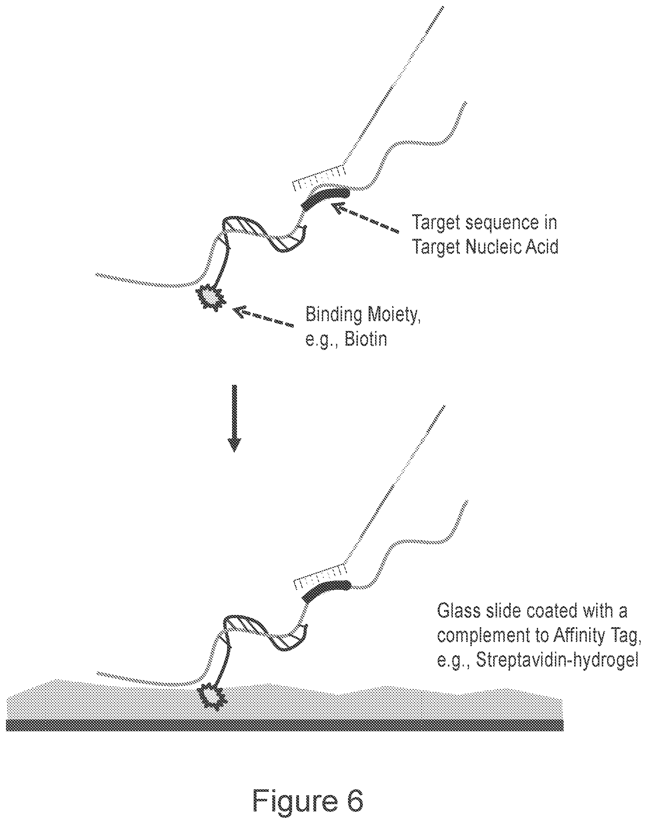

The present disclosure relates to methods for detecting a nucleic acid using a probe of the present invention. Examples of the method are shown in Figures 6 to 11.

The method comprises reversibly hybridizing at least one probe, of the present invention, to a target nucleic acid that is immobilized (e.g., at one, two, three, four, five, six, seven, eight, nine, ten, or more positions) to a substrate.

The substrate can be any solid support known in the art, *e.g.,* a coated slide and a microfluidic device, which is capable of immobilizing a target nucleic acid. In certain embodiments, the substrate is a surface, membrane, bead, porous material, electrode or array. The target nucleic acid can be immobilized onto any substrate apparent to those of skill in the art.

In embodiments, the target nucleic acid is bound by a capture probe which comprises a domain that is complementary to a portion of the target nucleic acid. The portion may be an end of the target nucleic acid or not towards an end.

Exemplary useful substrates include those that comprise a binding moiety selected from the group consisting of ligands, antigens, carbohydrates, nucleic acids, receptors, lectins, and antibodies. The capture probe comprises a binding moiety capable of binding with the binding moiety of the substrate. Exemplary useful substrates comprising reactive moieties include, but are not limited to, surfaces comprising epoxy, aldehyde, gold, hydrazide, sulfhydryl, NHS-ester, amine, thiol, carboxylate, maleimide, hydroxymethyl phosphine, imidoester, isocyanate, hydroxyl, pentafluorophenyl-ester, psoralen, pyridyl disulfide or vinyl sulfone, polyethylene glycol (PEG), hydrogel, or mixtures thereof. Such surfaces can be obtained from commercial sources or prepared according to standard techniques. Exemplary useful substrates comprising reactive moieties include, but are not limited to, OptArray-DNA NHS group (Accler8), Nexterion Slide AL (Schott) and Nexterion Slide E (Schott).

In embodiments, the capture probe's binding moiety is biotin and the substrate comprises avidin (e.g., streptavidin). Useful substrates comprising avidin are commercially available including TB0200 (Accelr8), SAD6, SAD20, SAD100, SAD500, SAD2000 (Xantec), SuperAvidin (Array-It), streptavidin slide (catalog #MPC 000, Xenopore) and STREPTA VIDINnslide (catalog #439003, Greiner Bio-one).

In embodiments, the capture probe's binding moiety is avidin (e.g., streptavidin) and the substrate comprises biotin. Useful substrates comprising biotin that are commercially available include, but are not limited to, Optiarray-biotin (Accler8), BD6, BD20, BD100, BD500 and BD2000 (Xantec).

In embodiments, the capture probe's binding moiety can comprise a reactive moiety that is capable of being bound to the substrate by photoactivation. The substrate could comprise the photoreactive moiety, or the first portion of the nanoreporter could comprise the photoreactive moiety. Some examples of photoreactive moieties include aryl azides, such as N((2-pyridyldithio)ethyl)-4-azidosalicylamide; fluorinated aryl azides, such as 4-azido-2,3,5,6-tetrafluorobenzoic acid; benzophenone-based reagents, such as the succinimidyl ester of 4-benzoylbenzoic acid; and 5-Bromo-deoxyuridine.

In embodiments, the capture probe's binding moiety can be immobilized to the substrate via other binding pairs apparent to those of skill in the art.

After binding to the substrate, the target nucleic acid may be elongated by applying a force (*e.g.,* gravity, hydrodynamic force, electromagnetic force "electrostretching", flow-stretching, a receding meniscus technique, and combinations thereof) sufficient to extend the target nucleic acid.

The target nucleic acid may be bound by a second capture probe which comprises a domain that is complementary to a second portion of the target nucleic acid. The portion may be an end of the target nucleic acid or not towards an end. Binding of a second capture probe can occur after or during elongation of the target nucleic acid or to a target nucleic acid that has not been elongated. The second capture probe can have a binding as described above.

A capture probe may comprise or be associated with a detectable label, *i.e.,* a fiducial spot.

The capture probe is capable of isolating a target nucleic acid from a sample. Here, a capture probe is added to a sample comprising the target nucleic acid. The capture probe binds the target nucleic acid via the region of the capture probe that his complementary to a region of the target nucleic acid. When the target nucleic acid contacts a substrate comprising a moiety that binds the capture probe's binding moiety, the nucleic acid becomes immobilized onto the substrate.

To ensure that a user "captures" as many target nucleic acid molecules as possible from high fragmented samples, it is helpful to include a plurality of capture probes, each complementary to a different region of the target nucleic acid. For example, there may be three pools of capture probes, with a first pool complementary to regions of the target nucleic acid near its 5' end, a second pool complementary to regions in the middle of the target nucleic acid, and a third pool near its 3' end. This can be generalized to "n-regions-of-interest" per target nucleic acid. In this example, each individual pool of fragmented target nucleic acid bound to a capture probe comprising or bound to a biotin tag. 1/nth of input sample (where n = the number of distinct regions in target nucleic acid) is isolated for each pool chamber. The capture probe binds the target nucleic acid of interest. Then the target nucleic acid is immobilized, via the capture probe's biotin, to an avidin molecule adhered to the substrate. Optionally, the target nucleic acid is stretched, *e.g.,* via flow or electrostatic force. All n-pools can be stretched-and-bound simultaneously, or, in order to maximize the number of fully stretched molecules, pool 1 (which captures most 5' region) can be stretched and bound first; then pool 2, (which captures the middle-of-target region) is then can be stretched and bound; finally, pool 3 is can be stretched and bound.

The number of distinct capture probes required is inversely related to the size of target nucleic acid fragment. In other word, more capture probes will be required for a highly-fragmented target nucleic acid. For sample types with highly fragmented and degraded target nucleic acids *(e.g.,* Formalin-Fixed Paraffin Embedded Tissue) it may be useful to include multiple pools of capture probes. On the other hand, for samples with long target nucleic acid fragments, *e.g.,* in vitro obtained isolated nucleic acids, a single capture probe at a 5' end may be sufficient.

A probe or a capture probe of the present invention may comprise one or more affinity reagents, each selected from the group consisting of ligands, antigens, carbohydrates, nucleic acids, receptors, lectins, haptens, and antibodies. The affinity reagent allows purification of a complex formed by the probe or the capture probe and a target nucleic acid. Such purification enriches the concentration of target nucleic acids to be detected.

In embodiments, the affinity reagent is biotin and a purification means (e.g., attached to a solid support) comprises avidin (e.g., streptavidin).

In embodiments, the affinity reagent is avidin (e.g., streptavidin) and the purification means (e.g., attached to a solid support) comprises biotin.

In embodiments, the affinity reagent comprises a nucleic acid having a known sequence. Thus, the probe or the capture probe comprising the affinity reagent can be purified from a sample using a purification probe comprising a nucleic acid complementary to the affinity reagent. Likewise, a complex comprising the probe or the capture probe comprising the affinity reagent can be purified from a sample using a purification probe comprising a nucleic acid complementary to the affinity reagent. The affinity moieties for a probe and for a capture probe for the same target nucleic acid may have different nucleic acid sequences. Alternately, the affinity reagent for a probe and the affinity reagent for a capture probe each for the same target nucleic acid may have the same nucleic acid sequence. Each affinity reagent for each probe in a population of probes may have the same nucleic acid sequence. Each affinity reagent for each capture probe in a population of capture probes may have the same nucleic acid sequence. Each affinity reagent for each probe in a population of probes may have a different nucleic acid sequence. Each affinity reagent for each capture probe in a population of capture probes may have a different nucleic acid sequence.

In embodiments, the affinity reagent is a hapten and a purification means (e.g., attached to a solid support) comprises a protein binding domain (e.g., an antibody).

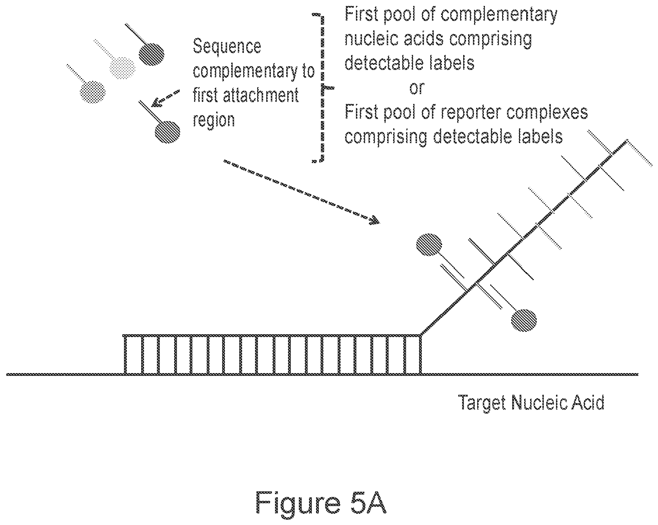

Figure 5A shows a schematic of a probe bound to a target nucleic acid. Here, the target nucleic acid comprises the sequence of TCAGTG. The probe's barcode domain was designed with attachment regions that specifically identify a bound TCAGTG with a particular linear color code or "linear order of detectable labels". A first pool of complementary nucleic acids comprising a detectable label or reporter complexes is shown at the top, each member of the pool has a different nucleotide sequence and an associated detectable label (e.g., a green-colored label and cyan-colored label). As an example, the nucleic acids in the first pool have sequences complementary to SEQ ID NOs: 1 to 4 of Table 1. In Figure 5A, the first attachment regions of the probe include one or more nucleotide sequence(s) that specifies that the first position should be labeled with a cyan-colored label (e.g., the attachment region comprises SEQ ID NO: 3 of Table 1). Thus, only the complementary nucleic acid specific to the first attachment position and carrying a cyan-label can bind the first position of the barcode domain of the shown probe. The cyan label is the first color in a linear color code that identifies a bound target nucleic acid.

The color associated with the first position is imaged and recorded in a system of the present invention.

The number of pools of complementary nucleic acids or reporter complexes is identical to the number of positions in the barcode domain. Thus, for a barcode domain having six positions, six pools will be cycled over the probes.

A probe may be provided to a target nucleic acid initially when capture probe is added to a sample comprising the target nucleic acid (See, Figure 6). Such probes can be provided at different concentrations, different buffer conditions, such as salt, and different temperatures to increase sensitivity and specificity for target nucleic acid.

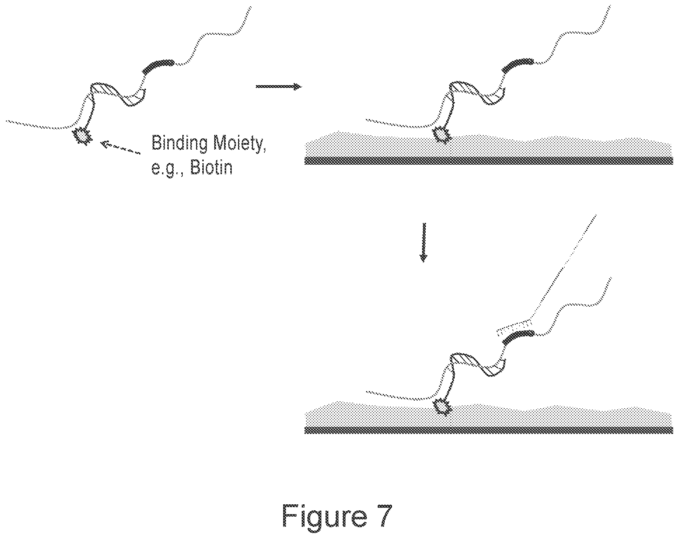

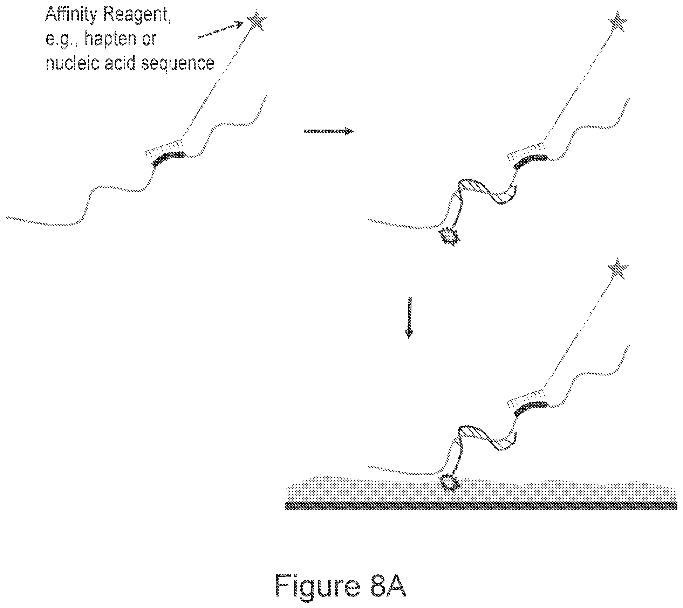

Capture probes and probe can have an affinity reagent for multi-stage purification purifications (See, Figures 7 and 8A to 8E). Where you can use either of the purifications alone or purify from both ends. Purification will increase specificity and purity of target capturing

A probe may be provided to a target nucleic acid and initially bound to the target nucleic acid completely lacking complementary nucleic acids comprising detectable labels or reporter complex comprising detectable labels. Such a probe will be smaller than a probe comprising detectable complementary nucleic acids. Such probes can be provided at higher concentrations than a probe comprising detectable labels. Such small probes will more rapidly and more efficiently bind to a target nucleic acid. Thus, providing data in a fraction of the time than required using probes comprising detectable labels.

Alternately, prior to contacting a target nucleic acid with a probe, the probe may be hybridized at its first position to a complementary nucleic acid comprising a detectable label or a reporter complex. Thus, when contacted with its target nucleic acid, the probe is capable of emitting a detectable signal from its first position and it is unnecessary to provide a first pool of complementary nucleic acids or reporter complexes that are directed to the first position on the barcode domain.

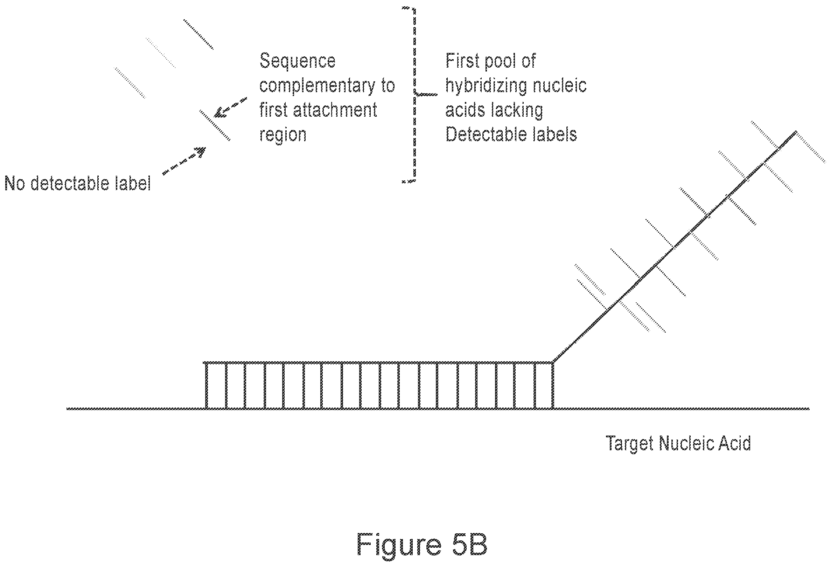

Figure 5B continues the method shown in Figure 5A. Here, the first complementary nucleic acids (or reporter complexes) that were bound to attachment regions in the first position of the barcode domain have been replaced with a first hybridizing nucleic acid lacking a detectable label. The first hybridizing nucleic acid and lacking a detectable label displaces the previously-bound complementary nucleic acids comprising a detectable label or the previously-bound reporter complexes. Thereby, the first position of barcode domain no longer emits a detectable signal.

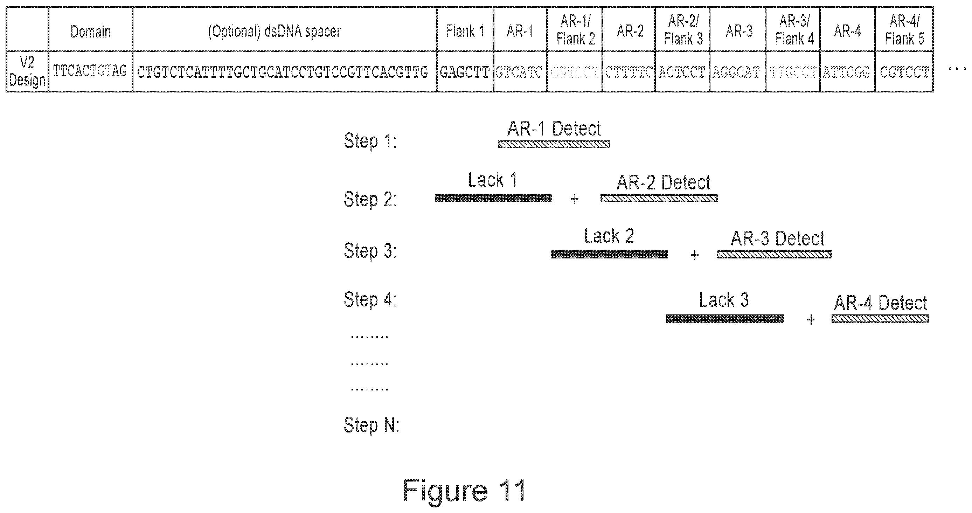

A hybridizing nucleic acid and lacking a detectable label may comprise an identical sequence as the previously-bound complementary nucleic acids comprising a detectable label or the previously-bound reporter complexes (e.g., SEQ ID NO: 1 to SEQ ID NO: 24). Preferably, the hybridizing nucleic acid and lacking a detectable label will be longer than the previously-bound complementary nucleic acids comprising a detectable label or the previously-bound reporter complexes. For this, the hybridizing nucleic acid further includes sequence that is complementary to a single-stranded polynucleotide or polynucleotide analogue region adjacent to the attachment region. Without being bound by theory, a hybridizing nucleic acid that is longer than its related complementary nucleic acid comprising a detectable label, will have a greater affinity for the barcode domain and readily displaces the complementary nucleic acid comprising a detectable label. Such hybridizing nucleic acids that are longer than their related complementary nucleic acids are shown in Figures 10 and 11.

In embodiments, the complementary nucleic acids comprising a detectable label or reporter complexes may be removed from the attachment region but not replaced with a hybridizing nucleic acid lacking a detectable label. This can occur, for example, by adding a chaotropic agent, increasing the temperature, changing salt concentration, adjusting pH, and/or applying a hydrodynamic force. In these embodiments fewer reagents (i.e., hybridizing nucleic acids lacking detectable labels) are needed.

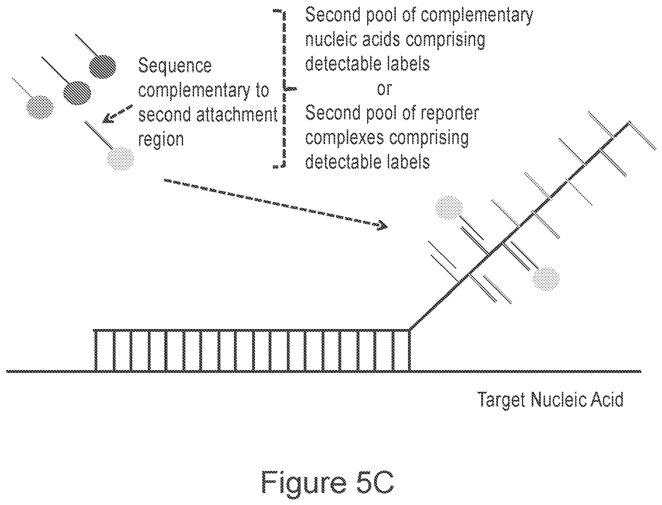

Figure 5C continues the method of the claimed invention. A second pool of complementary nucleic acids or reporter complexes is shown at the top (e.g., having sequences complementary to SEQ ID NOs: 5 to 8 of Table 1), each member of the pool has a different detectable label and a different nucleotide sequence. Moreover, the nucleotide sequences for the complementary nucleic acids or complementary nucleic acids of the reporter complexes of the first pool are different from the nucleotide sequences for those of the second pool. Here, only complementary nucleic acids from the second pool and comprising a yellow-colored detectable label binds the second position of the barcode domain (e.g., the complementary nucleic acid has a sequence complementary to SEQ ID NO: 8 of Table 1).

The color associated with the second position is imaged and recorded in a system of the present invention.

In embodiments, the steps shown in Figure 5C are subsequent to steps shown in Figure 5B. Here, once the first pool of complementary nucleic acids or reporter complexes (of Figure 5A) has been replaced with first hybridizing nucleic acids lacking a detectable label (in Figure 5B), then a second pool of complementary nucleic acids or reporter complexes is provided (as shown in Figure 5C). Alternately, the steps shown in Figure 5C are concurrent with steps shown in Figure 5B. Here, the first hybridizing nucleic acids lacking a detectable label (in Figure 5B) are provided simultaneously with a second pool of complementary nucleic acids or reporter complexes (as shown in Figure 5C).

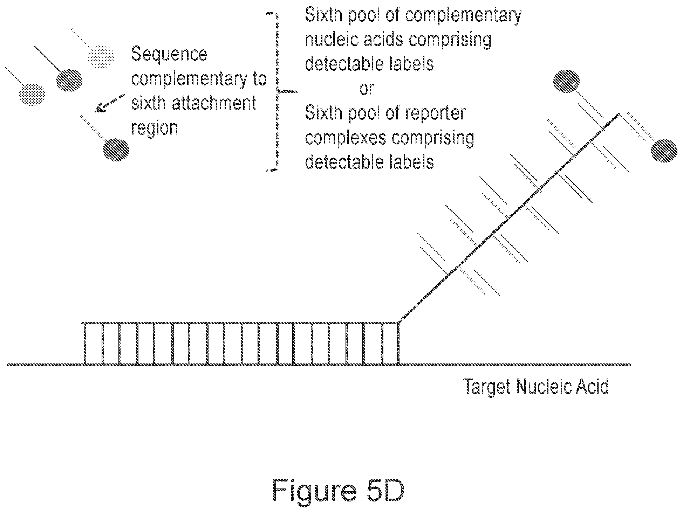

Figure 5D continues the method shown in Figure 5C. Here, the first through fifth positions on the barcode domain were bound by complementary nucleic acids comprising detectable labels or reporter complexes, the color associated with their positions were imaged and recorded, and the complementary nucleic acids have been replaced with hybridizing nucleic acids lacking detectable labels. The sixth position of the barcode domain is currently bound by a complementary nucleic acid comprising a detectable label or reporter complex, which identifies the sixth position in the target binding domain as being bound to a guanine (G).

The color associated with the sixth position is imaged and recorded in a system of the present invention.

At this point, the entire linear color code (i.e., a linear order of detectable labels) of a probe backbone has been detected; this linear code is then associated with the specific target nucleic acid that is complementary to a known nucleotide sequence of the target binding domain. As an example, a probe that can emit a linear color code of Green, Cyan, Red, Yellow, Yellow, Red is capable of being bound to *Fat2*. Thus, if the system of the present invention records a linear color code of Green, Cyan, Red, Yellow, Yellow, Red, then a user will know that *Fat2* was present in the sample.

Since each color associated with a probe's backbone domain is detected sequentially, it may be unnecessary for the probe backbone to be elongated to distinguish and resolve each color-label. This is an advantage over previous-generations of nucleic acid-detecting probes.

As mentioned above, complementary nucleic acids comprising detectable labels or reporter complexes can be removed from attachment regions but not replaced with hybridizing nucleic acid lacking detectable labels.

If needed, the rate of detectable label exchange can be accelerated by incorporating small single-stranded oligonucleotides that accelerate the rate of exchange of detectable labels (e.g., "Toe-Hold" Probes; see, e.g., Seeling et al., "Catalyzed Relaxation of a Metastable DNA Fuel"; J. Am. Chem. Soc. 2006, 128(37), pp12211-12220).

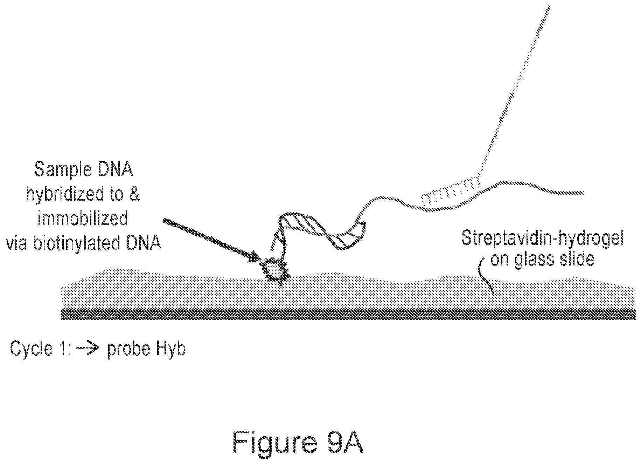

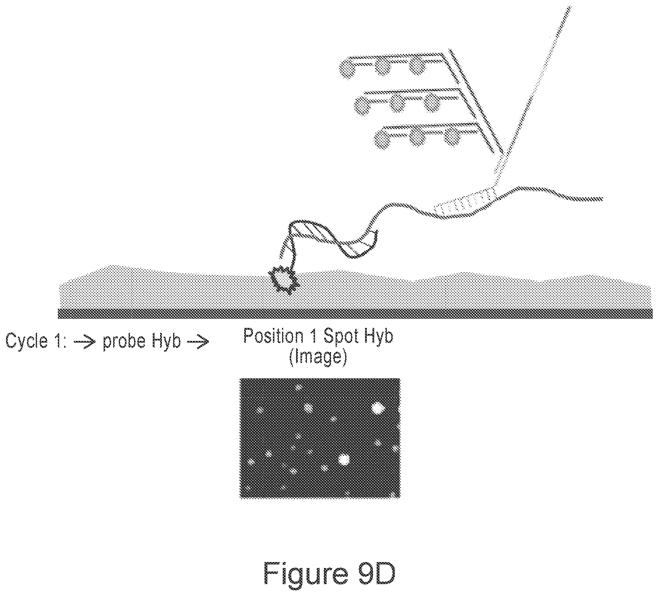

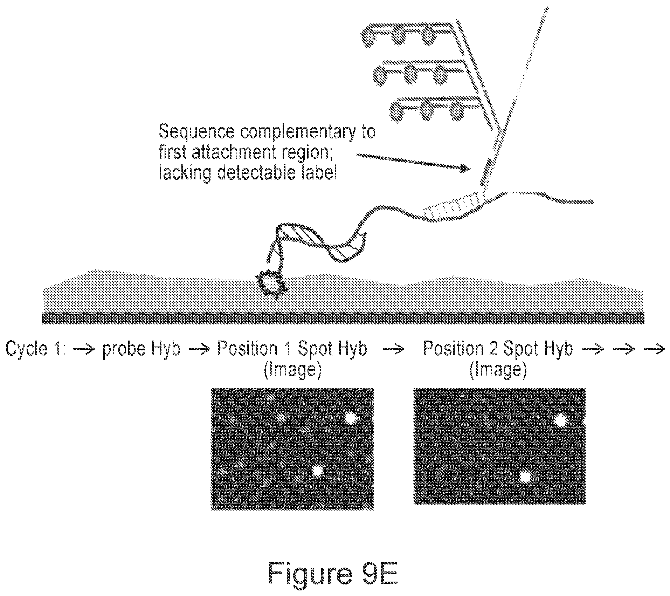

Like Figures 5A to 5D, Figures 9A and 9D to 9F show method steps of the present invention; however, Figures 9A and 9D to 9F clearly show that reporter complexes (comprising detectable labels) are bound to attachment regions of probes. Figures 9D and 9E show fluorescent signals sequentially emitted from probes hybridized to reporter complexes.

Figure 10 summarizes the steps shown in Figures 9D and 9E. At the top of the figure is shown the nucleotide sequence of an exemplary probe and identifies significant domains of the probe. The probe includes an optional double-stranded DNA spacer between its target binding domain and its barcode domain. The barcode domain comprises, in order, a "Flank 1" portion, an "AR-1" portion, an "AR-1/Flank 2" portion, an "AR-2" portion, and an "AR-2/Flank 3" portion. In Step 1, the "AR-1 Detect" is hybridized to the probe's "AR-1" and "AR-1/Flank 2" portions. "AR-1 Detect" corresponds to a reporter complex or complementary nucleic acid comprising a detectable label that encodes a first position thymidine. Thus, Step 1 corresponds to Figure 9D. In Step 2, the "Lack 1" is hybridized to the probe's "Flank 1" and "AR-1" portions. "Lack 1" corresponds to the hybridizing nucleic acid lacking a detectable label that is specific to the probe's first attachment region (as shown in Figure 9E as a black bar covering the first attachment region). By hybridizing to the "Flank 1" position, which is 5' to the reporter complex or complementary nucleic acid, the hybridizing nucleic acid more efficiently displaces the reporter complex/complementary nucleic acid from the probe. The "Flank" portions are also known as "Toe-Holds". In Step 3, the "AR-2 Detect" is hybridized to the probe's "AR-2" and "AR-2/Flank 3" portions. "AR-2 Detect" corresponds to a reporter complex or complementary nucleic acid comprising a detectable label that encodes a second position Guanine. Thus, Step 3 corresponds to Figure 9E. In this embodiment, hybridizing nucleic acid lacking a detectable label and complementary nucleic acids comprising detectable labels/reporter complexes are provided sequentially.

Alternately, hybridizing nucleic acid lacking a detectable label and complementary nucleic acids comprising detectable labels/reporter complexes are provided concurrently. This alternate embodiment is shown in Figure 11. In Step 2, the "Lack 1" (hybridizing nucleic acid lacking a detectable label) is provided along with the "AR-2 Detect" (reporter complex that encodes a second position Guanine). This alternate embodiment may be more time effective that the embodiment illustrated in Figure 10 because it combines two steps into one.

The detectable labels of the instant disclosure can be detected by any means known in the art. For example, the detectable label may be detected by a system comprising one or more of a microscope, camera, microprocessor and/or computer system. In one aspect the camera is a CCD camera. In one aspect, the microscope, camera and computer system can comprise a complementary metal-oxide semiconductor (CMOS-chip).

## Multiplexed detection of a plurality of nucleic acids

In embodiments, a plurality of nucleic acids are detected simultaneously, i.e., multiplexed detection. For this, a set or population of distinct probes is provided to a sample of immobilized nucleic acid targets. A set or population probes preferably includes at least two, e.g., 3, 4, 5, 6, 7, 8, 9, 10, 11, 12, 13, 14, 15, 16, 17, 18, 19 20, 30, 40, 50, 60, 70, 80, 90, 100, 200, 300, 400, 500, 600, 700, 800, 900, 1000 or more species of probes.

A set of probes may be pre-defined based upon the cell type or tissue type to be targeted. For example, if the tissue is a breast cancer, then the set of probes will include probes directed to expressed nucleic acids relevant to breast cancer cells (e.g., *Her2, EGFR,* and PR) and/or probes directed to nucleic acids expressed in normal breast tissues. Additionally, the set of probes may be pre-defined based upon developmental status of a cell or tissue to be targeted.

NanoString Technologies® nCounter® systems and methods allow simultaneous multiplexed identification a plurality (800 or more) distinct target proteins and/or target nucleic acids.

## Definitions:

In certain exemplary embodiments, the terms "annealing" and "hybridization," as used herein, are used interchangeably to mean the formation of a stable duplex. In one aspect, stable duplex means that a duplex structure is not destroyed by a stringent wash under conditions such as a temperature of either about 5 °C below or about 5 °C above the Tm of a strand of the duplex and low monovalent salt concentration, e.g., less than 0.2 M, or less than 0.1 M or salt concentrations known to those of skill in the art. The term "perfectly matched," when used in reference to a duplex means that the polynucleotide and/or oligonucleotide strands making up the duplex form a double stranded structure with one another such that every nucleotide in each strand undergoes Watson-Crick base pairing with a nucleotide in the other strand. The term "duplex" comprises, but is not limited to, the pairing of nucleoside analogs, such as deoxyinosine, nucleosides with 2-aminopurine bases, PNAs, and the like, that may be employed. A "mismatch" in a duplex between two oligonucleotides means that a pair of nucleotides in the duplex fails to undergo Watson-Crick bonding.

As used herein, the term "hybridization conditions," will typically include salt concentrations of less than about 1 M, more usually less than about 500 mM and even more usually less than about 200 mM. Hybridization temperatures can be as low as 5 °C, but are typically greater than 22 °C, more typically greater than about 30 °C, and often in excess of about 37 °C. Hybridizations are usually performed under stringent conditions, e.g., conditions under which a probe will specifically hybridize to its target subsequence. Stringent conditions are sequence-dependent and are different in different circumstances. Longer fragments may require higher hybridization temperatures for specific hybridization. As other factors may affect the stringency of hybridization, including base composition and length of the complementary strands, presence of organic solvents and extent of base mismatching, the combination of parameters is more important than the absolute measure of any one alone.

Generally, stringent conditions are selected to be about 5 °C lower than the Tm for the specific sequence at a defined ionic strength and pH. Exemplary stringent conditions include salt concentration of at least 0.01 M to no more than 1 M Na ion concentration (or other salts) at a pH 7.0 to 8.3 and a temperature of at least 25 °C. For example, conditions of 5X SSPE (750 mM NaCl, 50 mM Na phosphate, 5 mM EDTA, pH 7.4) and a temperature of 25-30 °C are suitable for allele-specific probe hybridizations. For stringent conditions, see for example, Sambrook, Fritsche and Maniatis, "Molecular Cloning A Laboratory Manual, 2nd Ed." Cold Spring Harbor Press (1989) and Anderson Nucleic Acid Hybridization, 1st Ed., BIOS Scientific Publishers Limited (1999). As used herein, the terms "hybridizing specifically to" or "specifically hybridizing to" or similar terms refer to the binding, duplexing, or hybridizing of a molecule substantially to a particular nucleotide sequence or sequences under stringent conditions.

Detectable labels associated with a particular position of a probe can be "readout" (e.g., its fluorescence detected) once or multiple times; a "readout" may be synonymous with the term "basecall". Multiple reads improve accuracy.

As used in herein, a "hybe and seq cycle" refers to all steps required to detect each attachment region on a particular probe or population of probes. For example, for a probe capable of detecting six positions on a target nucleic acid, one "hybe and seq cycle" will include, at least, hybridizing the probe to the target nucleic acid, hybridizing complementary nucleic acids/reporter complexes to attachment region at each of the six positions on the probe's barcode domain, and detecting the detectable labels associated with each of the six positions.

The term "k-mer probe" is synonymous with a probe of the present invention.

The methods described herein may be implemented and/or the results recorded using any device capable of implementing the methods and/or recording the results. Examples of devices that may be used include but are not limited to electronic computational devices, including computers of all types. When the methods described herein are implemented and/or recorded in a computer, the computer program that may be used to configure the computer to carry out the steps of the methods may be contained in any computer readable medium capable of containing the computer program. Examples of computer readable medium that may be used include but are not limited to diskettes, CD-ROMs, DVDs, ROM, RAM, non-transitory computer-readable media, and other memory and computer storage devices. The computer program that may be used to configure the computer to carry out the steps of the methods, identify the bound target nucleic acids, and/or record the results may also be provided over an electronic network, for example, over the internet, an intranet, or other network.

A "Consumable Card" can be incorporated into a fluorescence imaging device known in the art. Any fluorescence microscope with a number of varying features is capable of performing this readout. For instance: wide-field lamp, laser, LED, multi-photon, confocal or total-internal reflection illumination can be used for excitation and/or detection. Camera (single or multiple) and/or Photomultiplier tube (single or multiple) with either filter-based or grating-based spectral resolution (one or more spectrally resolved emission wavelengths) are possible on the emission-detection channel of the fluorescence microscope. Standard computers can control both the Consumable Card, the reagents flowing through the Card, and detection by the fluorescence microscope.

Probes can be detected and quantified using commercially-available cartridges, software, systems, *e.g.*, the nCounter® System using the nCounter® Cartridge.

Additional teaching relevant to the present invention are described in one or more of the following: U.S. 8,148,512, U.S. 7,473,767, U.S. 7,919,237, U.S. 7,941,279, U.S. 8,415,102, U.S. 8,492,094, U.S. 8,519,115, U.S. 2009/0220978, U.S. 2009/0299640, U.S. 2010/0015607, U.S. 2010/0261026, U.S. 2011/0086774, U.S. 2011/0145176, U.S. 2011/0201515, U.S. 2011/0229888, U.S. 2013/0004482, U.S. 2013/0017971, U.S. 2013/0178372, U.S. 2013/0230851, U.S. 2013/0337444, U.S. 2013/0345161, U.S. 2014/0005067, U.S. 2014/0017688, U.S. 2014/0037620, U.S. 2014/0087959, U.S. 2014/0154681, U.S. 2014/0162251, and U.S. 14/946,386,.

## EXAMPLES

### Example 1: The present invention provides rapid and highly efficient detection of target nucleic acids

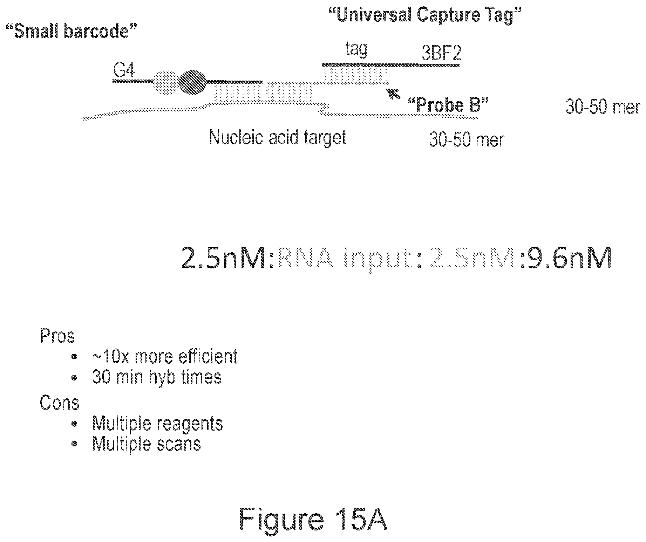

In Figure 15A "small barcodes" probes contained barcode sequences and target detection sequences in the range of 30-50mer. "Probe B" had a specific sequence (30-50mer) and a universal tag with biotin for deposition to a surface.

High concentrations of probes may be provided and applied to a sample comprising target nucleic acids. Probes of the present invention can be provided at 10 fold to 1000 fold higher concentrations than probes comprising detectable labels. Such high concentrated probes, in part, provide rapid detection of target nucleic acids.

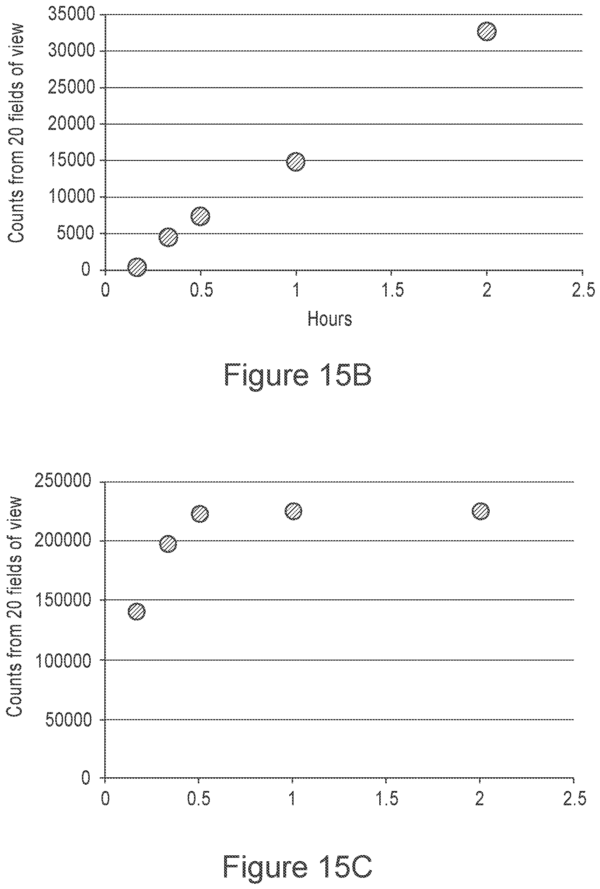

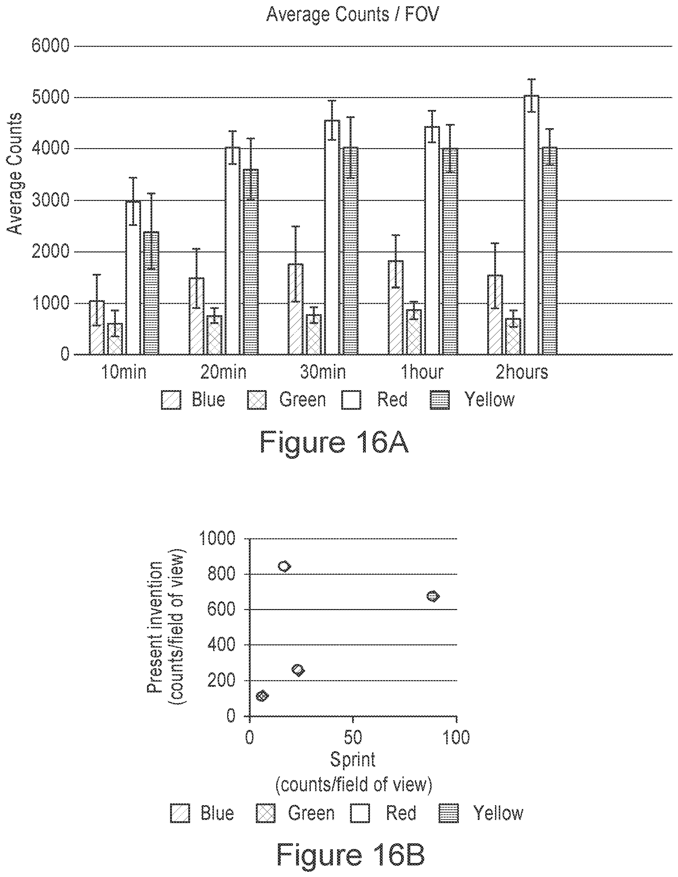

In Figure 15B, probes were provided at 250pM and in Figure 15C, probes were provided at 2.5nM. In other experiments utilizing standard nCounter workflow, probes comprising detectable labels are provided at 25pM. In Figure 15A, capture probes were provided at 100pM and in Figure 15B, capture probes were provided at 2.5nM. In other experiments utilizing standard nCounter workflow, capture probes comprising detectable labels are provided at 100pM. Figures 15A and 15B show that a target nucleic acid can be detected after ten minutes. Significant target detection in these experiments was achieved with the lower concentration probes in about two hours and within about thirty minutes with the higher concentration probes (Fig 16A). In other experiments utilizing standard nCounter workflow, probes comprising detectable labels require about sixteen and a half hours to detect a target nucleic acid.

Figure 16A shows average counts when four target nucleic acids were simultaneously detected using probes and methods of the present invention. Here, the four target nucleic acids were Myc (green), Oaz1 (blue), RPL13A (orange), and TubB (red). Probes and capture probes were provided at 2.5nM, target nucleic acid was 100ng of human reference RNA. Figure 16B shows that the present methods are about nine-times more efficient than methods in which probes are provided with detectable labels (identified in the Figure as "Sprint"). These results show a marked increase in efficiency in a much shorter time compared to standard nCounter workflow.

**Example 2: Sample preparation for processing FFPE tissue for use in Hyb & Count**

First, the nucleic acid(s) to be sequenced is extracted from formalin-fixed, paraffin embedded (FFPE) tissue in a single-step process. One or more 10 μm thick FFPE curl is heated in an aqueous-based nucleic acid extraction buffer to simultaneously melt the paraffin wax, decompose the tissue, and release nucleic acid from the cells. Suitable extraction buffers are known in the art and typically include proteinases, detergents such as Triton-100, chelating agents such as EDTA, and ammonium ions. The FFPE curl and extraction buffer are incubated at 56 °C for 30 minutes to separate the paraffin from the tissue and allow the Proteinase K to digest the tissue structure and expose the embedded cells to the detergent to enable cell lysis. The solution is inverted three times at 8 minute intervals to assist in mixing of the reagents during the tissue deparaffinization and digestion process. Following this step, the solution is heated to 98 °C to facilitate the reversal of the formaldehyde cross-links to further assist in the extraction of nucleic acids.

Once the nucleic acids have been extracted from the FFPE tissue, the solution is filtered using a glass fiber filter with 2.7 μm pore size (Whatman) to remove tissue debris and congealed paraffin. The resulting solution is a homogenous, semi-opaque solution containing nucleic acids which are highly fragmented due to the formalin-fixation process and storage conditions. If further fragmentation is required, the DNA can be mechanically sheered using a Covaris focused-ultrasonicator. Due to buffer conditions, extended sonication is required to shear the nucleic acids. Sonicating using the standard settings of 50W peak incident power, 20% duty factor, 200 cycles/burst were used for 600 seconds to achieve the maximal increase in targets captured (as seen in figure). To achieve shorter fragment length, emulsified paraffin can be precipitated out of the filtered solution by centrifuging at 21,000 g and 4 °C for 15 minutes. This allows the DNA to be sheared down to about 225 bp.

Next, target capture is performed by binding pairs of capture probes to targets during a rapid hybridization step. The 5' capture probe contains a 3' biotin moiety which allows the target the bind to the strepdavidin-coated flow cell surface during the target deposition process. The 3' capture probe contains a 5' tag sequence (G-sequence) that enables binding to beads during the purification process. The reaction rate is driven by the capture probe concentration which are added in the low nanomolar range to maximize the reaction rate. The capture probes hybridize to the target in a manner that flanks to region of interest in order to generate a window. For each DNA target, the capture probe set also includes an oligo composed of the same sequence as the window to hybridize to targets' antisense strand and prevent reannealing. The solution containing the capture probes is heated to 98 °C for 3 minutes to denature the genomic DNA, followed by a 15-minute incubation at 65 °C. The concentration of NaCl in the range of 400 mM to 600 mM is used for this hybridization reaction. A panel of over 100 targets that have been experimentally validated is listed in the Table 2, detailing the gene and exon of the targeted DNA region.

**Table 2**

| Gene | Target |
|------|--------|
| ABL1 | ABL1_ex4 |
| | ABL1_ex6 |
| | ABL1_ex7 |
| AKT1 | AKT1_ex6 |
| ALK | ALK_ex26 |
| APC | APC_ex5 |
| | APC_ex16 |
| | APC_ex17 |
| | APC_ex17 |
| | APC_ex17 |
| | APC_ex17 |
| | APC_ex17 |

(continued)

| Gene | Target |
|------|--------|
| ATM | ATM_ex8<br>ATM_ex9<br>ATM_ex11<br>ATM_ex26<br>ATM_ex34<br>ATM_ex39<br>ATM_ex49<br>ATM_ex49<br>ATM_ex55<br>ATM_ex59 |
| BRAF | BRAF_ex8<br>BRAF_ex11<br>BRAF_ex13<br>BRAF_ex15 |
| CDH1 | CDH1_ex9 |
| CSF1R | CSF1R_ex3<br>CSF1R_ex22 |
| CTNNB1 | CTNNB1_ex3<br>CTNNB1_ex6<br>CTNNB1_ex16 |
| EGFR | EGFR_ex3<br>EGFR_ex10<br>EGFR_ex15<br>EGFR_ex18<br>EGFR_ex20<br>EGFR_ex21 |
| ERBB2 | ERBB2_ex7 |
| ERBB4 | ERBB4_ex4<br>ERBB4_ex5<br>ERBB4_ex7<br>ERBB4_ex8<br>ERBB4_ex23<br>ERBB4_ex25 |
| EZH2 | EZH2_ex8<br>EZH2_ex11<br>EZH2_ex15 |
| FBXW7 | FBXW7_ex2<br>FBXW7_ex5<br>FBXW7_ex7<br>FBXW7_ex8<br>FBXW7_ex9<br>FBXW7_ex10 |
| FGFR1 | FGFR1_ex6 |
| FGFR2 | FGFR2_ex7 |
| FLT3 | FLT3_ex11<br>FLT3_ex12<br>FLT3_ex21 |

(continued)

| Gene | Target |
|------|--------|
| GNAQ | GNAQ_ex5 |
| IDH1 | IDH1_ex4<br>IDH1_ex10 |
| IDH2 | IDH2_ex4 |
| JAK2 | JAK2_ex3<br>JAK2_ex7<br>JAK2_ex14<br>JAK2_ex20 |
| KDR | KDR_ex7<br>KDR_ex7<br>KDR_ex9<br>KDR_ex11<br>KDR_ex27<br>KDR_ex30 |
| KIT | KIT_ex5<br>KIT_ex9<br>KIT_ex14<br>KIT_ex14<br>KIT_ex17<br>KIT_ex18 |
| KRAS | KRAS_ex2<br>KRAS_ex3<br>KRAS_ex4 |
| MEK | MEK_ex3 |
| MET | MET_ex2<br>MET_ex3<br>MET_ex11<br>MET_ex14<br>MET_ex16<br>MET_ex19 |
| MLH1 | MLH1_ex12<br>MLH1_ex16 |
| NOTCH1 | NOTCH1_ex26 |
| NRAS | NRAS_ex2<br>NRAS_ex3<br>NRAS_ex3<br>NRAS_ex4 |
| PDGFRA | PDGFRA_ex1<br>PDGFRA_ex4<br>PDGFRA_ex7<br>PDGFRA_ex10<br>PDGFRA_ex11<br>PDGFRA_ex14<br>PDGFRA_ex15<br>PDGFRA_ex16<br>PDGFRA_ex18<br>PDGFRA_ex23 |

(continued)

| Gene | Target |
|---|---|
| PIK3CA | PIK3CA_ex2<br>PIK3CA_ex3<br>PIK3CA_ex7<br>PIK3CA_ex10<br>PIK3CA_ex14<br>PIK3CA_ex21<br>PIK3CA_ex21 |
| PTEN | PTEN_ex5<br>PTEN_ex7<br>PTEN_ex8 |
| PTENP1 | PTENP1_ex1 |
| RB1 | RB1_ex10<br>RB1_ex17<br>RB1_ex17 |
| | RB1_ex20<br>RB1_ex22 |
| RET | RET_ex12<br>RET_ex15 |
| SMAD4 | SMAD4_ex3<br>SMAD4_ex8<br>SMAD4_ex9<br>SMAD4_ex10<br>SMAD4_ex11 |
| SMARCB1 | SMARCB1_ex5 |
| TP53 | TP53_ex4<br>TP53_ex6 |

After the targeted DNA regions are bound with capture probes, they are purified from the rest of the genomic DNA to create an enriched solution of the targets. Beads coated with the antisense oligo (anti G-sequence) to the 3' capture probes' binding sequence are incubated with the capture reaction mix for 15 minutes at room temperature. After the binding step, the beads are washed three times with 0.1x SSPE to remove non-target DNA and the biotin-containing 5' capture probes. Following the washes, the beads are re-suspended in 14 $\mu$L of 0.1x SSPE then heated at 45 °C for 10 minutes to elute the purified DNA targets from the beads. After elution, 1 $\mu$L of 5 M NaCl is added to ensure the capture probes remain bound to the DNA targets.

The final step of the sample preparation process is the deposition of the DNA targets onto the flow cell surface, where they can be analyzed using the probes of the present invention as disclosed herein. A syringe pump is utilized to control the rate at which the targets are loaded into the flow cell fluidic channel, such that all targets have time to diffuse across the height of the channel and bind to the streptavidin surface. This method of loading generates a density gradient of targets, where the highest number of molecules per unit area is greatest at the fluidic channel inlet and decreases along the channel length in the direction of the fluidic flow towards the outlet. A flow rate of 0.35 $\mu$L/second achieves a quantitative capture within a channel length of about 10 mm for a channel width of 1.6 mm and height of 40 $\mu$m. Once the targets are bound to the surface by the biotinylated 5' capture probe, a solution of biotinylated oligo (G-hooks) that are the reverse complement of the 3' capture probes' bind sequence are injected to pin down the free end of the targets to create a bridged structure, where the ssDNA region in the middle is the window of interest. Next, a solution of G-sequence oligos are added to hybridize to the excess G-hooks on the surface to reduce the amount of ssDNA on the surface.

To identify the targets that have been enriched for, 15mer probes were designed such that they could specifically bind to a single target in the panel. These probes were synthesized with an adapter sequence on the 3' end that could attach them to a unique barcoding oligo. Each barcoding oligo contained three unique reporter binding domains capable of binding reporter probes for target identification, enabling a 64-plex readout using a four-color reporter chemistry. These identification probes are injected into the fluidic channel and incubated for 1 minute to allow to hybridize to the targets.

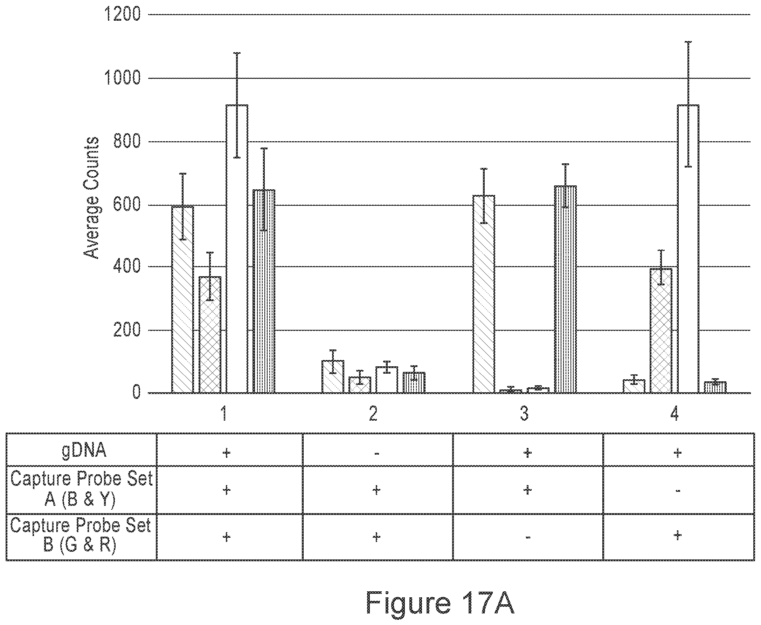

Subsequently, a stringent wash of 0.1x SSPE is used to remove unbound and non-specifically bound oligos. Three rounds of reporter probe hybridization are used to identify the targets, based on the targets' unique barcodes. The combination of the dual capture probe systems to capture select regions of the genome with the use of target-specific identification probes provides a highly specific system for target enrichment and detection. Figure 17A depicts the specificity in which a panel of 40 targets are captured and enumerated from 3 µg of purified, sheared gDNA. Lane 1 demonstrates the general target detected counts when all capture probes are used together, compared to lane 2 where no gDNA was present. The specificity of the system is verified by including only capture probes that enrich for targets that are detected with blue and yellow reporters in lane 3 or green and red in lane 4.

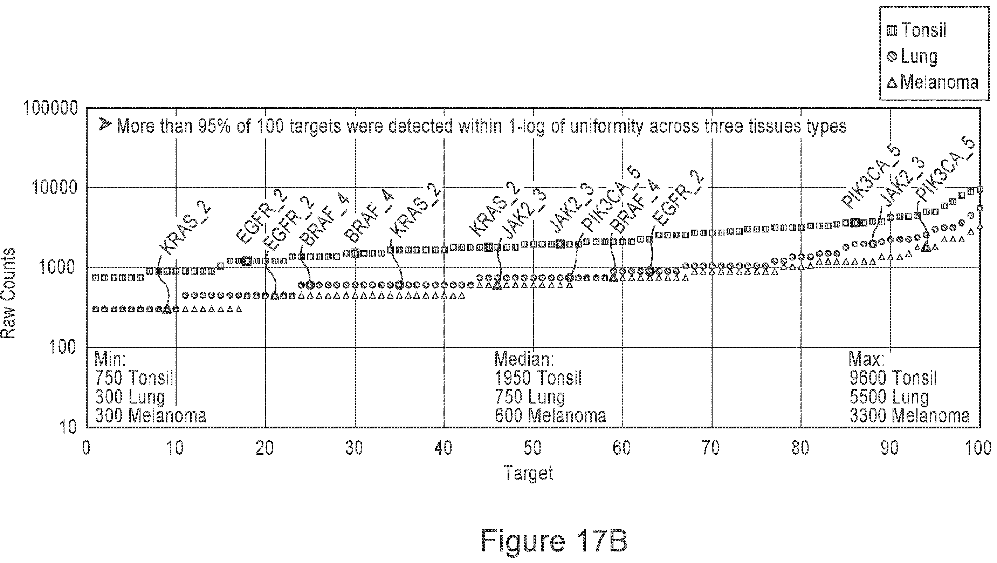

This workflow of DNA extraction, capture, and detection was applied to three FFPE tissue types: tonsil, lung, and melanoma. For all tissue types, capture and detection of a 100plex cancer target panel resulted in >95% of targets identified within 1-log uniformity. The counts for these targets across the three tissue types are displayed in Figure 17B.

### Example 3: Multi-color reporter image processing for Hyb & Seq

The image processing pipeline includes the following steps: background subtraction, registration, feature detection, and classification. In background subtraction, the mean background of any given channel is a function of shot noise and exposure. In our system, the blue channel has the highest background levels coupled with greater variance. A simple tophat filter with a circular structuring element of radius 7 pixels is applied to perform localized background subtraction.

For registration, it is imperative that the features of interest as perfectly aligned for multi-color and multi-cycle feature analysis. This system requires two forms of registration. For the first form, a local affine transformation is applied to all image channels within a single acquisition stack. This transformation is a function of the optical system and hence is consistent for a given instrument. This function is computed in advance for every run and is applied to every image acquired. For the second form, a global transformation in the form of a rigid shift is computed using normalized cross-correlation to capture drift of the mechanical gantry during the run.

The next step is feature detection. Once all the images are registered, feature are detected using a matched filter viz a LoG (Laplace of Gaussian) filter. The filter is applied with a fixed kernel size (matched to the diffraction limit of the features) and a varying standard deviation (matched to the wavelength of the corresponding channel) to match to enhance spot response. Local maxima are used to identify potential reporter locations. The associated intensity values for each identified feature are retrieved for classification.

The final step is classification. The multi-color reporter intensities are classified using the Gaussian naive-Bayes model. The model assumes that the reporter intensities are independent and follow a normal distribution. The model then calculates the probability that a specific feature $\hat{y}$ (specified by intensities in all channels $\widehat{x_i}$) belongs to a certain class ($C_k$) using a *maximum a posteriori* or MAP rule:

$$\hat{y} = argmax_{\{k \in \{1,...K\}\}} \, p(C_k) \prod_{i=1}^{n} p(x_i | C_k)$$

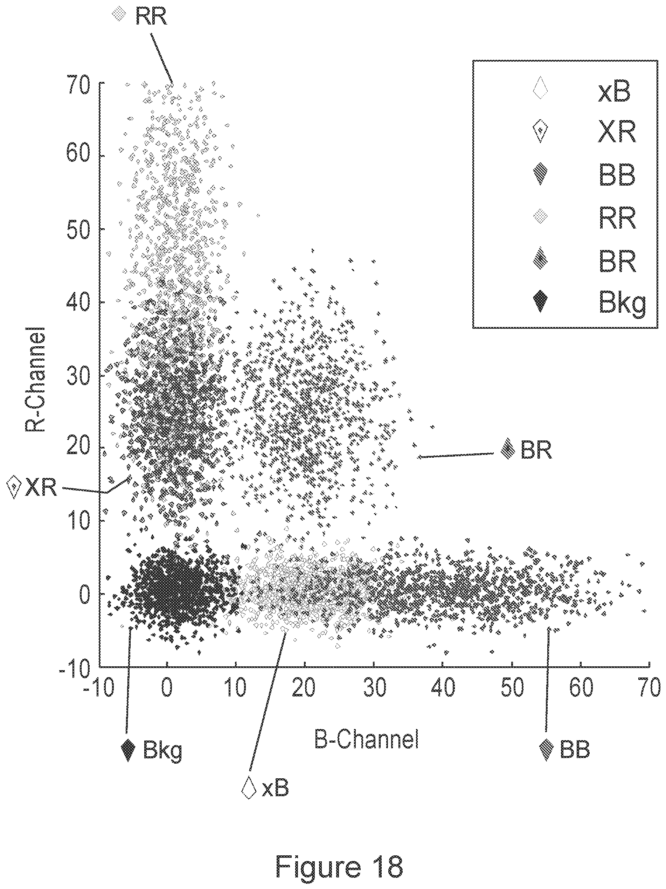

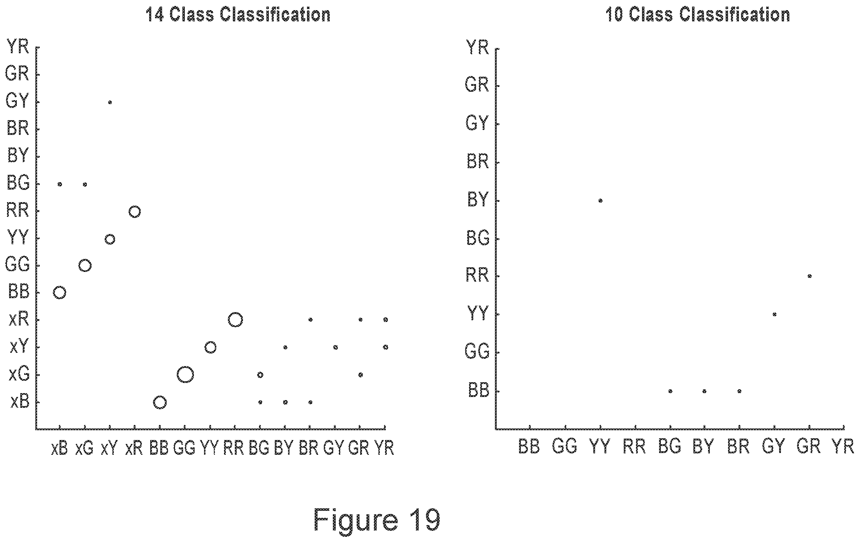

The intensity distributions for a dual color coded reported is shown in Figure 18. The figure illustrates the coding scheme using 2 dyes blue and red. There are six classes (including background) possible in a 2-color coding scenario. In the implemented system, the choice of four colors results in 14 potential classes. Note that there is some overlap between the single half dye vs full dye distributions. Consequently, classification between these classes presents a higher error rate as shown in Figure 19, with a maximum miss-classification rate of 11.8% between *'xG'* and *'GG'*. The miss-classification rates for the 10 Class model is less than 0.2%. Since each reporter requires a maximum of eight classes, it is simple to choose the ones with least classification error.

### Example 4: Function, design, preparation, and testing of two color reporter probes

Two-color reporter probes sequentially bind to three regions ($R_1$, $R_2$, $R_3$) in the barcode domain of the probe. Each region encodes eight "colors" defined by two-color fluorescent combinations such as "blue-blue" or "green-yellow". Three sequential "colors" are reported for each probe that, in turn, correspond to the reading of three dinucleotides that constitute the hexamer sequence. The two-color reporter probe is designed as follows: The two-color reporter probe is a 37 DNA oligomer branched structure designed to hold 15 fluorescent dyes for each color, with a total of 30 dyes per reporter probe. The 37 oligomers are classified into three sizes: (1) One 96 nt MainBranch consists of two parts, a 12-mer single-stranded DNA sequence later used for reporting of the hexamer and six 14-mers hybridized to six SubBranches, (2) Each of the six 89 nt SubBranches consist of two parts, one 14-mer hybridized to the MainBranch and five 15-mer repeats hybridized to five Dye oligos, (3) Each of the five 15 nt Dye oligos have one fluorescent dye modification at 5' end of the oligo.

One of the key design features for the two-color reporter probe is distinct SubBranch and Dye oligo sequences between the four different fluorescent dyes. This prevents "color-swapping" or cross-hybridization between the different fluorescent dyes. For example, each 15-mer Dye oligo for the Alexa 488 fluorophore, or blue color, corresponds to complementary sequences only to the blue SubBranch. The blue SubBranch further has a distinct 14-mer sequence that is complementary only to the blue 14-mer sequences on the MainBranch but not yellow, red, or green. Therefore, a specific MainBranch will have specific two-color sequences that dictate which 15 plus 15 dye combinations it will hold.

Another important design feature of the two-color reporter probe is the 12-mer sequence on the MainBranch which must satisfy the following: (1) distinct 12-mer sequences between $R_1$, $R_2$, and $R_3$ (2) encode eight different colors per region with high specificity (3) high binding efficiency and uniformity between the eight different colors and (4) efficient removal of all 12-mers through competitive toehold sequence.

The two-color reporter probe is prepared as described below. Four fluorescent dyes (B= blue, G= green, Y= yellow, R= red) make ten possible two-color combinations (BB, BG, BR, BY, GG, GR, GY, RR, YR, YY). Only eight of the ten two-color combinations are used for each of the three barcode regions of the probe, resulting in 24 different reporter probes (8+8+8 =24).

Preparation of the two-color reporter probe occurs in two sequential hybridization steps: (1) Dye oligos to SubBranch and then (2) Dye+SubBranch to MainBranch. Four separate Dye-to-SubBranch reactions are prepared by combining 100 uM of SubBranch and 600 uM of Dye oligo in 4.2X SSPE buffer at room temperature for 30 minutes. Twenty-four reporter probes are then prepared separately using 2 uM of MainBranch, 7.2 uM of SubBranch+Dye1, and 7.2 uM of SubBranch+Dye2 in 4.8X SSPE. These reactions are heated at 45C for 5 minutes and then cooled at room temperature for 30 minutes. The 24 Dye+SubBranch-to-MainBranch reactions are then pooled into three different pools corresponding to the barcode domain (i.e. $R_1$, $R_2$, $R_3$). For example, eight different two-color reporter probes (2 uM each) binding to the $R_1$ barcode domain are pooled together, diluting ten-fold to a final working concentration of 200 nM each reporter probe.

Following reporter probe preparation is standard testing for quality assurance. Each of the three pools of reporter probes are tested for binding to its corresponding barcode region ($R_1$, $R_2$, or $R_3$) in three separate flow cells. Testing is performed on a modified probe construct, with only the barcode domain present and immobilized on the flow cell. All eight 12-mers representing each color is multiplexed and all eight two-color reporter probes are expected to be identified with high color count.

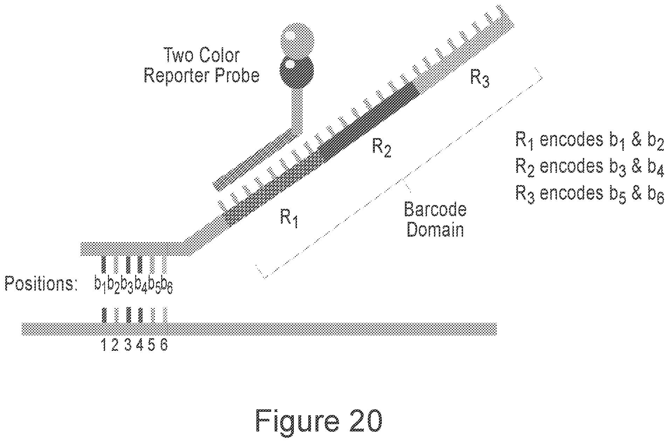

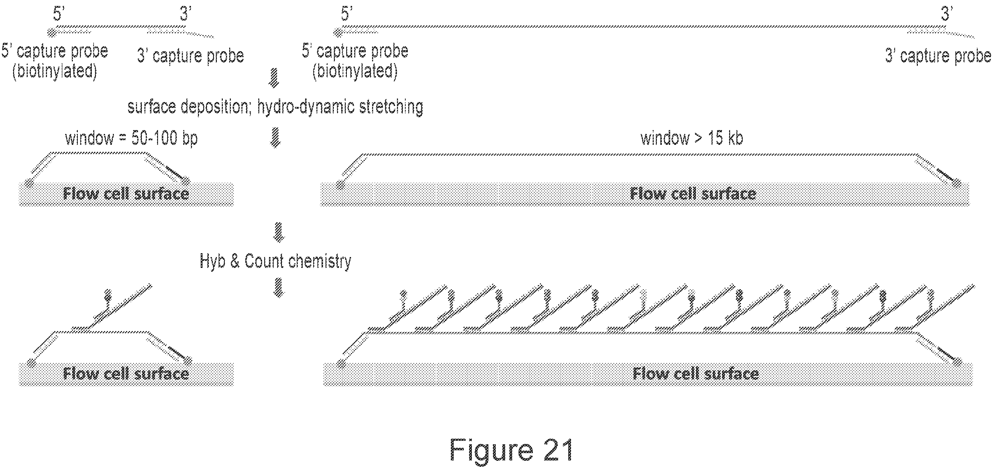

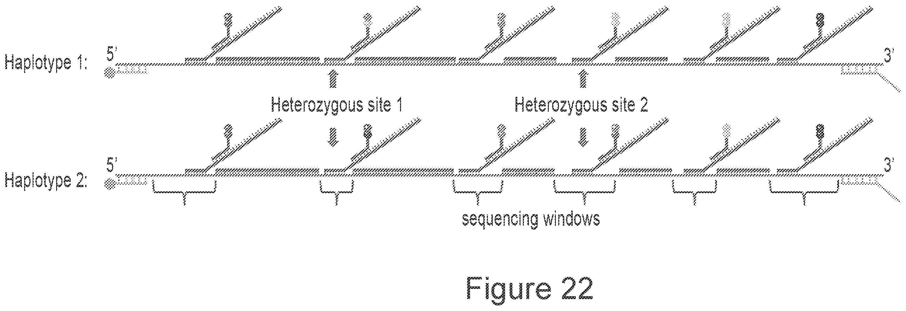

A schematic of a two color reporter probe is shown in Figure 20. These probes are used in the straightforward probe hybridization workflow for targeted capture of nucleic acids (depicted in Figure 21). Figure 22 shows additional capabilities of these probes with respect to their use of in identifying haplotypes of interest.

**Example 5: Three two color reporter probes and image subtraction**

The present example demonstrates pre-hybridization of three reporter complexes to the sequencing probe in solution prior to binding to the surface immobilized target. Solution hybridization is shown to be much more efficient than surface hybridization and can be performed in advance of the sequencing experiment to dramatically reduce total sample-to-answer runtime. The three reporter identities are determined by sequentially cleaving (via chemical or optical methods) the reporters off the sequencing probe and measuring the loss in fluorescent intensity.

The present disclosure requires the hybridization of one of a set of 4096 barcode molecules (BCs), also described herein as a probe in which the regions of the barcode domain may be bound by complementary nucleic acid molecules including a detectable label or complementary nucleic acid molecules of a reporter complex including a detectable label one for each possible hexamer sequence, to a target molecule which has been immobilized on the surface of a flow cell. The identity of the barcode, and the associated hexamer sequence within the target, requires binding and readout of 3 two-color fluorescent reporter probes (RPTRs), also described herein as a complementary nucleic acid molecule including a detectable label or a complementary nucleic acid molecule of a reporter complex including a detectable label. RPTRs are flowed into the flow cell to hybridize to the BC, imaged, and removed by toe holding in a sequential manner, requiring three RPTR flow cycles for each BC readout.

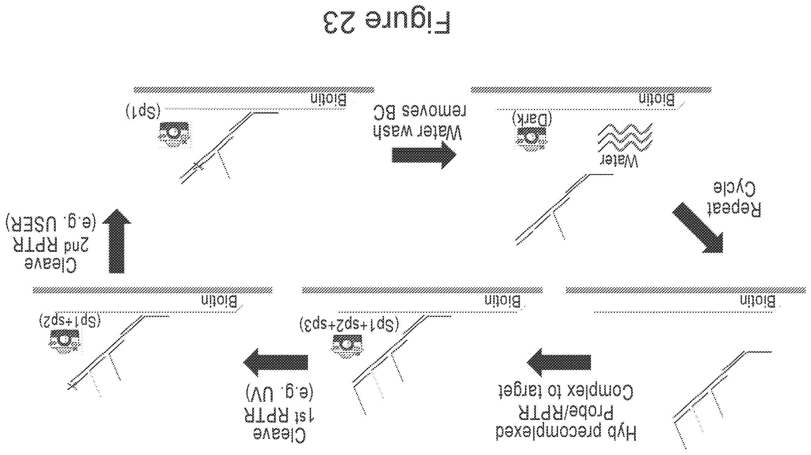

Figure 23 shows hybridization of all three RPTR probes to the BCs prior to being flowed into a flow cell. This BC/RPTR complex can be purified prior to use to ensure near 100% of BC/RPTR complexes are properly formed. The BC/RPTR complex is hybridized to a target on the surface and an image is taken that contains the fluorescent signal from all 6 colors (3 two-color RPTRs). One of the reporters is then cleaved, removing the fluorescent dyes from the complex. Cleavage mechanisms are discussed in greater detail herein. A second image is then taken which contains the fluorescent signal from only 4 colors (2 two-color RPTRs).

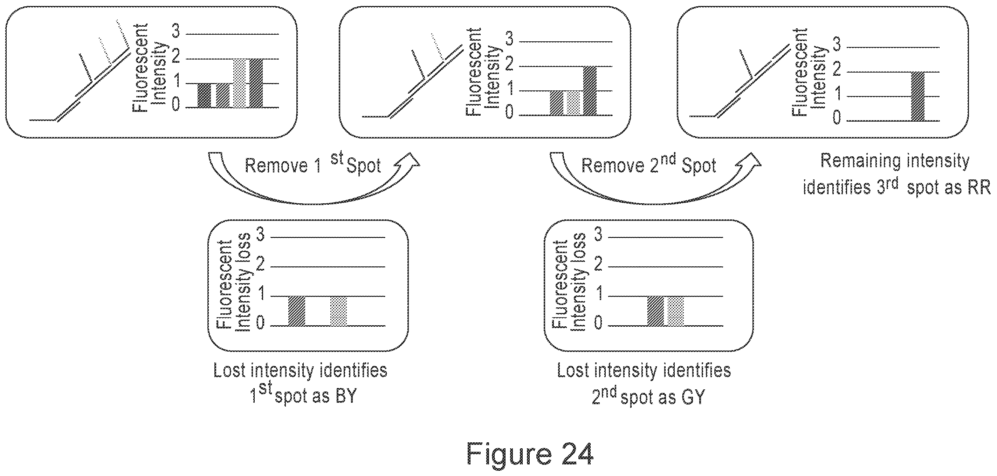

As shown in Figure 24, the identity of the lost RPTR can be obtained by comparison of the 6 color and 4 color images. Next, a second RPTR is removed using a different cleavage mechanism and a third image is taken which contains the fluorescent signal from 2 colors (1 two-color RPTR). Again, the cleaved RPTR's identity is determined by comparison of the 2 color and 4 color images. The remaining fluorescence signal identifies the third RPTR to unambiguously identify the BC and thus the hexamer sequence present in the target.

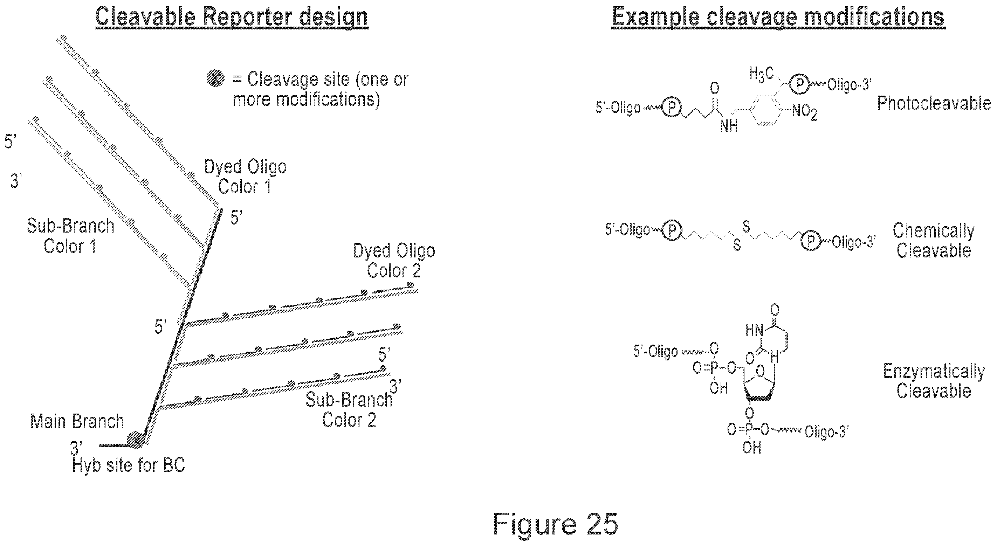

The cleavable RPTRs used in the first two readouts of the sequencing cycle are constructed similarly to the uncleavable version, consisting of 30 dyed oligos hybridized to 6 "Sub-Branch" oligos, also described herein as tertiary nucleic acid molecules, which are finally hybridized to a "Main Branch" oligo, also described herein as a secondary nucleic acid molecule, as shown in Figure 25. These RPTRs are made cleavable by synthesizing the "Main Branch" oligo with one or more of any of several cleavable modifications, such as photo-cleavable, chemically cleavable and enzymatically cleavable, placed between the portion of the "Main Branch" that binds the BC and the portion that binds the "Sub-Branches" and dyes. An example of a chemically cleavable modification incudes a disulfide moiety. An example of an enzymatically cleavable modification incudes a deoxyuracil (dU) containing moiety (cleavable using 'USER' enzyme mix from New England Biolabs. The cleavable modifications used for the two RPTRs within one sequencing cycle must be different to allow sequential cleavage.

Key attributes and advantages of this method are: (1) BC/RPTR complex can be prepared in advance of the sequencing run which permits greater control over hybridization (i.e. solution hyb instead of surface hyb and much longer hyb times); (2) This method has the potential to dramatically increase the number of BCs identified unambiguously because (a) BC/RPTR complexes can be HPLC purified to ensure each BC has all three RPTRs and (b) Cleavage efficiency is significantly higher than RPTR hybridization and toehold efficiencies; and (3) This method is much faster in terms of sequencing run time because (a) it does not require hybridization time for each RPTR binding to the BC, (b) Cleavage kinetics are significantly faster than toeholding, which is also hybridization based, for removing RPTR signals and (c) it requires many fewer reagent flow steps (8 vs 14 for the current method, though if using UV cleavable linkers only 6 flow steps are required). It also requires fewer images to be taken (4 vs 7 images, or if a final water wash dark image is omitted, 3 vs 6 images).

A proof of principle experiment was performed using a single BC, a UV-cleavable RPTR, a deoxyuracil (dU) containing RPTR (cleavable using 'USER' enzyme mix from New England Biolabs), and a standard RPTR. These components were hybridized into a BC/RPTR complex and hybridized to a synthetic 50mer BRAF exon 15 target sequence immobilized on a flow cell. The spot identities were determined by first imaging the full BC/RPTR complex followed by treatment with the USER enzyme to remove the dU containing RPTR and imaging again. Next, the photocleavable RPTR was cleaved using UV light exposure and a third image was taken as shown in Figure 27. Four clustered features in the images were processed to determine their fluorescent intensities and simple subtraction correctly identified the three RPTR identities.

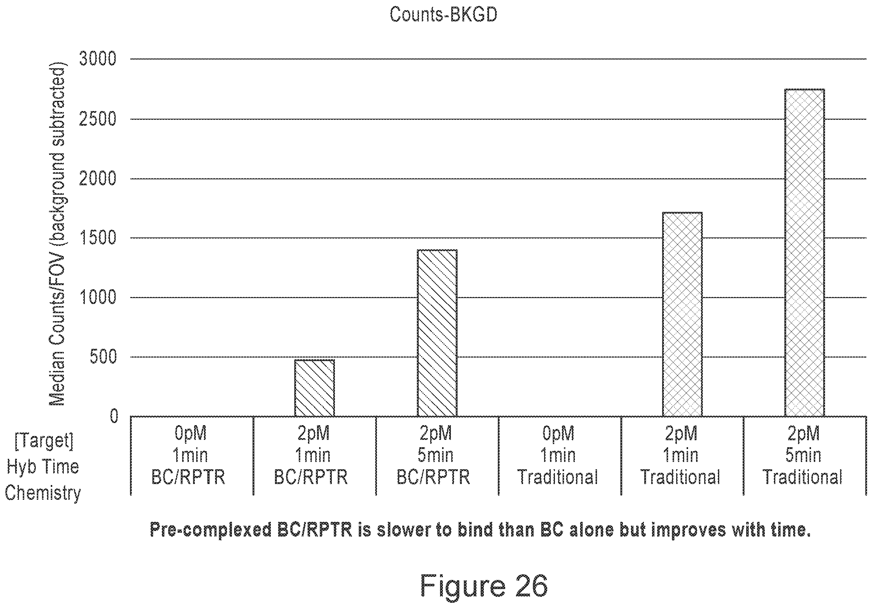

A major potential risk for this approach was the size of the BC/RPTR complex and the associated slowing of hybridization kinetics to the surface immobilized target. The increased size of the BC/RPTR complex relative to the BC alone does indeed slow binding kinetics; however, this can be overcome with longer incubation times as shown in Figure 26. The loss in hybridization time here can be offset by efficiency and speed reductions in other steps (i.e., elimination of RPTR hybs, reduction in imaging, reduction in flow steps, etc.).

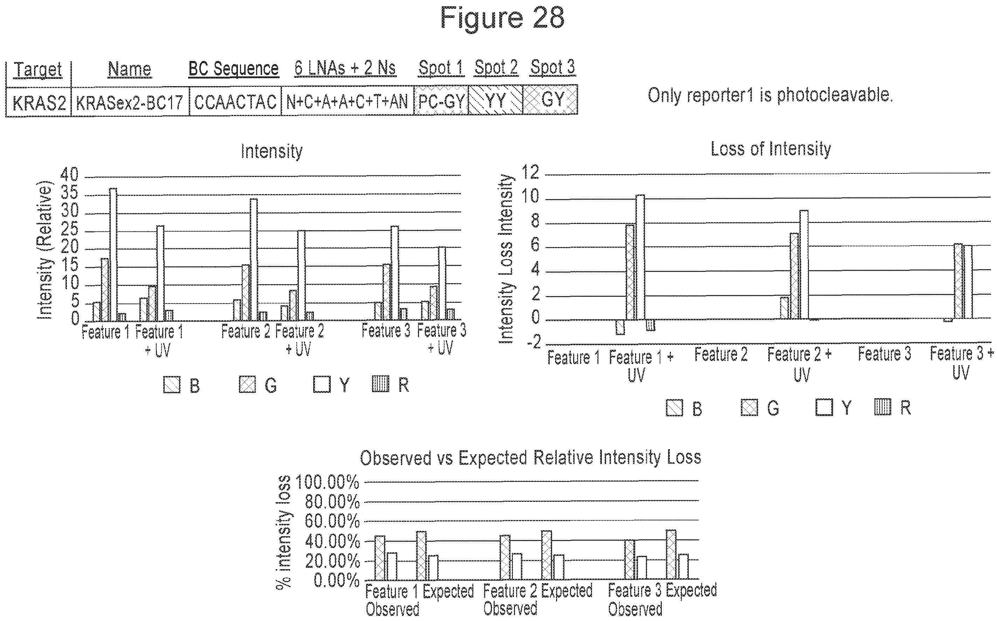

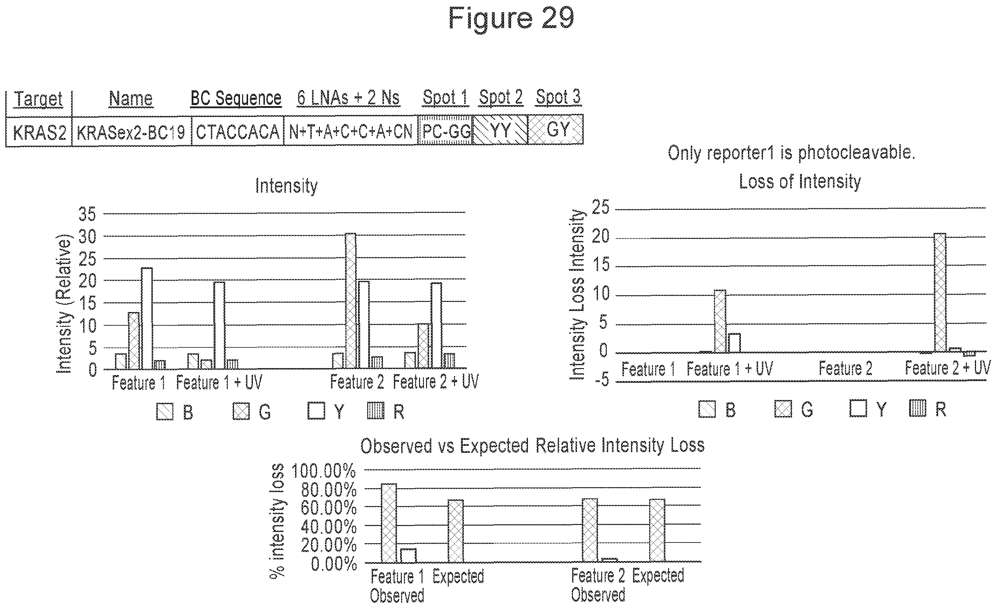

We also tested whether half-dye RPTRs can be detected using this image subtraction method. As these dyes have a smaller signal, they may be more difficult to reliably identify. To test this, a set of barcodes with many similar color RPTRs (mainly Green and Yellow) were prepared where only the spot 1 RPTR was cleavable as shown in Figure 28 and Figure 29. Images were taken before and after UV exposure to cleave the spot 1 RPTR. Both a PC-GY and PC-GG were detectable and yielded similar intensity changes to that expected by the number of dyes lost (e.g. a GYYYGY RPTR cleaved to a _YYGY would have lost 50% of its Green and 25% of its Yellow). A series of dye colors and class and related sequences are shown in the following table.

| SEQ ID NO | Class | Color | Name | Length (nt) | Full Sequence |
|---|---|---|---|---|---|
| 71 | Dye oligo | B | 5x dye B | 15 | /5Alex488N/CCTGCGAATGAGTCG |
| 72 | Dye oligo | G | 5x dye G | 15 | /5Alex546N/TCGAGTGCATGAGCT |
| 73 | Dye oligo | R | 5x dye R | 15 | /5Alex647N/AGTAGACCTGGCGTC |
| 74 | Dye oligo | Y | 5x dye Y | 15 | /5TexRd-XN/ATCACCGTGCAGCTA |

(continued)

| SEQ ID NO | Class | Color | Name | Length (nt) | Full Sequence |
|---|---|---|---|---|---|
| 75 | SubBranch | B | 5x6 dye B | 89 | TGCGACGCACCTATCGACTCATTCGCAGGCGACTCATTCGCAGGCGACTCATTCGCAGGCGACTCATTCGCAGGCGACTCATTCGCAGG |
| 76 | SubBranch | G | 5x6 dye G | 89 | AAGGTGTGCAGCCTAGCTCATGCACTCGAAGCTCATGCACTCGAAGCTCATGCACTCGAAGCTCATGCACTCGAAGCTCATGCACTCGA |
| 77 | SubBranch | R | 5x6 dye R | 89 | ACTGTTGCCGCCAAGACGCCAGGTCTACTGACGCCAGGTCTACTGACGCCAGGTCTACTGACGCCAGGTCTACTGACGCCAGGTCTACT |
| 78 | SubBranch | Y | 5x6 dye Y | 89 | AACGCCATTTGCCGTAGCTGCACGGTGATTAGCTGCACGGTGATTAGCTGCACGGTGATTAGCTGCACGGTGATTAGCTGCACGGTGAT |
| 79 | MainBranch | GG | R1 GG | 96 | AGGCTGCACACCTTAGGCTGCACACCTTAGGCTGCACACCTTAGGCTGCACACCTTAGGCTGCACACCTTAGGCTGCACACCTTAGGCGAGATGAC |
| 80 | MainBranch | GY | R1 GY | 96 | AGGCTGCACACCTTAGGCTGCACACCTTAGGCTGCACACCTTCGGCAAATGGCGTTCGGCAAATGGCGTTCGGCAAATGGCGTTAGGGAAGATGAC |
| 81 | MainBranch | YY | R1 YY | 96 | CGGCAAATGGCGTTCGGCAAATGGCGTTCGGCAAATGGCGTTCGGCAAATGGCGTTCGGCAAATGGCGTTCGGCAAATGGCGTTAGGGTGGATGAC |
| 82 | MainBranch | BB | R1 BB | 96 | ATAGGTGCGTCGCAATAGGTGCGTCGCAATAGGTGCGTCGCAATAGGTGCGTCGCAATAGGTGCGTCGCAATAGGTGCGTCGCAAGGACAGATGAC |
| 83 | MainBranch | RR | R1 RR | 96 | TTGGCGGCAACAGTTTGGCGGCAACAGTTTGGCGGCAACAGTTTGGCGGCAACAGTTTGGCGGCAACAGTTTGGCGGCAACAGTAGGTTAGATGAC |
| 84 | MainBranch | GR | R1 GR | 96 | AGGCTGCACACCTTAGGCTGCACACCTTAGGCTGCACACCTTTTGGCGGCAACAGTTTGGCGGCAACAGTTTGGCGGCAACAGTGTAGAAGATGAC |
| 85 | MainBranch | YR | R1 YR | 96 | CGGCAAATGGCGTTCGGCAAATGGCGTTCGGCAAATGGCGTTTTGGCGGCAACAGTTTGGCGGCAACAGTTTGGCGGCAACAGTAGGAACGATGAC |
| 86 | MainBranch | BR | R1 BR | 96 | ATAGGTGCGTCGCAATAGGTGCGTCGCAATAGGTGCGTCGCATTGGCGGCAACAGTTTGGCGGCAACAGTTTGGCGGCAACAGTAGGAGTGATGAC |
| 87 | MainBranch | BB | R2 BB | 96 | ATAGGTGCGTCGCAATAGGTGCGTCGCAATAGGTGCGTCGCAATAGGTGCGTCGCAATAGGTGCGTCGCAATAGGTGCGTCGCAAGCCATGAAAAG |
| 88 | MainBranch | BG | R2 BG | 96 | ATAGGTGCGTCGCAATAGGTGCGTCGCAATAGGTGCGTCGCAAGGCTGCACACCTTAGGCTGCACACCTTAGGCTGCACACCTTAGCGCTGAAAAG |
| 89 | MainBranch | BY | R2 BY | 96 | ATAGGTGCGTCGCAATAGGTGCGTCGCAATAGGTGCGTCGCACGGCAAATGGCGTTCGGCAAATGGCGTTCGGCAAATGGCGTTAGCATCGAAAAG |
| 90 | MainBranch | GG | R2 GG | 96 | AGGCTGCACACCTTAGGCTGCACACCTTAGGCTGCACACCTTAGGCTGCACACCTTAGGCTGCACACCTTAGGCTGCACACCTTAGCCGAGAAAAG |
| 91 | MainBranch | GR | R2 GR | 96 | AGGCTGCACACCTTAGGCTGCACACCTTAGGCTGCACACCTTTTGGCGGCAACAGTTTGGCGGCAACAGTTTGGCGGCAACAGTAGCTGGGAAAAG |
| 92 | MainBranch | GY | R2 GY | 96 | AGGCTGCACACCTTAGGCTGCACACCTTAGGCTGCACACCTTCGGCAAATGGCGTTCGGCAAATGGCGTTCGGCAAATGGCGTTAGCGAAGAAAAG |
| 93 | MainBranch | RR | R2 RR | 96 | TTGGCGGCAACAGTTTGGCGGCAACAGTTTGGCGGCAACAGTTTGGCGGCAACAGTTTGGCGGCAACAGTTTGGCGGCAACAGTAGCTCGGAAAAG |
| 94 | MainBranch | YY | R2 YY | 96 | CGGCAAATGGCGTTCGGCAAATGGCGTTCGGCAAATGGCGTTCGGCAAATGGCGTTCGGCAAATGGCGTTCGGCAAATGGCGTTAGCGTGGAAAAG |
| 95 | MainBranch | BG | R3 BG | 96 | ATAGGTGCGTCGCAATAGGTGCGTCGCAATAGGTGCGTCGCAAGGCTGCACACCTTAGGCTGCACACCTTAGGCTGCACACCTTGTAAGTCCGAAT |
| 96 | MainBranch | YR | R3 YR | 96 | CGGCAAATGGCGTTCGGCAAATGGCGTTCGGCAAATGGCGTTTTGGCGGCAACAGTTTGGCGGCAACAGTTTGGCGGCAACAGTGTAACACCGAAT |
| 97 | MainBranch | BB | R3 BB | 96 | ATAGGTGCGTCGCAATAGGTGCGTCGCAATAGGTGCGTCGCAATAGGTGCGTCGCAATAGGTGCGTCGCAATAGGTGCGTCGCAGTACATCCGAAT |
| 98 | MainBranch | BY | R3 BY | 96 | ATAGGTGCGTCGCAATAGGTGCGTCGCAATAGGTGCGTCGCACGGCAAATGGCGTTCGGCAAATGGCGTTCGGCAAATGGCGTTGTAATCCCGAAT |
| 99 | MainBranch | GG | R3 GG | 96 | AGGCTGCACACCTTAGGCTGCACACCTTAGGCTGCACACCTTAGGCTGCACACCTTAGGCTGCACACCTTAGGCTGCACACCTTGTACGACCGAAT |

(continued)

| SEQ ID NO | Class | Color | Name | Length (nt) | Full Sequence |
|---|---|---|---|---|---|
| 100 | MainBranch | GY | R3 GY | 96 | AGGCTGCACACCTTAGGCTGCACACCTTAGGCTGCACACCTTCGGCAAAT GGCGTTCGGCAAATGGCGTTCGGCAAATGGCGTTGTAGAACCGAAT |
| 101 | MainBranch | RR | R3 RR | 96 | TTGGCGGCAACAGTTTGGCGGCAACAGTTTGGCGGCAACAGTTTGGCGG CAACAGTTTGGCGGCAACAGTTTGGCGGCAACAGTGTATCGCCGAAT |
| 102 | MainBranch | GR | R3 GR | 96 | AGGCTGCACACCTTAGGCTGCACACCTTAGGCTGCACACCTTTTGGCGGC AACAGTTTGGCGGCAACAGTTTGGCGGCAACAGTGTAGTTCCGAAT |

**Claims**

1. A complex comprising:

   a probe capable of binding a specific region of at least one target nucleic acid, wherein the at least one probe comprises a target binding domain and a barcode domain,
   wherein the target binding domain comprises 10 - 100 nucleotides and is capable of binding the specific region of the target nucleic acid, and wherein the target binding domain comprises a known nucleotide sequence,
   wherein the barcode domain comprises a first attachment region bound by a first complementary nucleic acid molecule and an at least second attachment region bound by an at least second complementary nucleic acid molecule,
   wherein the first complementary nucleic acid molecule is a first complementary nucleic acid molecule of a first reporter complex, wherein the first reporter complex comprises:

   i) the first complementary nucleic acid molecule indirectly linked to a primary nucleic acid molecule via a photocleavable linker; and
   ii) a first detectable label, thereby associating a detectable label with the first attachment region,

   wherein the second complementary nucleic acid molecule is a second complementary nucleic acid molecule of a second reporter complex, wherein the second reporter complex comprises a second detectable label, thereby associating a detectable label with the at least second attachment region,
   wherein the sequences of the first attachment region and the at least second attachment region are different.

2. The complex of claim 1, wherein the target binding domain comprises:

   i) 20-60 nucleotides; or
   ii) 35-50 nucleotides.

3. The complex of claim 1 or claim 2, wherein the second reporter complex comprises the second complementary nucleic acid molecule indirectly linked to a primary nucleic acid molecule via a photocleavable linker.

4. The complex of any one of claims 1-3, wherein the barcode domain further comprises an at least third attachment region bound by an at least third complementary nucleic acid,
   wherein the third complementary nucleic acid molecule comprises a third detectable label, thereby associating a detectable label with the at least third attachment region.

5. The complex of claim 4, wherein the third complementary nucleic acid molecule is a third complementary nucleic acid molecule of a third reporter complex, wherein the third reporter complex comprises the third complementary nucleic acid molecule is indirectly linked to a primary nucleic acid molecule via a photocleavable linker.

6. The complex of any one of claims 1-5, wherein the primary nucleic acid molecules are hybridized to at least one, at least two, at least three, at least four, at least five or at least six secondary nucleic acid molecules, optionally wherein second nucleic acid molecules comprise a photocleavable linker and/or at least one detectable label.

7. The complex of claim 6, wherein the secondary nucleic acid molecules are hybridized to at least one, at least two, at least three, at least four, at least five, at least six or at least seven tertiary nucleic acid molecules comprising at least one detectable label.

8. The complex of any one of claims 1-7, wherein the detectable labels comprise multiple moieties each capable of being identified by their emission spectrum

   preferably wherein the moieties are fluorescent moieties.

9. A method of detecting at least one target nucleic acid in a sample, the method comprising:

   a) providing the complex of any one of claims 1-8, wherein the complex is hybridized to the at least one target nucleic acid in the sample;
   b) detecting the first detectable label associated with the first attachment position and the second detectable label associated with the at least second attachment position;
   c) removing the first detectable label;
   d) detecting the second detectable label associated with the at least second attachment position; and
   e) detecting the at least one target nucleic acid in the sample based on the detectable labels detected in step (b) and step (d).

10. The method of claim 9, wherein the detecting in step (d) comprises subtracting a signal from second detectable label associated with the at least second attachment region in step (d) form a signal from detecting the first detectable label associated with the first attachment region and the second detectable label associated with the at least second attachment region in step (b).

11. The method of claim 9 or claim 10, wherein step (b) further comprises detecting the third detectable label associated with the at least third attachment position.

12. The method of claim 11, wherein the step (d) further comprises detecting the third detectable label associated with the at least third attachment position.

13. The method of claim 12, wherein the method further comprises, after (d) and before step (e):

    $d_1$) removing the second detectable label; and
    $d_2$) detecting the third detectable label associated with the at least third attachment region,
    and wherein step (e) comprises detecting the at least one target nucleic acid in the sample based on the detectable labels detected in step (b), step (d), and step ($d_2$).

14. The method of any one of claims 9-13, wherein the sample is a tissue sample.

**Patentansprüche**

1. Komplex, umfassend:

   eine Sonde, die in der Lage ist, eine spezifische Region mindestens einer Zielnukleinsäure zu binden, wobei die mindestens eine Sonde eine Zielbindedomäne und eine Strichcode-Domäne umfasst,
   wobei die Zielbindedomäne 10-100 Nukleotide umfasst und in der Lage ist, die spezifische Region der Zielnukleinsäure zu binden, und wobei die Zielbindedomäne eine bekannte Nukleotidsequenz umfasst,
   wobei die Strichcode-Domäne eine erste Anbringungsregion, die durch ein erstes komplementäres Nukleinsäuremolekül gebunden ist, und eine wenigstens zweite Anbringungsregion, die durch ein wenigstens zweites komplementäres Nukleinsäuremolekül gebunden ist, umfasst,
   wobei es sich bei dem ersten komplementären Nukleinsäuremolekül um ein erstes komplementäres Nukleinsäuremolekül eines ersten Reporterkomplexes handelt, wobei der erste Reporterkomplex Folgendes umfasst:

   i) das erste komplementäre Nukleinsäuremolekül, das über einen photospaltbaren Linker indirekt mit einem primären Nukleinsäuremolekül verknüpft ist; und
   ii) eine erste nachweisbare Markierung, wodurch eine nachweisbare Markierung mit der ersten Anbringungsregion assoziiert wird,

wobei es sich bei dem zweiten komplementären Nukleinsäuremolekül um ein zweites komplementäres Nukleinsäuremolekül eines zweiten Reporterkomplexes handelt, wobei der zweite Reporterkomplex eine zweite nachweisbare Markierung umfasst, wodurch eine nachweisbare Markierung mit der wenigstens zweiten Anbringungsregion assoziiert wird,

wobei die Sequenzen der ersten Anbringungsregion und der wenigstens zweiten Anbringungsregion unterschiedlich sind.

2. Komplex nach Anspruch 1, wobei die Zielbindedomäne Folgendes umfasst:

   i) 20-60 Nukleotide; oder
   ii) 35-50 Nukleotide.

3. Komplex nach Anspruch 1 oder Anspruch 2, wobei der zweite Reporterkomplex das zweite komplementäre Nukleinsäuremolekül umfasst, das über einen photospaltbaren Linker indirekt mit einem primären Nukleinsäuremolekül verknüpft ist.

4. Komplex nach einem der Ansprüche 1-3, wobei die Strichcode-Domäne ferner eine wenigstens dritte Anbringungsregion umfasst, die durch eine wenigstens dritte komplementäre Nukleinsäure gebunden ist, wobei das dritte komplementäre Nukleinsäuremolekül eine dritte nachweisbare Markierung umfasst, wodurch eine nachweisbare Markierung mit der wenigstens dritten Anbringungsregion assoziiert wird.

5. Komplex nach Anspruch 4, wobei es sich bei dem dritten komplementären Nukleinsäuremolekül um ein drittes komplementäres Nukleinsäuremolekül eines dritten Reporterkomplexes handelt, wobei der dritte Reporterkomplex das dritte komplementäre Nukleinsäuremolekül umfasst, indirekt über einen photospaltbaren Linker mit einem primären Nukleinsäuremolekül verknüpft ist.

6. Komplex nach einem der Ansprüche 1-5, wobei die primären Nukleinsäuremoleküle an mindestens ein, mindestens zwei, mindestens drei, mindestens vier, mindestens fünf oder mindestens sechs sekundäre Nukleinsäuremoleküle hybridisiert sind, gegebenenfalls wobei zweite Nukleinsäuremoleküle einen photospaltbaren Linker und/oder mindestens eine nachweisbare Markierung umfassen.

7. Komplex nach Anspruch 6, wobei die sekundären Nukleinsäuremoleküle an mindestens ein, mindestens zwei, mindestens drei, mindestens vier, mindestens fünf, mindestens sechs oder mindestens sieben tertiäre Nukleinsäuremoleküle, die mindestens eine nachweisbare Markierung umfassen, hybridisiert sind.

8. Komplex nach einem der Ansprüche 1-7, wobei die nachweisbaren Markierungen mehrere Gruppierungen umfassen, die jeweils anhand ihres Emissionsspektrum identifiziert werden können, vorzugsweise wobei es sich bei den Gruppierungen um Fluoreszenzgruppierungen handelt.

9. Verfahren zum Nachweisen mindestens einer Zielnukleinsäure in einer Probe, wobei das Verfahren Folgendes umfasst:

   a) Bereitstellen des Komplexes nach einem der Ansprüche 1-8, wobei der Komplex an die mindestens eine Zielnukleinsäure in der Probe hybridisiert ist;
   b) Nachweisen der ersten nachweisbaren Markierung, die mit der ersten Anbringungsposition assoziiert ist, und der zweiten nachweisbaren Markierung, die mit der wenigstens zweiten Anbringungsposition assoziiert ist;
   c) Entfernen der ersten nachweisbaren Markierung;
   d) Nachweisen der zweiten nachweisbaren Markierung, die mit der wenigstens zweiten Anbringungsposition assoziiert ist; und
   e) Nachweisen der mindestens einen Zielnukleinsäure in der Probe aufgrund der in Schritt (b) und Schritt (d) nachgewiesenen nachweisbaren Markierungen.

10. Verfahren nach Anspruch 9, wobei das Nachweisen in Schritt (d) Subtrahieren eines Signals von der zweiten nachweisbaren Markierung, die mit der wenigstens zweiten Anbringungsregion in Schritt (d) assoziiert ist, von einem Signal vom Nachweisen der ersten nachweisbaren Markierung, die mit dem ersten Anbringungsregion assoziiert ist, und der zweiten nachweisbaren Markierung, die mit der wenigstens zweiten Anbringungsregion assoziiert ist, in Schritt (b) umfasst.

**11.** Verfahren nach Anspruch 9 oder Anspruch 10, wobei Schritt (b) ferner Nachweisen der dritten nachweisbaren Markierung, die mit der wenigstens dritten Anbringungsposition assoziiert ist, umfasst.

**12.** Verfahren nach Anspruch 11, wobei der Schritt (d) ferner Nachweisen der dritten nachweisbaren Markierung, die mit der wenigstens dritten Anbringungsposition assoziiert ist, umfasst.

**13.** Verfahren nach Anspruch 12, wobei das Verfahren ferner nach (d) und vor Schritt (e) Folgendes umfasst:

d$_1$) Entfernen der zweiten nachweisbaren Markierung; und
d$_2$) Nachweisen der dritten nachweisbaren Markierung, die mit der wenigstens dritten Anbringungsregion assoziiert ist,
und wobei Schritt (e) Nachweisen der mindestens einen Zielnukleinsäure in der Probe aufgrund der in Schritt (b), Schritt (d) und Schritt (d$_2$) nachgewiesenen nachweisbaren Markierungen umfasst.

**14.** Verfahren nach einem der Ansprüche 9-13, wobei es sich bei der Probe um eine Gewebeprobe handelt.


**Revendications**

**1.** Complexe comprenant :

une sonde pouvant se lier à une région spécifique d'au moins un acide nucléique cible, la sonde comprenant un domaine de liaison à une cible et un domaine de code-barres.
dans lequel le domaine de liaison à une cible comprend de 10 à 100 nucléotides et peut se lier à la région spécifique de l'acide nucléique cible, et dans lequel le domaine de liaison à une cible comprend une séquence nucléotidique connue,
dans lequel le domaine de code-barres comprend une première région de liaison liée par une première molécule d'acide nucléique complémentaire et au moins une deuxième région de liaison liée par au moins une deuxième molécule d'acide nucléique complémentaire.
dans lequel la première molécule d'acide nucléique complémentaire est une première molécule d'acide nucléique complémentaire d'un premier complexe rapporteur, le premier complexe rapporteur comprenant :

i) la première molécule d'acide nucléique complémentaire indirectement liée à une molécule d'acide nucléique primaire par un lieur photoclivable ; et
ii) un premier marqueur détectable, associant ainsi un marqueur détectable à la première région de liaison,

dans lequel la deuxième molécule d'acide nucléique complémentaire est une deuxième molécule d'acide nucléique complémentaire d'un deuxième complexe rapporteur, le deuxième complexe rapporteur comprenant un deuxième marqueur détectable, associant ainsi un marqueur détectable à la ou aux deuxièmes régions de liaison,
dans lequel les séquences de la première région de liaison et de la ou des deuxièmes régions de liaison sont différentes.

**2.** Complexe selon la revendication 1, dans lequel le domaine de liaison à une cible comprend :

i) 20 à 60 nucléotides ; ou
ii) 35 à 50 nucléotides.

**3.** Complexe selon la revendication 1 ou 2, dans lequel le deuxième complexe rapporteur comprend la deuxième molécule d'acide nucléique complémentaire indirectement liée à une molécule d'acide nucléique primaire par un lieur photoclivable.

**4.** Complexe selon l'une quelconque des revendications 1 à 3, dans lequel le domaine de code-barres comprend en outre au moins une troisième région de liaison liée par au moins un troisième acide nucléique complémentaire, dans lequel la troisième molécule d'acide nucléique complémentaire comprend un troisième marqueur détectable, associant ainsi un marqueur détectable à la ou aux troisièmes régions de liaison.

**5.** Complexe selon la revendication 4, dans lequel la troisième molécule d'acide nucléique complémentaire est une troisième molécule d'acide nucléique complémentaire d'un troisième complexe rapporteur, le troisième complexe rapporteur comprenant la troisième molécule d'acide nucléique complémentaire étant indirectement lié à une molécule d'acide nucléique primaire par un lieur photoclivable.

6. Complexe selon l'une quelconque des revendications 1 à 5, dans lequel les molécules d'acide nucléique primaires sont hybridées à au moins une, au moins deux, au moins trois, au moins quatre, au moins cinq ou au moins six molécules d'acide nucléique secondaires, facultativement, les deuxièmes molécules d'acide nucléique comprenant un lieur photoclivable et/ou au moins un marqueur détectable.

7. Complexe selon la revendication 6, dans lequel les molécules d'acide nucléique secondaires sont hybridées à au moins une, au moins deux, au moins trois, au moins quatre, au moins cinq, au moins six ou au moins sept molécules d'acide nucléique tertiaires comprenant au moins un marqueur détectable.

8. Complexe selon l'une quelconque des revendications 1 à 7, dans lequel les marqueurs détectables comprennent des fragments multiples, chacun pouvant être identifié par son spectre d'émission de préférence, les fragments étant des fragments fluorescents.

9. Procédé de détection d'au moins un acide nucléique cible dans un échantillon, le procédé comprenant :

a) la fourniture du complexe selon l'une quelconque des revendications 1 à 8, le complexe étant hybridé au(x) acide(s) nucléique(s) cible(s) dans l'échantillon ;
b) la détection du premier marqueur détectable associé à la première position de liaison et du deuxième marqueur détectable associé à la ou aux deuxièmes positions de liaison ;
c) l'élimination du premier marqueur détectable ;
d) la détection du deuxième marqueur détectable associé à la ou aux deuxièmes positions de liaison ; et
e) la détection de l'acide nucléique cible dans l'échantillon sur la base des marqueurs détectables détectés à l'étape (b) et à l'étape (d).

10. Procédé selon la revendication 9, dans lequel la détection à l'étape (d) comprend la soustraction d'un signal du deuxième marqueur détectable associé à la ou aux deuxièmes régions de liaison à l'étape (d) du signal de détection du premier marqueur détectable associé à la première région de liaison et du deuxième marqueur détectable associé à la ou aux deuxièmes régions de liaison à l'étape (b).

11. Procédé selon la revendication 9 ou 10, dans lequel l'étape (b) comprend en outre la détection du troisième marqueur détectable associé à la ou aux troisièmes positions de liaison.

12. Procédé selon la revendication 11, dans lequel l'étape (d) comprend en outre la détection du troisième marqueur détectable associé à la ou aux troisièmes positions de liaison.

13. Procédé selon la revendication 12, le procédé comprenant en outre, après l'étape (d) et avant l'étape (e) :

$d_1$) le retrait du deuxième marqueur détectable ; et
$d_2$) la détection du troisième marqueur détectable associé à la ou aux troisièmes régions de liaison,
et dans lequel l'étape (e) comprend la détection du ou des acides nucléiques cibles dans l'échantillon sur la base des marqueurs détectables détectés à l'étape (b), à l'étape (d) et à l'étape ($d_2$).

14. Procédé selon l'une quelconque des revendications 9 à 13, dans lequel l'échantillon est un échantillon de tissu.

Figure 1

Figure 2

Figure 3

Second Position on
Barcode Domain having
six attachment regions

$n_1$ $n_2$ $n_3$ $n_4$

Target binding domain

# Figure 4

Figure 5A

Sequence complementary to first attachment region

First pool of hybridizing nucleic acids lacking Detectable labels

No detectable label

Target Nucleic Acid

Figure 5B

Figure 5C

Sequence complementary to sixth attachment region

Sixth pool of complementary nucleic acids comprising detectable labels

or

Sixth pool of reporter complexes comprising detectable labels

Target Nucleic Acid

Figure 5D

Target sequence in
Target Nucleic Acid

Binding Moiety,
e.g., Biotin

Glass slide coated with a
complement to Affinity Tag,
e.g., Streptavidin-hydrogel

Figure 6

Binding Moiety,
e.g., Biotin

Figure 7

Affinity Reagent,
e.g., hapten or
nucleic acid sequence

Figure 8A

Affinity Reagent

Figure 8B

Affinity Reagent,
e.g., nucleic
acid sequence

Affinity Reagent,
e.g., nucleic
acid sequence

Figure 8C

Affinity Reagent,
e.g., nucleic
acid sequence

Figure 8D

Affinity Reagent,
e.g., nucleic
acid sequence

Affinity Reagent,
e.g., nucleic
acid sequence

Figure 8E

Figure 9A

Reporter complex comprising a detectable label

Figure 9B

Figure 9C

Cycle 1: → probe Hyb →    Position 1 Spot Hyb
                                (Image)

# Figure 9D

Cycle 1: → probe Hyb → Position 1 Spot Hyb → Position 2 Spot Hyb → → →
(Image)                    (Image)

Figure 9E

Cycle 1: → probe Hyb → Position 1 Spot Hyb → Position 2 Spot Hyb → → → → Position 6 Spot Hyb
(Image) (Image) (Image)

Figure 9F

| | Target Binding Domain | (Optional) dsDNA spacer | Flank 1 | AR-1 | AR-1/ Flank 2 | AR-2 | AR-2/ Flank 3 | AR-3 | AR-3/ Flank 4 | AR-4 | AR-4/ Flank 5 | |
|---|---|---|---|---|---|---|---|---|---|---|---|---|
| V2 Design | TTCACTGTAG | CTGTCTCATTTTGCTGCATCCTGTCCGTTCACGTTG | GAGCTT | GTCATC | CGTCCT | CTTTTC | ACTCCT | AGGCAT | TTGCCT | ATTCGG | CGTCCT | · · · |

Step 1: AR-1 Detect

Step 2: Lack 1

Step 3: AR-2 Detect

Flank = Flanking single-stranded polynucleotide
AR = Attachment Region
Detect = Complementary Nucleic Acid Comprising Detectable Label
Lack = Hybridizing Nucleic Acid Lacking Detectable Label

Figure 10

EP 4 324 929 B1

| | Domain | (Optional) dsDNA spacer | Flank 1 | AR-1 | AR-1/ Flank 2 | AR-2 | AR-2/ Flank 3 | AR-3 | AR-3/ Flank 4 | AR-4 | AR-4/ Flank 5 |
|---|---|---|---|---|---|---|---|---|---|---|---|
| V2 Design | TTCACTGTAG | CTGTCTCATTTTGCTGCATCCTGTCCGTTCACGTTG | GAGCTT | GTCATC | GGTCCT | CTTTTC | ACTCCT | AGGCAT | TTGCCT | ATTCGG | CGTCCT |

· · ·

Step 1:            AR-1 Detect

Step 2:      Lack 1      +      AR-2 Detect

Step 3:            Lack 2      +      AR-3 Detect

Step 4:                  Lack 3      +      AR-4 Detect

........

........

........

Step N:

# Figure 11

**5x5**

(1 primary nucleic acid molecule, 5 secondary nucleic acid molecules, 5 tertiary nucleic acid molecule with detectable label(s))

Tertiary nucleic acid molecule

3′ 12 bases

Primary nucleic acid molecule

**4x3**

Tertiary nucleic acid molecule

3′ 12 bases

Primary nucleic acid molecule

**3x4**

Tertiary nucleic acid molecule

3′ 12 bases

Primary nucleic acid molecule

**Spacer 3x4**

Tertiary nucleic acid molecule

3′ 12 bases  Spacer region (20-40 bases)

Primary nucleic acid molecule

Figure 12A

## Average Counts / FOV

☑ G - Channel Singlets > 16

## Figure 12B

Exemplary recipe for constructing reporter
complexes comprising detectable labels

| Volume (uL) | Primary n. a. molecule (10uM) | Secondary n. a. molecule (10uM) | Tertiary n. a. molecule (100uM) | H2O |
|---|---|---|---|---|
| 5x4 | 1 | 4.5 | 2.25 | 92.25 |
| 5x3 | 1 | 4.5 | 1.8 | 92.7 |
| 4x4 | 1.28 | 4.5 | 2.25 | 91.97 |
| 4x3 | 1.28 | 4.5 | 1.8 | 92.42 |
| 3x4 | 1.8 | 4.5 | 2.25 | 91.45 |

## Figure 12C

## 4x4 'extra-handles'

## 4x3 'extra-handles'

Figure 13A

## Average Counts/FOV

Figure 13B

Figure 14A

EP 4 324 929 B1

'7x3 extra handles' Kinetics

Average Counts / FOV

Figure 14B

"Small barcode"

"Universal Capture Tag"

tag    3BF2

G4

"Probe B"    30-50 mer

Nucleic acid target    30-50 mer

2.5nM:RNA input: 2.5nM :9.6nM

Pros
- ~10x more efficient
- 30 min hyb times

Cons
- Multiple reagents
- Multiple scans

Figure 15A

Figure 15B

Figure 15C

Figure 16A

Figure 16B

Figure 17A

| | 1 | 2 | 3 | 4 |
|---|---|---|---|---|
| gDNA | + | - | + | + |
| Capture Probe Set A (B & Y) | + | + | + | - |
| Capture Probe Set B (G & R) | + | + | - | + |

Figure 17B

EP 4 324 929 B1

Figure 18

14 Class Classification

10 Class Classification

Figure 19

Figure 20

Figure 21

Haplotype 1: 5'

Heterozygous site 1          Heterozygous site 2

Haplotype 2: 5'

sequencing windows

Figure 22

Figure 23

Figure 24

## Cleavable Reporter design

## Example cleavage modifications

Figure 25

Counts-BKGD

Pre-complexed BC/RPTR is slower to bind than BC alone but improves with time.

Figure 26

EP 4 324 929 B1

Figure 27

# Figure 28

| Target | Name | BC Sequence | 6 LNAs + 2 Ns | Spot 1 | Spot 2 | Spot 3 |
|--------|------|-------------|---------------|--------|--------|--------|
| KRAS2 | KRASex2-BC17 | CCAACTAC | N+C+A+A+C+T+AN | PC-GY | YY | GY |

Only reporter1 is photocleavable.

Intensity

Loss of Intensity

Observed vs Expected Relative Intensity Loss

EP 4 324 929 B1

# Figure 29

| Target | Name | BC Sequence | 6 LNAs + 2 Ns | Spot 1 | Spot 2 | Spot 3 |
|--------|------|-------------|---------------|--------|--------|--------|
| KRAS2 | KRASex2-BC19 | CTACCACA | N+T+A+C+C+A+CN | PC-GG | YY | GY |

Intensity

Intensity (Relative)

Feature 1    Feature 1 + UV    Feature 2    Feature 2 + UV

☐ B    ☒ G    ☐ Y    ▥ R

Only reporter1 is photocleavable.

Loss of Intensity

Intensity Loss Intensity

Feature 1    Feature 1 + UV    Feature 2    Feature 2 + UV

☐ B    ☒ G    ☐ Y    ▥ R

Observed vs Expected Relative Intensity Loss

% intensity loss

Feature 1    Expected       Feature 2    Expected
Observed                     Observed

EP 4 324 929 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4757141 A
- US 5151507 A
- US 5091519 A
- US 5188934 A
- US 5366860 A
- US 5847162 A
- US 4318846 A
- US 5800996 A
- US 5066580 A
- US 5688648 A
- US 6322901 B
- US 6576291 B
- US 6423551 B
- US 6251303 B
- US 6319426 B
- US 6426513 B
- US 6444143 B
- US 5990479 A
- US 6207392 B
- US 20020045045 A
- US 20030017264 A
- US 4230558 A
- US 4811218 A
- US 8148512 B
- US 7473767 B
- US 7919237 B
- US 7941279 B
- US 8415102 B
- US 8492094 B
- US 8519115 B
- US 20090220978 A
- US 20090299640 A
- US 20100015607 A
- US 20100261026 A
- US 20110086774 A
- US 20110145176 A
- US 20110201515 A
- US 20110229888 A
- US 20130004482 A
- US 20130017971 A
- US 20130178372 A
- US 20130230851 A
- US 20130337444 A
- US 20130345161 A
- US 20140005067 A
- US 20140017688 A
- US 20140037620 A
- US 20140087959 A
- US 20140154681 A
- US 20140162251 A
- US 946386

**Non-patent literature cited in the description**

- **LIN et al.** Submicrometre geometrically encoded fluorescent barcodes self-assembled from DNA. *Nature Chemistry*, October 2012, vol. 4 (10), 832-9
- **JUNGMANN et al.** Multiplexed 3D cellular super-resolution imaging with DNA-PAINT and Exchange-PAINT. *Nature Methods,* 2014, vol. 11 (3)
- **HAUGLAND**. Handbook of Fluorescent Probes and Research Chemicals. Molecular Probes, Inc., 2002
- **KELLER** ; **MANAK**. DNA Probes. Stockton Press, 1993
- Oligonucleotides and Analogues: A Practical Approach. IRL Press, 1991
- **WETMUR**. Critical Reviews in Biochemistry and Molecular Biology. 1991, vol. 26, 227-259
- **WHEELESS et al.** Flow Cytometry: Instrumentation and Data Analysis. Academic Press, 1985, 21-76
- **SEELING et al.** Catalyzed Relaxation of a Metastable DNA Fuel. *J. Am. Chem. Soc.*, 2006, vol. 128 (37), 12211-12220
- **FRITSCHE** ; **MANIATIS**. Molecular Cloning A Laboratory Manual. Cold Spring Harbor Press, 1989
- Anderson Nucleic Acid Hybridization. BIOS Scientific Publishers Limited, 1999